# EUROPEAN PATENT SPECIFICATION

(11) **EP 1 549 735 B1**
(45) Date of publication and mention of the grant of the patent: **24.08.2011**
(21) Application number: 03777599.6
(22) Date of filing: 14.10.2003
(51) Int. Cl.: C12N 1/20, C12N 15/64, C12N 15/70, C12Q 1/02

(54) **CIRCULAR DNA MOLECULE HAVING A CONDITIONAL ORIGIN OF REPLICATION, PROCESS FOR THEIR PREPARATION AND THEIR USE IN GENE THERAPY**
ZIRKULÄRES DNA-MOLEKÜL MIT EINEM KONDITIONALEN REPLIKATIONSURSPRUNG, VERFAHREN ZUR HERSTELLUNG DAVON UND IHRE VERWENDUNG IN DER GENTHERAPIE
MOLECULE D'ADN CIRCULAIRE COMPRENANT UNE ORIGINE DE REPLICATION CONDITIONNELLE, PROCEDE DE PREPARATION DE CETTE MOLECULE ET UTILISATION CORRESPONDANTE DANS LA THERAPIE GENIQUE

(30) Priority: 11.10.2002 US 268948
(43) Date of publication of application: 06.07.2005
(73) Proprietor: Aventis Pharma S.A., 92160 Antony (FR)
(72) Inventor: SOUBRIER, Fabienne, F-94320 Thiais (FR)
(74) Representative: Bouvet, Philippe
(86) International application number: PCT/US2003/032512
(87) International publication number: WO 2004/033664

(56) References cited:
- WO-A-97/10343
- US-A- 5 851 808
- US-A- 5 874 259
- US-A1- 2001 014 476
- SOUBRIER F ET AL: "PCOR: A NEW DESIGN OF PLASMID VECTORS FOR NONVIRAL GENE THERAPY" GENE THERAPY, MACMILLAN PRESS LTD., BASINGSTOKE, GB, vol. 6, August 1990 (1990-08), pages 1482-1488, XP001078780 ISSN: 0969-7128
- CARRIÈRE M ET AL: "Optimization of plasmids for gene delivery" S.T.P. PHARMA SCIENCES 2001 FRANCE, vol. 11, no. 1, 2001, pages 1-6, XP002354427 ISSN: 1157-1489
- MIRON ALEXANDER ET AL: "Multiple pathways of copy control of gamma replicon of R6K: Mechanisms both dependent on and independent of cooperativity of interaction of pi protein with DNA affect the copy number" PROCEEDINGS OF THE NATIONAL ACADEMY OF SCIENCES OF THE UNITED STATES OF AMERICA, vol. 91, no. 14, 1994, pages 6438-6442, XP002354428 ISSN: 0027-8424
- SUMMERS D: "TIMING, SELF-CONTROL AND A SENSE OF DIRECTION ARE THE SECRETS OF MULTICOPY PLASMID STABILITY" MOLECULAR MICROBIOLOGY, BLACKWELL SCIENTIFIC, OXFORD, GB, vol. 29, no. 5, September 1998 (1998-09), pages 1137-1145, XP001056700 ISSN: 0950-382X
- SOUBRIER F ET AL: "Improvement of pCOR plasmid copy number for pharmaceutical applications" APPLIED MICROBIOLOGY AND BIOTECHNOLOGY, vol. 66, no. 6, March 2005 (2005-03), pages 683-688, XP002354429 ISSN: 0175-7598
- KELLEY ET AL: 'Conformational changes induced by integration host factor at origin gamma of R6K and copy number control' JOURNAL OF BIOLOGICAL CHEMISTRY vol. 266, no. 24, 25 August 1991, pages 15924 - 15937, XP002903923

## Description

The present invention relates to a novel conditional replication DNA molecule which can be used in gene therapy or for the production of recombinant proteins. The novel DNA molecules according to the present invention are designated pCOR™ herein after.

Gene therapy consists in correcting a deficiency or an anomaly by introducing genetic information into the affected organ or cell. This information may be introduced either *in vitro* into a cell extracted from the organ and then reinjected into the body, or *in vivo,* directly into the target tissue. As a molecule of high molecular weight and of negative charge, DNA has difficulty in spontaneously crossing phospholipid cell membranes. Various vectors are thus used in order to enable gene transfer to take place: viral vectors, on the one hand, and natural or synthetic chemical and/or biochemical vectors, on the other hand.

Viral vectors (retroviruses, adenoviruses, adeno-associated viruses, etc.) are very effective, in particular for crossing membranes, but present a certain number of risks such as pathogenicity, recombination, replication, and immunogenicity.

Chemical and/or biochemical vectors allow these risks to be avoided (for reviews, see Behr, 1993, Cotten and Wagner 1993). These are, for example, cations (calcium phosphate, DEAE-dextran, etc.) which act by forming precipitates with DNA, which may be "phagocytosed" by the cells. They may also be liposomes in which the DNA is incorporated and which fuse with the plasma membrane. Synthetic gene-transfer vectors are generally lipids or cationic polymers which complex the DNA and form with it a particle bearing positive surface charges. As illustrations of vectors of this type, mention may be made in particular of dioctadecylamidoglycylspermine (DOGS, Transfectam^{™}) or N-[1-(2,3-dioleyloxy)propyl]-N,N,N-trimethylammonium (DOTMA, Lipofectin^{™}).

However, the use of chemical and/or biochemical vectors or naked DNA implies the possibility of producing large amounts of DNA of pharmacological purity. The reason for this is that in gene therapy techniques, the medicinal product consists of the DNA itself and it is essential to be able to manufacture, in suitable amounts, DNAs having properties which are appropriate for therapeutic use in man.

In the case of non-viral vectorology, the vectors used are plasmids of bacterial origin. The plasmids generally used in gene therapy carry (i) an origin of replication, (ii) a marker gene such as a gene for resistance to an antibiotic (kanamycin, ampicillin, etc.) and (iii) one or more transgenes with sequences necessary for their expression (enhancer(s), promoter(s), polyadenylation sequences, etc.). However, the technology currently available is not entirely satisfactory.

On the one hand, the risk remains of dissemination in the body. Thus, a bacterium which is present in the body can, at low frequency, receive this plasmid. There is a greater likelihood of this taking place if it involves an *in vivo* gene therapy treatment in which the DNA may be disseminated in the body of the patient and may come into contact with bacteria which infect this patient or bacteria of the commensal flora. If the bacterium receiving the plasmid is an enterobacterium, such as *E. coli,* this plasmid can be replicated. Such an event then leads to dissemination of the therapeutic gene. Insofar as the therapeutic genes used in gene therapy treatments can code, for example, for a lymphokine, a growth factor, an anti-oncogene or a protein whose function is defective in the host and which thus makes it possible to correct a genetic defect, the dissemination of some of these genes could have unforeseeable and worrying effects (for example if a pathogenic bacterium acquired a human growth factor gene).

On the other hand, the plasmids generally used in non-viral gene therapy also possess a marker for resistance to an antibiotic (ampicillin, kanamycin, etc.). The bacterium acquiring such a plasmid thus has an undeniable selective advantage since any antibiotic therapy, using an antibiotic from the same family as that which selects the plasmid resistance gene, will lead to selection of the plasmid in question. In this respect, ampicillin belongs to the α-lactams, which is the family of antibiotics which is most frequently used worldwide. The use in bacteria of selection markers which are not antibiotic-resistance genes would thus be particularly advantageous. This would avoid the selection of bacteria which may have received a plasmid carrying such a marker.

WO9710343 discloses the XAC-1 *pir116* strain (ara, *Δ(lac-proB)ₓ₁₁₁, gyrA, argEₐₘ*, *rpoB, thi, uidA(ΔMlul)::pir116*/*F'[proB⁺ lacl₃₇₃ lacZᵤ₁₁₈ₐₘ*]).

SOUBRIER ET AL (GENE THERAPY, vol. 6, 1999, 1482-1488) and CARRIÈRE ET AL (S.T.P. PHARMA SCIENCES, vol. 11, no. 1, 2001, 1-6), both disclose the *XAC pir116* strain, too, as well as a *traD* derivative of said strain.

MIRON ALEXANDER ET AL (PROCEEDINGS OF THE NATIONAL ACADEMY OF SCIENCES OF THE UNITED STATES OF AMERICA, vol. 91, no. 14, 1994, 6438-6442) discloses the characterisation of a number of *pir* mutations, including *pir* 42, but neither discloses nor suggests making double mutants and using them to increase the copy number.

It is thus particularly important to seek to limit the dissemination of therapeutic genes and resistance genes as much as possible.

The invention relates to claim 1

The DNA molecules have the advantage of removing the risks associated with dissemination of the plasmid, such as (1) replication and dissemination, which can lead to uncontrolled overexpression of the therapeutic gene, (2) dissemination and expression of resistance genes. The genetic information contained in the DNA molecules according to the invention effectively comprises the therapeutic gene(s) and the signals for regulating its (their) expression, a functional conditional origin of replication which greatly limits the host cell spectrum of this plasmid, a selection marker of reduced size which is preferably different from a gene which imparts resistance to an antibiotic and, where appropriate, a DNA fragment which allows the resolution of plasmid multimers. The probability of these molecules (and thus the genetic information which they contain) being transferred to a microorganism, and maintained stably, is very limited.

Lastly, the vectors according to the invention, also referred to as miniplasmids on account of their circular structure, their reduced size and their supercoiled form, have the following additional advantages: on account of their size which is reduced in comparison with the ColE1-derived plasmids used conventionally, the DNA molecules according to the invention potentially have better *in vivo* bioavailability, and the DNA molecules or pCOR stay in a stable extrachromosomal form in the host prokaryotic or eukaryotic cells that do not contain the initiating protein. In particular, they have improved capacities of cell penetration and distribution. Thus, it is acknowledged that the diffusion coefficient in tissues is inversely proportional to the molecular weight (Jain, 1987). Similarly, in the cell, high molecular weight molecules have poorer permeability across the plasma membrane. In addition, in order for the plasmid to pass into the nucleus, which is essential for its expression, the high molecular weight is also a disadvantage, the nuclear pores imposing a size limit for diffusion into the nucleus (Landford *et al.*, 1986). The reduction in size of the non-therapeutic parts of the DNA molecule (origin of replication and selection gene in particular) according to the invention also makes it possible to decrease the size of the DNA molecules. The part which allows the replication and selection of this plasmid in the bacterium (1 kb) is decreased by a factor of 3, counting, for example, 3 kb for the origin of replication and the resistance marker vector part. This decrease (i) in molecular weight and (ii) in negative charge imparts improved tissue, cellular and nuclear bioavailability and diffusion to the molecules of the invention.

More precisely, the present invention relates to a circular DNA molecule, which is useful in gene therapy, this molecule comprising at least one nucleic acid sequence of interest, characterized in that the region which allows its replication comprises an origin of replication whose functionality in a host cell requires the presence of at least one specific protein which is foreign to the said host cell.

This DNA molecule may be in single- or double-stranded form and advantageously possesses a supercoiled form.

For the purposes of the present invention, the host cells used can be of various origins. They can be eukaryotic or prokaryotic cells. According to a preferred embodiment of the invention, they are prokaryotic cells.

The replication of bacterial plasmids conventionally requires the presence of at least one protein, which is coded for by the host cell, of the RNA polymerase, Rnase, DNA polymerase, etc. type. For the reasons already explained above, it is not possible to overcome entirely, with this type of replication, any possible risks of dissemination in the treated organism. Advantageously, the functionality of the origin of replication of the DNA molecule according to the invention requires the presence of a specific protein which is foreign to the host cell. The significance of this characteristic is to reduce the host spectrum of the claimed plasmid to specific strains that express this initiator protein. The DNA molecule developed within the context of the present invention thus advantageously possesses a so-called conditional origin of replication.

The conditional origin of replication used according to the present invention may originate from plasmids or bacteriophages which share the following characteristics: they contain in their origin of replication repeat sequences, or iterons, and they code for at least one replication-initiating protein (Rep) which is specific to them. By way of example, mention may be made of the conditional replication systems of the following plasmids and bacteriophages:

| **plasmid or bacteriophage** | **specific initiator protein** |
|---|---|
| RK2 (Stalker *et al.,* 1981) | TrfA |
| R1 (Ryder *et al.*, 1981) | RepA |
| pSC101 (Vocke and Bastia, 1983) | RepA |
| F (Murotsu *et al.*, 1981) | protein E |
| Rts1 (Itoh *et al*., 1982,1987) | RepA |
| RSF1010 (Miao *et al.*, 1995) | RepC |
| P1 (Abeles *et al.*, 1984) | RepA |
| P4 (Flensburg and Calendar, 1987) | alpha protein |
| lambda (Moore *et al.,* 1981) | protein O |
| phi 82 (Moore *et al.*, 1981) | protein O from phi 82 |
| phi 80 | protein O from phi 80 |

According to a preferred embodiment of the invention, the origin of replication used in the DNA molecules claimed is derived from a natural *E. coli* plasmid referred to as R6K.

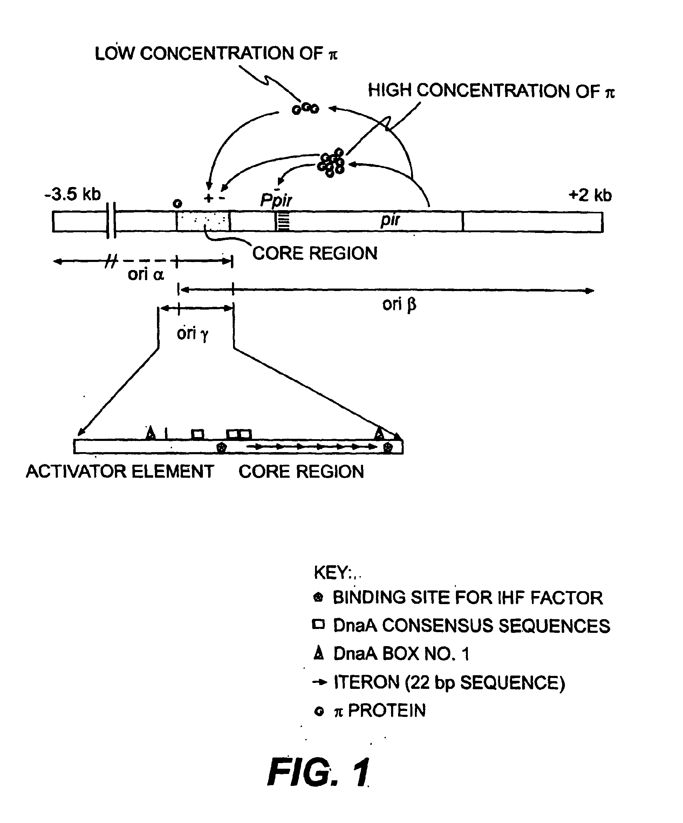

The replication functions of R6K are grouped together in a 5.5 kbp DNA fragment (Figure 1) comprising 3 origins of replication α, β, and γ (γ and α providing 90 % of the replication) and an operon coding for the Π replication-initiator protein and the protein Bis. The minimum amount of genetic information required to maintain this plasmid at its characteristic number of copies (15 copies per genome) is contained in two elements: the 400 bp of ori γ and the gene *pir*, whose product is the Π initiator protein.

Ori γ may be divided into two functional parts: the core part and the activator element (Figure 1). The core part, which is essential for replication, contains the iterons (7 direct repeats of 22 bp) to which the Π protein represented in SEQ ID No. 1 becomes bound, and flanking segments, which are targets of the host proteins (IHF, DnaA).

According to a preferred mode of the invention, the origin of replication of the vector claimed consists entirely or partially of this γ origin of replication of the plasmid R6K and more preferably, entirely or partially of SEQ ID No. 1 or one of its derivatives.

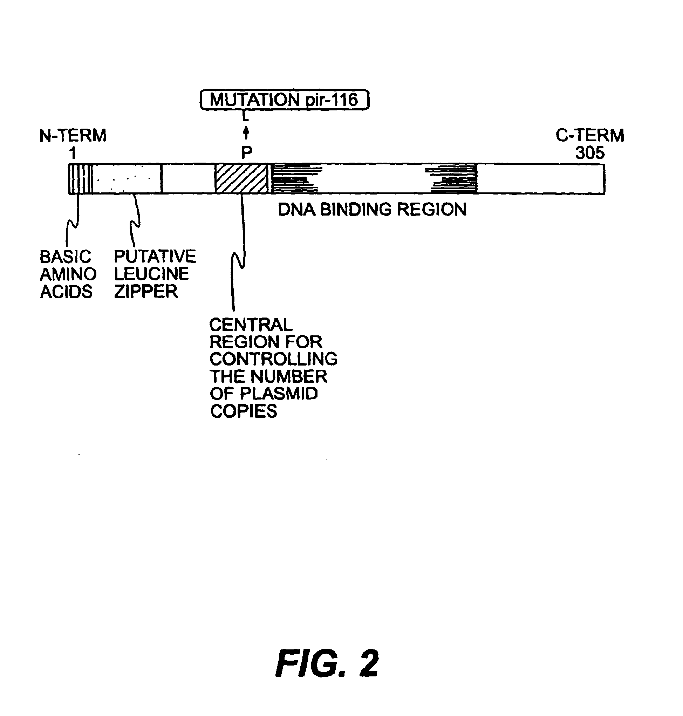

The origin of replication described above, which has the advantage of being of very limited size, is functional solely in the presence of a specific initiator protein, protein Pi, produced by the gene *pir* (SEQ ID No. 2). Since this protein can act in trans, it is possible to physically dissociate the ori gamma from the *pir* gene, which may be introduced into the genome of the cell which is chosen as the specific host for these plasmids. Mutations in Π may alter its inhibitory functions (Inuzuka and Wada, 1985) and lead to an increase in the number of copies of the R6K derivatives, up to more than 10 times the initial number of copies. These substitutions may be within a domain of 40 amino acids, which therefore appears to be responsible for the control by Π of the number of plasmid copies (Figure 2), or in other regions of the Π protein.

According to an advantageous embodiment of the present invention, the Π protein, expressed in the host cell, results from the expression of the gene represented in SEQ ID No. 2 or one of its derivatives as defined above and more particularly of the gene pir 116 which comprises a mutation when compared with the pir gene. This mutation corresponds to the replacement of a proline by a leucine at position 106 from the start codon. In this context, the number of copies of the R6K derivatives is about 250 copies per genome.

For the purposes of the present invention, the term derivative denotes any sequence which differs from the sequence considered, obtained by one or more modifications of genetic and/or chemical nature, as well as any sequence which hybridizes with these sequences or fragments thereof and whose product possesses the activity indicated with regard to the replication-initiator protein Π. The term modification of the genetic and/or chemical nature may be understood to refer to any mutation, substitution, deletion, addition and/or modification of one or more residues. The term derivative also comprises the sequences homologous with the sequence considered, derived from other cellular sources and in particular cells of human origin, or from other organisms, and possessing an activity of the same type. Such homologous sequences may be obtained by hybridization experiments. The hybridizations may be performed starting with nucleic acid libraries, using the native sequence or a fragment thereof as probe, under conventional conditions of stringency (Maniatis et al., cf. General techniques of molecular biology), or, preferably, under conditions of high stringency.

Besides a conditional origin of replication as defined above, the DNA molecules claimed contain a region comprising one (or more) gene(s) which make it possible to ensure selection of the DNA molecule in the chosen host.

This may be a conventional marker of gene type which imparts resistance to an antibiotic, such as kanamycin, ampicillin, chloramphenicol, streptomycin, spectinomycin, lividomycin or the like.

However, according to a preferred embodiment of the invention, this region is different from a gene which imparts resistance to an antibiotic. It may thus be a gene whose product is essential for the viability of the host envisaged, under defined culturing conditions. It may be, for example:
- a gene coding for a suppressor tRNA, of natural or synthetic origin. This is, more preferably, an amber codon tRNA (TAG)
- a gene whose product is necessary for metabolism of the cell, under certain culturing conditions, namely a gene involved in the biosynthesis of a metabolite (amino acid, vitamin, etc.), or a catabolism gene which makes it possible to assimilate a substance present in the culture medium (specific nitrogen or carbon source), etc.

According to a preferred mode of the invention, this region contains an expression cassette of a gene coding for a suppressor tRNA for specific codons. This latter may be chosen, in particular, from those coding for phenylalanine, cysteine, proline, alanine and histidine amino acids. It is more particularly a suppressor tRNA for amber codons (TAG).

In this particular case, the system used to select, in the host cells, the DNA molecules which are the subject of the present invention includes two elements: 1) on the DNA molecule, a gene coding for a suppressor transfer RNA for the amber codon (TAG) which constitutes the selection marker, known as (*sup*) gene and 2) a specific host, one of whose genes, which is essential under certain culture conditions, contains an amber TAG codon. This cell may grow, under the culture conditions for which the product of the gene containing the TAG codon is essential, only if the plasmid allowing the expression of *sup* is present in the cell. The culture conditions thus constitute the pressure for selection of the DNA molecule. The *sup* genes used may be of natural origin (Glass *et al.,* 1982) or may originate from a synthetic construction (Normanly *et al.*, 1986, Kleina *et al*., 1990).

Such a system offers great flexibility insofar as, depending on the gene containing an amber mutation, it is possible to determine various selective media. In the bacterium *Lactococcus lactis* for example, the amber codon is located in a purine biosynthesis gene. This allows the selection of the plasmid carrying the gene coding for the suppressor tRNA when the bacteria multiply in milk. Such a marker has the advantage of being very small and of containing no "foreign" sequences, originating from phages or transposons.

According to a particular embodiment of the invention, the DNA molecule also comprises a DNA fragment, the target for site-specific recombinases, which allows the resolution of plasmid multimers.

Thus, such a fragment, introduced on to a DNA molecule which is circular and whose origin of replication is, for example, ori gamma, allows the resolution of multimers of such a plasmid. Such multimers are observed, in particular, when the DNA molecule is prepared in a strain carrying a mutated allele of *pir,* such as *pir116*, which makes it possible to increase the number of copies of the R6K derivatives.

This recombination may be achieved by means of various systems which entail site-specific recombination between sequences. More preferably, the site-specific recombination of the invention is obtained by means of specific intramolecular recombination sequences which are capable of recombining with each other in the presence of specific proteins, generally referred to as recombinases. In this specific case, these are the recombinases XerC and XerD. For this reason, the DNA molecules according to the invention generally also comprise a sequence which allows this site-specific recombination. The specific recombination system present in the genetic constructions according to the invention (recombinases and specific recognition site) may be of different origins. In particular, the specific sequences and the recombinases used may belong to different structural classes, and in particular to the transposon Tn3 resolvase family or to the bacteriophage lambda integrase family. Among the recombinases belonging to the transposon Tn3 family, mention may be made in particular of the resolvase of transposon Tn3 or of transposons Tn*21* and Tn*522* (Stark *et al.,* 1992); the Gin invertase of bacteriophage mu or alternatively plasmid resolvases, such as that of the *par* fragment of RP4 (Abert et al., Mol. Microbiol. 12 (1994) 131). Among the recombinases belonging to the bacteriophage λ integrase family, mention may be made in particular of the integrase of the phages lambda (Landy et al., Science 197 (1977) 1147), P22 and Φ80 (Leong et al., J. Biol. Chem. 260 (1985) 4468), HP1 of *Haemophilus influenzae* (Hauser et al., J. Biol. Chem. 267 (1992) 6859), the Cre integrase ofphage P1, the integrase of plasmid pSAM2 (EP 350 341) or alternatively the FLP recombinase of the 2µ plasmid and the XerC and XerD recombinases from *E. coli.*

Preferably, the DNA molecules which form the subject of the present invention contain the fragment *cer* from the natural *E. coli* plasmid ColE1. The cer fragment used is a 382 bp *Hpa*II fragment from ColE1 which has been shown to bring about, in *cis,* the resolution of plasmid multimers (Summers *et al.*, 1984; Leung *et al.*, 1985). It is also possible to use a *Hpa*II-*Taq*I fragment of smaller size (280 bp) or a smaller fragment (about 220 bp), contained in the *Hpa*II fragment, which fragments possess the same properties (Summers and Sherratt, 1988). This resolution takes place by way of a specific intramolecular recombination, which involves four proteins encoded by the genome of *E. coli:* ArgR, PepA, XerC and XerD (Stirling *et al.,* 1988, 1989; Colloms *et al.*, 1990, Blakely *et al.*, 1993). It was found that insertion of the fragment cer from the natural E.coli plasmid ColE1 allows to obtain a high resolution of plasmids multimers, thereby resulting in high proportion of monomers in a reproducible manner. This is particularly unexpected as it has been shown that the insertion of the *cer* site into a minicircle which contains the ColE1 origin of replication from pBluescript SK+ did not result in efficient multimer resolution (Kreiss et al., Appl.Microbiol.Biotechnol, 49:560-567 (1998)), and thus effective resolution in *cis* of plasmids is unpredictable and seems to depend on the plasmid conformation. In the case of the pCOR plasmid, an effective *cis* resolution is reached when *cer* is present on the pCOR, thereby resulting in a unexpectedly high monomers of pCOR in a reproducible manner.

In this respect, it is particularly advantageous to use all or part of the *cer* fragment of ColE1 or one of its derivatives as defined above.

According to an implementation variant, the DNA molecules of the invention may also comprise a sequence capable of interacting specifically with a ligand. Preferably, this is a sequence capable of forming, by hybridization, a triple helix with a specific oligonucleotide. This sequence thus makes it possible to purify the molecules of the invention by selective hybridization with a complementary oligonucleotide immobilized on a support (see application WO 96/18744 and WO 02/07727). The sequence may be naturally present in the origin of replication of the plasmid as described in US publication application 2003/186268 of the Applicant, or naturally present in the transgene as described in WO 02/07727, and alternatively can be positioned at any site in the DNA molecule of the invention, provided that it does not affect the functionality of the gene of interest and of the origin of replication. Formation of a triple helix by hybridization thus occurs between the oligonucleotide and the specific complementary sequence present in the DNA. In this connection, to obtain the best yields and the best selectivity, an oligonucleotide and a specific sequence which are fully complementary are used in the method of the invention. These can be, in particular, an oligonucleotide poly(CTT) and a specific sequence poly(GAA). For example, oligonucleotides containing repeated motifs such as CTT are capable of forming a triple helix with a specific sequence containing complementary units (GAA). The sequence in question can, in particular, be a region containing 7, 14 or 17 GAA units, and in the oligonucleotides a corresponding numbers of repeat CTT. In this case, the oligonucleotide binds in an antiparallel orientation to the polypurine strand. These triple helices are stable only in the presence of Mg²⁺ (Vasquez et al., Biochemistry, 34: 7243-7251 (1995); Beal and Dervan, Science, 251: 1360-1363 (1991)).

As stated above, the specific sequence can be a sequence naturally present in the pCOR, or may be a synthetic sequence introduced artificially in the latter. It is especially advantageous to use an oligonucleotide capable of forming a triple helix with a sequence naturally present in the pCOR, for example in the origin of replication of a plasmid or in a marker gene. The synthesis of oligonucleotides capable of forming triple helices with these natural homopurine-homopyrimidine regions is particularly advantageous, as it may be applied to unmodified pCOR plasmids. Particularly preferred target sequences which can form triplex structures with particular oligonucleotides have been identified in ColE1 and in pCOR origins of replication. ColE1-derived plasmids contain a 12-mer homopurine sequence (5'-AGAAAAAAAGGA-3') (SEQ ID NO: 33) mapped upstream of the RNA-II transcript involved in plasmid replication (Lacatena et al., Nature, 294: 623 (1981)). This sequence forms a stable triplex structure with the 12-mer complementary 5'-TCTTTTTTTCCT-3' (SEQ ID NO: 34) oligonucleotide. The pCOR backbone contains a homopurine stretch of 14 non repetitive bases (5'-AAGAAAAAAAAGAA-3') (SEQ ID NO: 35) located in the A+T-rich segment of the γ origin replicon of pCOR (Levchenko et al., Nucleic Acids Res., 24:1936 (1996)). This sequence forms a stable triplex structure with the 14-mer complementary oligonucleotide 5'-TTCTTTTTTTTCTT-3' (SEQ ID NO: 36). The corresponding oligonucleotides 5'-TCTTTTTTTCCT-3' (SEQ ID NO: 37) and 5'-TTCTTTTTTTTCTT-3' (SEQ ID NO: 38) efficiently and specifically target their respective complementary sequences located within the origin of replication of either ColE1 ori or pCOR (oriy). Also, use of an oligonucleotide capable of forming a triple helix with a sequence present in an origin of replication or a marker gene is especially advantageous, since it makes it possible, with the same oligonucleotide, to purify any DNA containing the said origin of replication or said marker gene. Hence it is not necessary to modify the plasmid or the double-stranded DNA in order to incorporate an artificial specific sequence in it.

Although fully complementary sequences are preferred, it is understood, however, that some mismatches may be tolerated between the sequence of the oligonucleotide and the sequence present in the DNA, provided they do not lead to too great a loss of affinity. The sequence 5'-AAAAAAGGGAATAAGGG-3' (SEQ ID NO: 39) present in the *E. coli* β-lactamase gene may be mentioned. In this case, the thymine interrupting the polypurine sequence may be recognized by a guanine of the third strand, thereby forming a G*TA triplet which it is stable when flanked by two T*AT triplets (Kiessling et al., Biochemistry, 31: 2829-2834 (1992)).

According to a particular embodiment, the oligonucleotides used may comprise the sequence (CCT)ₙ, the sequence (CT)ₙ or the sequence (CTT)ₙ, in which n is an integer between 1 and 15 inclusive. It is especially advantageous to use sequences of the type (CT)ₙ or (CTT)ₙ. Oligonucleotides may also combine (CCT), (CT) or (CTT) units.

The oligonucleotides used may be natural (composed of unmodified natural bases) or chemically modified. In particular, the oligonucleotide may advantageously possess certain chemical modifications enabling its resistance to or its protection against nucleases, or its affinity for the specific sequence, to be increased.

As a DNA molecule representative of the present invention, the plasmid pXL2774 and its derivatives may be claimed most particularly. For the purposes of the invention, the term derivative is understood to refer to any construction derived from pXL2774 and containing one or more genes of interest other than the luciferase gene. Mention may also be made of the plasmids pXL3029, pXL3030, and plasmid pXL3179 or NV1FGF containing an expression cassette of a therapeutic gene. In a most preferred embodiment, the invention relates to a pCOR comprising the FGFa or FGF-1 gene as described in US patent 4,686,113 of the Applicant, which is designated pXL 3179 or NV1FGF.

The present invention also relates to the development of a process for the construction of specific host cells, which are particularly effective for the production of these therapeutic DNA molecules.

Another subject of the present invention relates to a process for the production of a circular DNA molecule, characterized in that a host cell is cultured containing at least one DNA molecule as defined above and a protein, which may or may not be expressed *in situ,* which conditions the functionality of the origin of replication of the said DNA molecule, which is specific and which is foreign to the said host cell, under conditions which allow the selection of host cells transformed by the said DNA molecules.

More preferably, the protein which conditions the functionality of the origin of replication of the DNA molecule is expressed *in situ* from a corresponding gene. The gene coding for the replication-initiating protein may be carried by a subsidiary replicon, which is compatible with the derivatives of the conditional origin of replication used or which may be introduced into the genome of the host cell by recombination, by means of a transposon, a bacteriophage or any other vector. In the particular case in which the gene expressing the protein is placed on a subsidiary replicon, the latter also contains a promoter region for functional transcription in the cell, as well as a region which is located at the 3' end and which specifies a transcription termination signal. As regards the promoter region, this may be a promoter region which is naturally responsible for expressing the gene under consideration when the latter is capable of functioning in the cell. It may also be a case of regions of different origin (responsible for expressing other proteins), or even of synthetic origin. In particular, it may be a case of promoter sequences for prokaryotic or bacteriophage genes. For example, it may be a case of promoter sequences obtained from the cell genome.

As genes coding for the replication-initiating protein, use may be made either of wild-type genes or of mutated alleles which make it possible to obtain an increased number of copies of the plasmids (or derivatives) specific for the initiator protein which conditions the functionality of the origin of replication used in the DNA molecule.

Such mutants have been described in particular for the plasmids R6K (Inuzuka and Wada, 1985; Greener *et al.*, (1990), Rts1 (Terawaki and Itoh, 1985, Terawaki *et al.*, 1990; Zeng *et al.*, 1990), F (Seelke *et al.*, 1982; Helsberg *et al.*, 1985; Kawasaki *et al.,* 1991), RK2 (Durland *et al.*, 1990; Haugan *et al*., 1992, 1995), pSC101 (Xia *et al.,* 1991; Goebel *et al.*, 1991; Fang *et al.,* 1993).

In the particular case in which the DNA molecule used possesses an origin of replication derived from the plasmid R6K, the initiator protein is a derivative of the Π protein of this same plasmid. It is particularly advantageous to express a mutated form of this protein which is capable of increasing the number of initial copies appreciably. To do this, the gene incorporated into the host cell is preferably represented by all or part of the sequence represented in SEQ ID No. 2 or one of its derivatives and more preferably by the *pir116* gene. The associated mutation corresponds to the replacement of a proline by a leucine. According to a particular embodiment of the invention, this *pir116* gene is directly incorporated into the host cell genome.

Advantageously, one of the genes of the specific host cell, which is essential under the culture conditions chosen, contains a specific codon which is recognizable by the selected suppressor tRNA in the DNA molecule. According to a preferred mode of the invention, this is an amber TAG codon. In this particular case, the cell may grow, under culture conditions for which the product of the gene containing the TAG codon is essential, only if the plasmid allowing the expression of *sup* is present in the host cell. The culture conditions thus constitute the pressure for selection of the DNA molecule.

Preferably, the gene containing the amber codon is a gene involved in the biosynthesis of an amino acid, arginine. This gene, *argE,* codes for an N-acetylornithinase (Meinnel *et al.,* 1992) and in this case contains a TAG codon corresponding to a point mutation Gln-53 (CAG)-> TAG; the pressure for selection of the plasmid carrying the *sup* gene is then provided by culturing in minimal M9 medium (Maniatis *et al.,* 1989). However, this could also be, for example, a gene for biosynthesis of a vitamin or a nucleic acid base, or alternatively a gene which allows a specific nitrogen or carbon source to be used or any other gene whose functionality is essential for cellular viability under the chosen culture conditions.

The host cell is preferably chosen from *E. coli* strains and is more preferably represented by the strain *E. coli XAC-1.*

According to a specific embodiment of the invention, the host cell used in the claimed process is a cell of the *E. coli* strain *XAC-1,* containing the *pir116* gene in its genome and transformed by the plasmid pXL2774 or one of its derivatives.

According to an advantageous variant of the invention, the host cell used in the process claimed is a prokaryotic cell in which the *endA1* gene or a homologous gene is inactivated. The *endA* gene codes for endonuclease I of *E. coli.* This periplasmic enzyme has a non-specific activity of cleaving double-stranded DNA (Lehman, I. R., G. G. Roussos and E. A. Pratt (1962) J. Biol. Chem. 237: 819-828; Wright M. (1971) J. Bacteriol. 107: 87-94). A study carried out on various strains of Escherichia coli (wild-type or endA) showed that the degradation of plasmid DNA incubated in extracts of these bacterial strains existed in the *endA+* strains but not in the *endA* mutants. (Wnendt S. (1994) BioTechniques 17: 270-272). The quality of the plasmid DNA isolated from *endA+* strains or from *endA* mutants was studied by the company Promega using their purification system (Shoenfeld, T., J. Mendez, D. Storts, E. Portman, B.†Patterson, J. Frederiksen and C. Smith. 1995. Effects of bacterial strains carrying the endA1 genotype on DNA quality isolated with Wizard plasmid purification systems. Promega notes 53). Their conclusion is as follows: the quality of the DNA prepared from *endA* mutants is, overall, better than that of DNA prepared in the *endA+* strains tested.

The quality of the plasmid DNA preparations is thus affected by any contamination with this endonuclease (relatively long-term degradation of the DNA).

The deletion or mutation of the *endA* gene can be envisaged without difficulty insofar as the mutants no longer having this endonuclease activity behave on the whole like wild-type bacteria (Dürwald, H. and H. Hoffmann-Berling (1968) J. Mol. Biol. 34: 331-346).

The *endA1* gene can be inactivated by mutation, total or partial deletion, disruption, etc. Inactivation of the *endA* gene of the *E. coli* strain chosen to produce the pCOR plasmids can be achieved more particularly by transferring, by means of the P1 bacteriophage, the ΔendA::Tc^{R} deletion described by Cherepanov and Wackernagel (Cherepanov, P. P. and W. Wackernagel. 1995. Gene disruption in Escherichia coli: TcR and KmR cassettes with the option of Flp-catalyzed excision of the antibiotic-resistance determinant. Gene 158:9-14) or by exchanging the wild-type allele present in the genome of the bacterium of interest with a mutated or deleted allele of *endA*, by homologous recombination. The use of this type of strain in the context of the present invention makes it possible advantageously to improve the quality of the DNA produced.

The invention also relates to any recombinant cell containing a DNA molecule as defined above. This may be a cell of various origins, of eukaryotic, prokaryotic, etc. type.

According to another embodiment of the invention, the *E. coli* XAC-1 host cell used in the process claimed is designated TEX1, and comprises a *traD* gene, or a homologous gene thereof, inactivated to abolish F' transfer. The *traD* is at the 5' end of one of the *tra* operon and encodes a 81.7 kDa membrane protein that is directly involved in DNA transfer and DNA metabolism (Frost et al., Microbiology Reviews, 1994, 58: 162-210). *traD* mutants do not transfer DNA (Panicker et al., J.Bacteriol., 1985, 162:584-590). The episomal *traD* gene may be inactivated by mutation, total or partial deletion, or disruption using methods well known to those of skill in the art (See Example 9). One method of inactivating this gene is described in Example 1, and the resulting *E. coli* XAC-1 *pir116 endA⁻ traD⁻* strain so obtained is designated TEX1 (Soubrier et al., Gene Therapy, 1999, 6: 1482-1488).

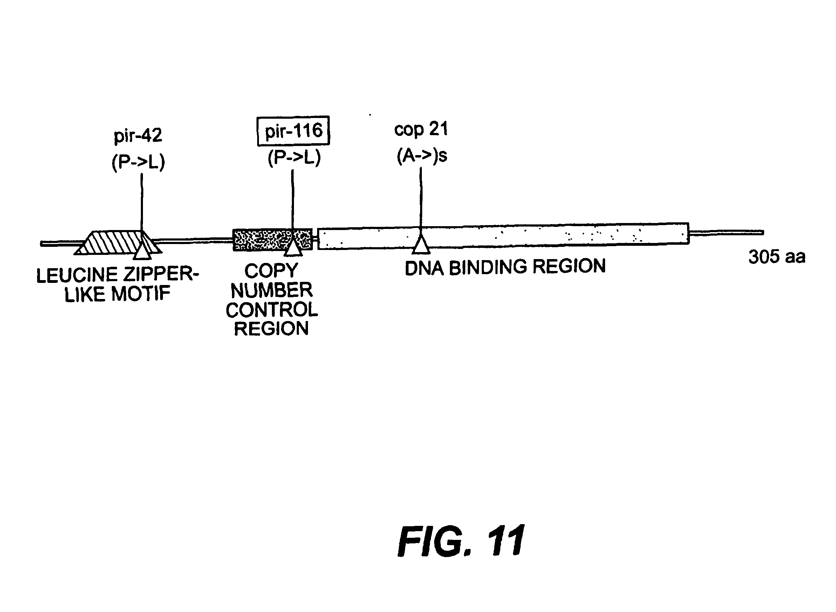

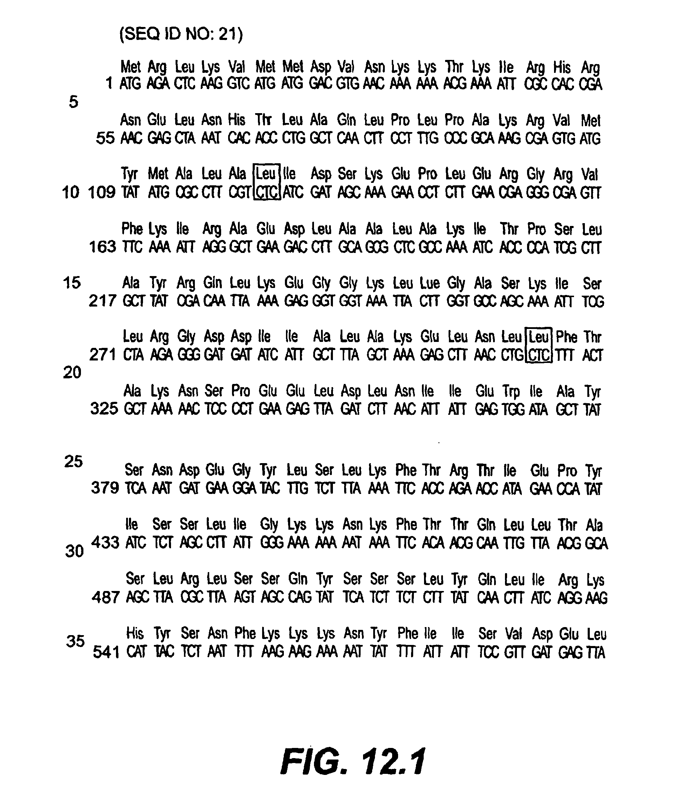

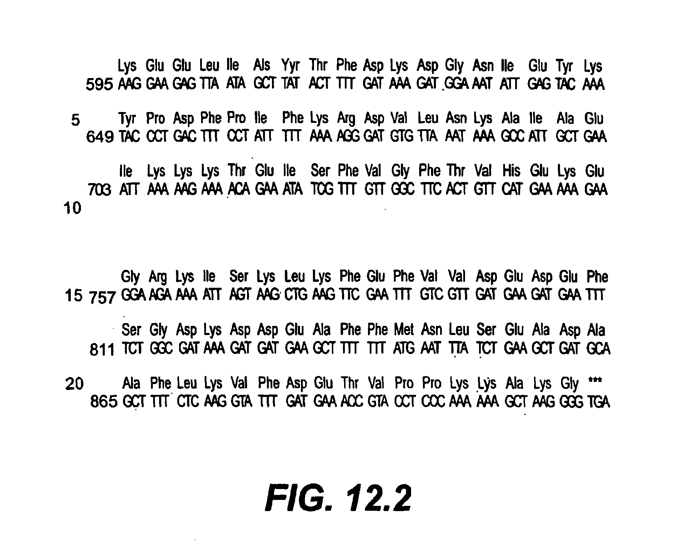

According to one embodiment of the invention, the host cell used in the claimed process is a cell of the *E. coli* strain XAC-1, containing the *pir116* mutation combined with the *pir42* mutation. The *pir116* and *pir42* mutations affect different domains of the pi protein. The *pir116* mutation affects the copy number control region, whereas the *pir42* mutation affects the putative leucine zipper motif, as displayed in Figure 11. The nucleotide and amino acid sequences of the *pir* gene containing the *pir116* and *pir42* mutations are set forth in Figure 12 and SEQ ID NOs: 21 and 22, respectively. The *pir42* mutation comprises a C to T transition at position 124 from the methionine initiator codon, and thus results in substitution of the proline at position 42 by a leucine. The *pir42* mutation was described by Miron et al. (Proc Natl Acad Sci U S A, 1994. 91(14): p. 6438-42; EMBO J, 1992. 11(3): p. 1205-16), and was reported to increase the copy number of an "ori gamma R6K-Km^{R} - *pir42"* plasmid by 2.5 fold as compared to the same plasmid harboring the wild-type *pir* gene. However the *pir42* mutation was never used or described in combination with the *pir116* mutation and while other copy-up mutations such as *cop21* in the *pir* gene combined with the *pir116* do not exhibit an increase of the plasmid copy number, combination of the *pir116* and *pir42* mutations in a *E. coli* XAC-1 *endA⁻ traD⁻* strain surprisingly showed a significant increase of the plasmid copy number. Applicants have thus shown unexpected results of this combination in terms of copy number of the plasmids produced in *E. coli* host strains comprising the mutated *pir116* and *pir42* gene as compared with strains harboring *pir116* alone, or in a host cell comprising the *pir116* mutation combined with another mutation of the *pir* gene, such as the mutation *cop21* (Inuzuka et al., FEBS Lett, 1988. 228(1): p. 7-11). For example, *E. coli* TEX1*pir42* (=XAC-1 *endA⁻ traD⁻ pir116 pir42*) exhibited a 2-5 fold increase in the number of plasmids, as compared to a *pir116* strain, or strains comprising combined *pir116* and *cop21* mutations (See Example 11). In other embodiments, the *pir* gene comprises at least one mutation, which, for example, may occur in the copy number control region, in the leucine zipper-like motif, in the DNA binding region, or in one or more of these regions or another region of the protein pi coded by the *pir* gene.

The prokaryotic host cell according to the present invention also comprises one or more mutations in the same or a different domain of the protein pi, coded by the *pir* gene copy, such as the DNA binding domain, and/or the copy number control region and/or the leucine-zipper motif. The prokaryotic recombinant host cell may comprise the heterologous *pir* gene is in a plasmid or in the genome of the host cell.

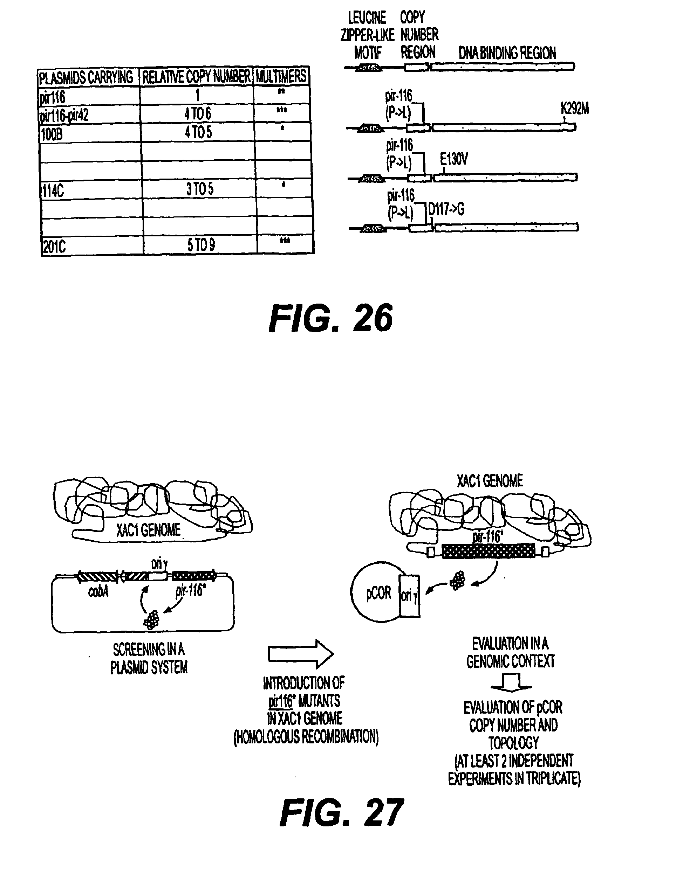

Such mutations may be screened by using the fluorescence-based screening method according to one aspect of the present invention as described thereafter. As shown in the Example 13, host cells comprising at least one mutation in the *pir* gene, the mutation pir116 and a mutation in the DNA binding domain were screened using the fluorescence-based screening method according to the present invention. Host cells comprising mutations present in the DNA binding domain in addition to *the pir116, i.e.,* as for example in the construct 100B, wherein the tyrosine (K) at position 292 is replaced by a methionine (M), in the construct 114C, wherein a glutamic acid (E) at position 130 is replaced by a valine (V), or in the construct 201C, wherein an aspartic acid (D) at position 117 is replaced by a glycine (G) (Fig. 26) are tested for their capacity to produce high copy number of plasmid using the fluorescence-based screening method.

According to another embodiment of the present invention, the host cell used in the process claimed is a prokaryotic host cell in which the *recA* gene or a homologous gene has been inactivated. Preferably, the host cell according to the present invention is *E. coli* strain XAC-1 comprising mutations *pir116, pir42, endA⁻, traD⁻, recA⁻.* Such a strain is designated *TEX2pir42. recA* may be inactivated by methods well known to those in the art. *recA* encodes a major recombination protein and mutations in this gene reduce the frequency of recombination-mediated alteration in plasmids and intramolecular recombination that could lead to the multimerization of plasmids. As described in Example 12, a deleted *recA* gene containing 3 translation stop codons (one in each frame) at its 5' end may be obtained by PCR. The resulting inactivated gene was then introduced by gene replacement into TEX1 genome (Example 12.1).

These cells are obtained by any technique known to those skilled in the art which allows the introduction of the said plasmid into a given cell. Such a technique may be, in particular, transformation, electroporation, conjugation, fusion of protoplasts or any other technique known to those skilled in the art.

Strain XAC-1*pir116* was deposited under the terms of the Budapest Treaty with the Collection Nationale De Cultures de Micro-organismes (CNCM), Institut Pasteur, 28, rue Dr. Roux, 75724 Paris Cedex 15, France, on October 10^{th}, 2003 under accession no. I-3108.

Strain TEX2*pir42* was deposited under the terms of the Budapest Treaty with the Collection Nationale De Cultures de Micro-organismes (CNCM), Institut Pasteur, 28, rue Dr. Roux, 75724 Paris Cedex 15, France, on October 10^{th}, 2003, under accession no. I-3109.

The DNA molecules according to the invention may be used in any application of vaccination or of gene and cell therapy for transferring a gene to a given cell, tissue or organism, or for the production of recombinant proteins *in vitro.*

In particular, they may be used for direct *in vivo* administration or for the modification of cells *in vitro* or *ex vivo,* for the purpose of implanting them into a patient.

In this respect, another subject of the present invention relates to any pharmaceutical composition comprising at least one DNA molecule as defined above. This molecule may or may not be associated therein with a chemical and/or biochemical transfection vector. This may in particular involve cations (calcium phosphate, DEAE-dextran, etc.) or liposomes. The associated synthetic vectors may be cationic polymers or lipids. Examples of such vectors which may be mentioned are DOGS (Transfectam^{™}) or DOTMA (lipofectin^{™}).

The pharmaceutical compositions according to the invention may be formulated for the purpose of topical, oral, parenteral, intranasal, intravenous, intramuscular, subcutaneous, intraocular, or transdermal administrations. The claimed plasmid is preferably used in an injectable form or in application. It may be mixed with any vehicle which is pharmaceutically acceptable for an injectable formulation, in particular for a direct injection to the site to be treated. This may involve, in particular, sterile, isotonic solutions or dry compositions, in particular freeze-dried compositions, which, by addition, depending on the case, of sterilized water or of physiological saline, allow injectable solutions to be made up. This may in particular involve Tris or PBS buffers diluted in glucose or in sodium chloride. A direct injection into the affected region of the patient is advantageous since it allows the therapeutic effect to be concentrated at the level of the affected tissues. The doses used may be adapted as a function of various parameters, and in particular as a function of the gene, the vector, the mode of administration used, the pathology concerned or the desired duration of the treatment.

The DNA molecules of the invention may contain one or more genes of interest, that is to say one or more nucleic acids (synthetic or semi-synthetic DNA, gDNA, cDNA, etc.) whose transcription and, possibly, whose translation in the target cell generate products of therapeutic, vaccinal, agronomic or veterinary interest.

Among the genes of therapeutic interest which may be mentioned more particularly are genes coding for enzymes, blood derivatives, hormones and lymphokines: interleukins, interferons, TNF, etc. (FR 92/03120), growth factors, neurotransmitters or their precursors or synthetic enzymes, and trophic factors (BDNF, CNTF, NGF, IGF, GMF, aFGF, bFGF, NT3, NT5, VEGF-B, VEGF-C etc.; apolipoproteins: ApoAI, ApoAIV, ApoE, etc. (FR 93/05125), dystrophin or a minidystrophin (FR 91/11947), tumour-suppressing genes: p53, Rb, Rap1A, DCC, k-rev, etc. (FR 93/04745), genes coding for factors involved in coagulation: factors VII, VIII, IX, etc., suicide genes: thymidine kinase, cytosine deaminase, etc.; or alternatively all or part of a natural or artificial immunoglobulin (Fab, ScFv, etc.), an RNA ligand (WO 91/19813), etc. The therapeutic gene may also be an antisense sequence or gene, whose expression in the target cell makes it possible to control the expression of genes or the transcription of cellular mRNAs. Such sequences may, for example, be transcribed, in the target cell, into RNAs which are complementary to cellular mRNAs and thus block their translation into protein, according to the technique described in patent EP 140,308. A insert of interest that may be carried by the pCOR of the invention is a RNAi, whose is capable of interfering with the translation of a target gene (Wilson et al., Curr Opin Mol Ther. 2003 Aug;5(4):389-96) and thereby regulating the expression of such gene.

The gene of interest may also be a vaccinating gene, that is to say a gene coding for an antigenic peptide, capable of generating an immune response in man or animals, for the purpose of producing vaccines. These antigenic peptides may in particular be specific antigenic peptides of Epstein-Barr virus, HIV virus, hepatitis B virus (EP 185,573), or pseudorabies virus, or alternatively specific antigenic peptides of tumours (EP 259,212).

Generally, in the DNA molecules of the invention, the gene of therapeutic, vaccinal, agronomic or veterinary interest also contains a promoter region for functional transcription in the target organism or cell, as well as a region located at the 3' end which specifies a transcription termination signal and a polyadenylation site. As regards the promoter region, it may be a promoter region naturally responsible for expression of the gene under consideration when this region is capable of functioning in the cell or the organism concerned. The promoter regions may also be regions of different origin (responsible for the expression of other proteins) or even of synthetic origin. In particular, they may be promoter sequences from eukaryotic or viral genes. For example, they may be promoter sequences obtained from the genome of the target cell. Among the eukaryotic promoters which may be used are any promoters or derived sequence which stimulates or suppresses the transcription of a gene in a specific or non-specific, inducible or non-inducible, strong or weak manner. The eukaryotic promoters may in particular be ubiquitous promoters (promoters of the genes for HPRT, PGK, α-actin, tubulin, etc.), intermediate filament promoters (promoters of the genes for GFAP, desmin, vimentin, neurofilaments, keratin, etc.), therapeutic gene promoters (for example the promoters of the genes for MDR, CFTR, factor VIII, ApoAI, etc.) tissue-specific promoters (promoters of the genes for pyruvate kinase, villin, intestinal fatty acid-binding protein, α-actin of smooth muscle, etc.) or alternatively promoters which respond to a stimulus (steroid hormone receptor, retinoic acid receptor, etc.). Similarly, they may be promoter sequences obtained from the genome of a virus, such as, for example, the promoters of the adenovirus E1A and MLP genes, the CMV early promoter or alternatively the LTR promoter of RSV, etc. In addition, these promoter regions may be modified by addition of activating or regulatory sequences or sequences which allow tissue-specific expression or expression which is predominantly tissue-specific.

Moreover, the gene of interest may also contain a signal sequence which directs the synthesized product into the secretory pathways of the target cell. This signal sequence may be the natural signal sequence of the synthesized product, but it may also be any other functional signal sequence or an artificial signal sequence. Preferred signal sequence used according to the present invention is the secretion signal peptide of human interferon as described Taniguchi et al. (Gene, 1980,233 (4763):541-5)

Depending on the gene of interest, the DNA molecules of the invention may be used for the treatment or prevention of several pathologies, including genetic diseases (dystrophy, cystic fibrosis, etc.), neurodegenerative diseases (Alzheimer's disease, Parkinson's disease, ALS, etc.), cancers, pathologies associated with coagulation disorders or with dyslipoproteinaemias, pathologies associated with viral infections (hepatitis, AIDS, etc.), or in the agronomic and veterinary fields, etc.

According a preferred embodiment, the DNA molecules of the present invention are used for treating critical limb ischemia pathologies such as for example peripheral arterial occlusive disease and intermittent claudication.

Moreover, the present invention also relates to the use of conditional replication DNA molecules for the production of recombinant proteins. Bacteria can be used to produce proteins of various origins, eukaryotic or prokaryotic. Among the bacteria, *E. coli* constitutes the organism of choice for expressing heterologous genes on account of its ease of manipulation, the large number of expression systems available and the large amounts of proteins which can be obtained. It is understood that the system of the invention can be used in other organisms, the tropism being determined by the nature of the origin of replication, as indicated above. For this use, the nucleic acid sequence of interest comprises a coding region under the control of expression signals that are appropriate for the host chosen, in particular a prokaryotic host. These may be, for example, Plac, Ptrp, PT7, Ptrc, Ptac, PL, P_{BAD} or PR promoters, the Shine-Dalgarno sequence, etc. (this set constitutes the expression cassette). The nucleic acid sequence of interest can be any sequence coding for a protein which is of value in the fields of pharmacy, agri-foods, chemistry or agrochemistry. This may be a structural gene, a complementary DNA sequence, a synthetic or semi-synthetic sequence, etc.

The expression cassette can be introduced onto the conditional replication vector which is the subject of the invention, thus constituting a conditional replication vector which allows the expression of proteins of interest in *E. coli.* This vector has several advantages: no use of antibiotic to select it in the bacterium (reduced cost, no need for a study regarding the presence of antibiotic or of potentially toxic derived products in the finished product), virtually no probability of dissemination of the plasmid in nature (conditional origin of replication), possible fermentation in entirely defined medium. The examples given show the advantageous properties of these conditional vectors for the production of recombinant proteins.

As described above, the DNA molecule according to the present invention comprises an origin of replication ORIγ derived from R6K wherein the *pir* gene is removed and is introduced into the genome of a specific host cell that is used for the production of the DNA molecules at large scale. There is always a need to produce increasing quantities of plasmid for clinical trials and/or for use in DNA- based gene therapy. Production host cells have been engineered to carry the pir gene containing at least one mutation, such as the mutation *pir116* and/or *pir42.* Use of such mutated host strain results in an increase of the plasmids copy number and thus significantly raises the yield of production. Also, conformation of the plasmids so produced is very satisfying.

According to a particular aspect, a novel fluorescence-based method of screening for copy-up mutant is provided. This fluorescence-based screening method is far superior to the classical method of screening based on the level of resistance to antibiotic in the bacteria, which may not be used when the basal copy number of plasmid is already very high such as the one obtained using the mutant *pir116, e.g.*, around 400 copies of plasmid per cell. The fluorescence-based method of screening according to the present invention preferably uses the *cobA* gene as red fluorescence reporter gene of copy-up number. The *cobA* gene which is a gene from *Pseudomonas denitrificans* (Crouzet et al., J. Bacteriol. 1999, 172: 5968-79) encodes uro III methyltransferase, an enzyme of the vitamin B12 pathway, which adds two methyl groups to urogen will molecule. Wildt et al. (Nature Biotechnology, vol. 17, 1999, pp1175) has described the use of *cobA* as a fluorescent transcriptional reporter gene for *E. coli,* yeast and mammalian cells. For example, such fluorescent reporter gene was used for the selection of recombinant plasmids containing *E. coli* strains which accumulate fluorescent porhyrinoid compounds due to overexpression of the *cobA* gene encoding the uroIII methyltransferase. When illuminated with UV light, the cells fluoresced with a bright red color (Biotechniques, 1995, vol 19, no. 7, p. 760).

The Applicant has surprisingly found a close correlation between the copy number of plasmid carrying the *cobA* gene and the level of fluorescence from pink to red. The fluorescence-based method of screening of copy-up mutants according to the present invention is thus useful for screening various mutants which can then be evaluated in the genome of the production host cell, such as *E.coli*, or mutants of any genes such as in the *pir* gene, which are inserted in the genome of the production host cell or carried in a plasmid.

In addition to the correlation with the copy number of plasmids, the fluorescence-based method of screening of the present invention is easily and rapidly conducted as it is only requires plating and culturing the transformed host cells overnight and exposing to UV lights, to reveal intensity of the fluorescence produced, thereby deducing directly the number of copy of plasmids in the host cell.

Thus, the present invention provides for a method for detecting a plasmid copy-up mutation comprising:
(a) introducing at least one mutation into a target sequence;
(b) transforming the mutated target sequence into a host cell comprising a plasmid, wherein the plasmid comprises a nucleotide sequence encoding uroIII methyltransferase and the copy number of the plasmid is effected by the target sequence;
(c) growing the host cell under conditions wherein the nucleotide sequence is expressed to produce a culture of host cells;
(d) exposing the culture of host cells to UV light; and
(e) detecting fluorescence produced by the culture of host cells.

According to the present invention, the method further comprises comparing the fluorescence detected in (e) with fluorescence produced by a culture of host cells comprising an non-mutated target sequence.

Preferably, the uroIII methyltransferase gene is coded by the *cobA* gene from *Pseudomonas denitrificans.*

The mutation may be present in a plasmid comprising a heterologous *pir* gene comprising at least one mutation. The plasmid may comprise at least one mutation in the *pir* other regions such as in the copy control region and/or in the DNA binding domain, and/or in the leucine-zipper motif and/or in another region of the pir gene. Also, the plasmid may comprise at least one mutation in the heterologous *pir* gene copy number control region and the leucine zipper-like motif. The plasmid may further comprise a mutation in the *pir* gene DNA binding region. Furthermore, the plasmid may comprise one or more mutations in the same or a different region of the *pir* gene coding for the copy control region and/or the DNA binding region, and/or the leucine zipper-like motif, or other region of the protein Π.

Within limitation, the prokaryotic recombinant host cell according to the present invention comprises the pir116 mutation and a second mutation in the DNA binding region such as *pir292, pir130,* or *pir117* (Fig. 26).

Such mutated production host strain may be advantageously produced using an universal plasmid tool such as the minicircle. The minicircle technology is described inter alia in US patents 6,143,530 and 6,492,164 of the Applicant or in PCT application WO 96/26270.

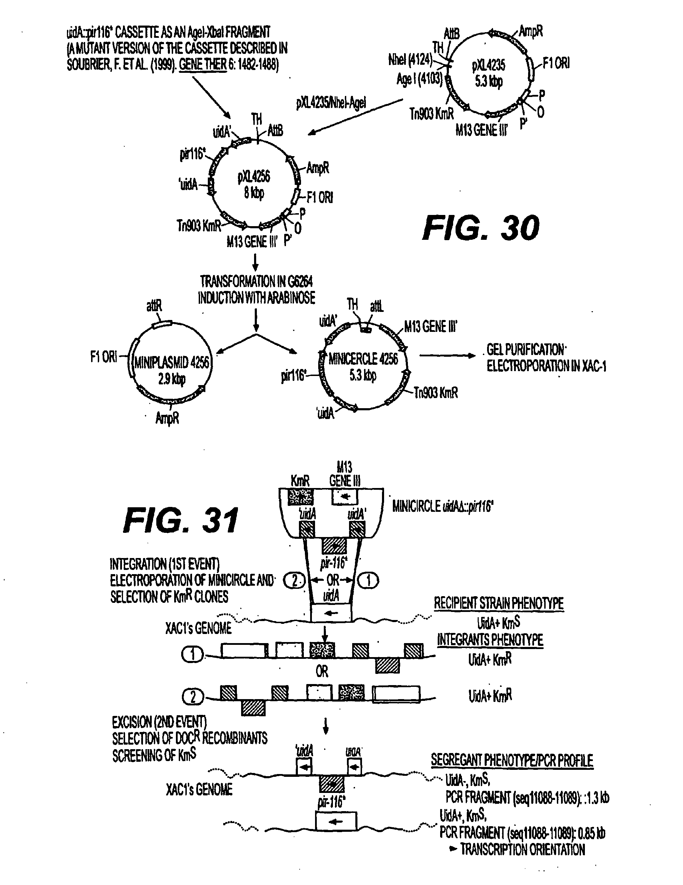

Minicircles are recombinant DNA molecules that do not contain any origin of replication, and thus represent excellent suicide vector for gene replacement of the genome of any microorganisms. In particular, the gene or genes of interest are flanked by the two sequences permitting site-specific recombination, positioned in the direct orientation in the minicircle. The position in the direct orientation indicates that the two sequences follow the same 5'-3' polarity in the recombinant DNA minicircle. The minicircle genetic constructions are generally circular double-stranded DNA molecules devoid of origin of replication, but may also be in linear form and contain the gene or genes of interest flanked by the two sequences permitting site-specific recombination, positioned in the direct orientation. According to this particular embodiment of the invention, the minicircle may be used to transform any competent microorganisms for the purpose of the gene replacement within the genome thereof (Figure 31).

The minicircle for gene replacement is generated from a parent plasmid comprising at least:
a) an origin of replication and, a selection marker gene,
b) two sequences permitting site-specific recombination, positioned in the direct orientation, and,
c) placed between said sequences b), one or more genes of interest.

The specific recombination system present in the genetic constructions can be of different origins. In particular, the specific sequences and the recombinases used can belong to different structural classes, and in particular to the integrase family of bacteriophage λ or to the resolvase family of the transposon Tn3. Among recombinases belonging to the integrase family of bacteriophage λ, there may be mentioned, in particular, the integrase of the phages lambda (Landy et al., Science 197: 1147, 1977), P22 and Φ80 (Leong et al., J. Biol. Chem. 260: 4468, 1985), HP1 of *Haemophilus influenza* (Hauser et al., J. Biol. Chem. 267 6859, 1992), the Cre integrase of phage P1, the integrase of the plasmid pSAM2 (EP 350,341) or alternatively the FLP recombinase of the 2µ plasmid. The minicircles are thus prepared by recombination by means of a site-specific system of the integrase family of bacteriophage λ, the DNA molecules according to the invention generally comprise, in addition, a sequence resulting from the recombination between two *att* attachment sequences of the corresponding bacteriophage or plasmid.

Among recombinases belonging to the family of the transposon Tn3, there may be mentioned, in particular, the resolvase of the transposon Tn3 or of the transposons Tn*21* and Tn*522* (Stark et al., Trends Genet, 8, 432-439, 1992); the Gin invertase of bacteriophage mu, or, alternatively, the resolvase of plasmids, such as that of the *par* fragment of RP4 (Albert et al., Mol. Microbiol. 12: 131, 1994). When the minicircles are prepared by recombination by means of a site-specific system of the family of the transposon Tn3, they generally comprise, in addition to the gene of interest that is aimed to be inserted in a microorganism genome, a sequence resulting from the recombination between two recognition sequences of the resolvase of the transposon in question. Sequences permitting site-specific recombination may also be derived from the *loxP* region of phage P1, which is composed essentially of two repeat sequences capable of recombining specifically with one another in the presence of a protein, designated Cre (Sternberg et al., J. Mol. Biol. 150: 467, 1971). The plasmid used to produce the minicircle thus comprises (a) a bacterial origin of replication and, a selection marker gene; (b) the repeat sequences of bacteriophage P1 (*loxP* region); and (c), placed between said sequences (b), one or more genes of interest that one's wish to insert in a microorganism genome.

Minicircles may comprise sequences permitting site-specific recombination are derived from a bacteriophage, such as attachment sequences (*attP* and *attB* sequences) of a bacteriophage or sequences derived from such attachment sequences. These sequences are capable of recombining specifically with one another in the presence of a recombinase referred to as an integrase with or without an excisionase. The term "sequences derived from such attachment sequences" includes the sequences obtained by modification(s) of the attachment sequences of the bacteriophages that retain the capacity to recombine specifically in the presence of the appropriate recombinase. Thus, such sequences can be reduced fragments of these sequences or, alternatively, fragments extended by the addition of other sequences (restriction sites, and the like). They can also be variants obtained by mutation(s), in particular by point mutation(s). The terms *attP* and *attB* sequences of a bacteriophage or of a plasmid denote, according to the invention, the sequences of the recombination system specific to said bacteriophage or plasmid, that is to say the *attP* sequence present in said phage or plasmid and the corresponding chromosomal *attB* sequence. Attachment sequences are well known in the art, and include *inter alia* the attachment sequences of the phages λ, P22, Φ80, P1, and HP1 of *Haemophilus influenzae* or, alternatively, of plasmid pSAM2 or the 2µ plasmid.

The minicircles are easly produced from the parent plasmid described above. The method for the production of the minicircle consists in bringing into contact culture of cells that are transformed with the parent plasmid with the integrase with or without the excisionase, so as to induce the site-specific recombination. The culture and the integrase with or without the excisionase are brought into contact either by transfection or infection with a plasmid or a phage containing the gene for said integrase and when applicable the gene for the excisionase. Alternatively, for example, the expression of genes coding for said integrase and when applicable the excisionase, present in the host cell, are induced. As mentioned below, these genes may be present in the host cell in integrated form in the genome, on a replicative plasmid, or, alternatively, on the plasmid of the invention, in the non-therapeutic portion.

To permit the production of the minicircles according to the invention by site-specific recombination *in vivo,* the integrase with/without the excisionase used are introduced into, or induced in, cells or the culture medium at a particular instant. For this purpose, different methods may be used. According to a first method, a host cell is used containing, for example, the recombinase gene, *i.e.,* the integrase gene with or without the excisionase gene, in a form permitting its regulated expression. The integrase gene with or without the excisionase gene may, for example, be introduced under the control of a promoter, or of a system of inducible promoters, or, alternatively, in a temperature-sensitive system.

In particular, the integrase gene may be present in a temperature-sensitive phage, latent during the growth phase, and induced at a suitable temperature (for example, lysogenic phage λ Xis⁻ *cI*857).

Alternatively, the gene may be under the control of a regulated promoter, for example, the placUV5 promoter, the host cell is designated E.coli G6191.

Preferably, the integrase with or without the excisionase gene may be under the control of a regulated promoter, for example the P_{BAD} promoter of the araBAD (arabinose) operon, which is regulated by arabinose (Guzman et al., J. Bacteriol, 1995, 4121-4130; US5,028,530). Particularly, use of P_{BAD} promoter allows sufficient expression of excisionase and integrase in presence of arabinose, as the inducing agent, and thus more than 90% of recombination of the plasmids which are present in high copies number in the bacteria, whereas in absence of arabinose, the promoter is tightly inhibited. The cassette for expression of the integrase with/without excisionase may be carried by a plasmid, a phage, or even by the plasmid of the invention in the non-therapeutic region. It may be integrated in the genome of the host cell or maintained in replicative form. Such host cells are in particular E.coli G6264 and E.coli G6289. According to another method, the cassette for expression of the gene(s) is carried by a plasmid or a phage used to transfect or infect the cell culture after the growth phase. In this case, it is not necessary for the gene to be in a form permitting its regulated expression. In particular, any constitutive promoter may be used. The DNA may also be brought into contact with the integrase and when applicable the excisionase *in vitro,* on a plasmid preparation, by direct incubation with the protein.

The minicircle so produced thus comprises an expression cassette containing one or more genes of interest to be inserted in the targeted microorganism, lacks an origin of replication and comprises a sequence attR resulting from site-specific recombination between an attB and an attP sequence, or a sequence attL resulting from site-specific recombination between an attB and an attP sequence. The minicircle may thus be used as universal suicide vector for gene replacement in any microorganisms. In effect, the minicircle carrying a gene for replacement flanked by homologous sequences and a antibiotic resistance gene will easily integrate in a targeted site of the genome of any microorganism by homologous recombination as represented in Figure 31. A second event of excision which may be triggered by a second selection pressure may then efficiently select the microorganisms only carrying the new inserted gene within their genome.

The present invention thus also relates to a method of gene engineering of a microorganism. This novel method may used to engineer any microorganism regardless of their origin. In effect, the minicircle does not contain any origin of replication, and thus can be used universally for gene replacement in any types of microorganisms. This method represents an advantageous alternative to the use of the bacteriophage M13 for gene replacement by double homologous recombination in a micro-organism.

According to a particular embodiment of the present invention, the minicircle comprises a first selectable marker such as an antibiotic resistance gene, allowing selecting for the first recombination event. Preferred second selectable marker is the gene III or the functional deleted gene III'. The gene III or its functional variant is capable of conferring sensibility to deoxycholate as described in Boecke et al. (Mol. Gen. Genet., 186, 185-92, 1982) and thus allows for counter-selecting the second event of recombination (Fig. 31). The method thus consists in introducing the minicircle into the microorganism by any transformation method well known in the art, and preferably by electroporation, selecting the event of integration of the minicircle in a culture supplemented with an antibiotic or under another pression of selection, and selecting a second event of excision by treating with deoxycholate or another appropriate pression of selection.

The present invention will be described more fully with the aid of the examples which follow, which should be considered as non-limiting illustrations.

### Brief description of the drawings:

Figure 1: Functional organization of the region of R6K involved in replication.
Figure 2: Organization of the functional domains of the Π protein of the plasmid R6k.
Figure 3: Representation of the protocol for introducing the *pir* gene into the genome of *E. coli* XAC1.
Figure 4: Construction scheme for vectors pXL2666, 2730 and 2754.
Figure 5: Construction ofpXL2774.
Figure 6: Growth and production kinetics in a 2L fermenter.
Figure 7: Growth and production kinetics in an 800L fermenter.
Figure 8: Construction of pXL3056.

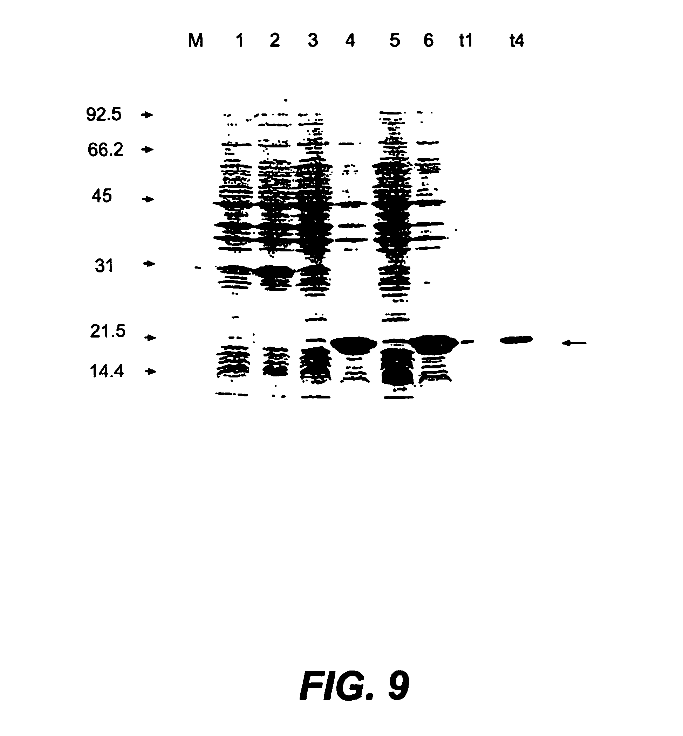

Figure 9: Visualization of the aFGF protein produced by *E. coli* XAC-1*pir116* (pXL3056+PT7po123) after induction. The denatured total cell extracts are deposited on 12.5%-SDS polyacrylamide gel. M: molecular mass marker (Biorad, Low range). Each band is identified by an arrow and a figure which indicates its mass in kDaltons. 1: XAC-1*pir116* (pXL3056+pUC4K) not induced; 2: XAC-1*pir116* (pXL3056+pUC4K) induced at 42°C; 3: XAC-1*pir116* (pXL3056+PT7po123) clone 1, not induced; 4: XAC-1*pir116* (pXL3056+PT7po123) clone 1, induced at 42°C; 5: XAC-1*pir116* (pXL3056+PT7po123) clone 2, not induced; 6: XAC-1*pir116* (pXL3056+PT7po123) clone 2, induced at 42°C; t1: 1 µg of purified aFGF; t4: 4 µg of purified aFGF.
Figure 10: Schematic representations for vectors pXL3029, pXL3030, and pXL3179 or NV1FGF.
Figure 11: Schematic representation of the functional domains of R6K π initiator proteins.
Figure 12: Nucleotide and amino acid sequences of the *pir* gene comprising the *pir116* and *pir42* mutations.
Figure 13: Construction of *pir116pir42* suicide vector for homologous recombination.
Figure 14: Schematic representation of the PCR products obtained when amplifying the region *uidA::pir116 +*/*- pir42.*
Figure 15: Agarose gel electrophoresis showing the topology of pCOR plasmid pXL3179 produced in TEX1 or *TEX1pir42.*
Figure 16: Schematic representation of the pXL3749 suicide plasmid carrying *pir-116cop21* gene.
Figure 17: Agarose gel electrophoresis showing the plasmid copy number of pXL2979 when produced in *E. coli* host cell TEX1*cop21* (lines 1-4), in *E. coli* host cell XAC1*pir* (lines 5-8), in *E. coli* TEX1 (lines 9-12).
Figure 18: Representation of the cloning strategy for the construction of the *recA-*suicide vector.
Figure 19: Schematic representation of the PCR products obtained when amplifying regions of *E. coli* TEX2 strain.
Figure 20: Agarose gel electrophoresis showing the topology of pCOR pXL3179 produced in *E. coli* TEX2*pir42* (line B), in *E. coli* TEX1*pir42* (line C), in *E. coli* TEX1 (line D).
Figure 21: Analysis of plasmid pXL3179 produced by fermentation in *E. coli* TEX2*pir42.*
Figure 22: Fluorescence-based assay showing that fluorescence increases with plasmid copy number.
Figure 23: Diagram of plasmids screened in the fluorescence-based assay.
Figure 24: Diagram of plasmid pXL3830.
Figure 25: Agar plate demonstrating fluorescence-based screening for copy-up mutants generated by random mutagenesis.
Figure 26: Evaluation of copy-up mutants identified by the fluorescence-based screening method.
Figure 27: Diagram of the strategy for evaluating *pir116* mutants inserted into the bacterial genome.
Figure 28: Evaluation of pXL3179 copy number in different *pir116** mutant *E. coli* strains.
Figure 29: Construction of a plasmid used to generate minicircle vectors for homologous recombination in *E. coli.*
Figure 30: Construction of a minicircle vector used to generate *pir116** mutant *E. coli* strains.
Figure 31: Diagram of gene replacement by homologous recombination using a minicircle vector.
Figure 32: Demonstration of double recombinant clones grown on medium containing sodium deoxycholate.
Figures 33A and B: The results of control PCR on double recombinants.

### I - MATERIALS AND METHODS

### A) Materials

### 1) Culture media

Complete LB, 2XTY and SOC media and minimal M9 medium (Maniatis *et al.,* 1989) were used. Agar media were obtained by addition of 15 g of Difco agar. Furthermore, if necessary, these media were supplemented with antibiotics (ampicillin or kanamycin) at respective concentrations of 100 mg/l and 50 mg/l. The chromogenic substrates X-Gal and X-Gluc were used at a concentration of 40 mg/l.

### 2) E. coli strains, plasmids and bacteriophages

The *E. coli* strains, plasmids and bacteriophages used are respectively identified in the examples below.

### B) Methods

### 1) Manipulation of the DNA

The isolation of bacterial DNA (plasmid and genomic) and phage DNA (replicative form of M13), digestion with restriction endonucleases, ligation of the DNA fragments, agarose gel electrophoresis (in TBE buffer) and other standard techniques were carried out according to the manufacturers' recommendations, for the use of enzymes, or in accordance with the procedures described in "Molecular Cloning: a Laboratory Manual" (Maniatis et al., 1989).

The DNA size markers used during the electrophoreses are as follows: 1 kb ladder (BRL) for the linear fragments and the supercoiled DNA marker (Stratagene) for the undigested plasmids.

Sequencing was carried out according to the Sanger technique (Sanger *et al.*, 1977) adapted to the automated method using fluorescent dideoxynucleotides and Taq DNA polymerase (PRISM Ready Reaction DyeDideoxy Terminator Cycle Sequencing Kit, Applied Biosystems).

The oligodeoxynucleotides used (designated by "seq+no.", see below) were synthesized on the "Applied Biosystems 394 DNA/RNA Synthesizer" by the phosphoramidite method, using α-cyanoethyl protecting groups (Sinha *et al.*, 1984). After synthesis, the protecting groups are removed by treatment with ammonia. Two precipitations with butanol allow the oligonucleotide to be purified and concentrated (Sawadogo *et al.*, 1991).

### Sequences of the oligonucleotides used for the PCR amplification:

SEQ ID No. 3 5'-GACCAGTATTATTATCTTAATGAG-3'
SEQ ID No. 4 5'-GTATTTAATGAAACCGTACCTCCC-3'
SEQ ID No. 5 5'-CTCTTTTAATTGTCGATAAGCAAG-3'
SEQ ID No. 6 5'-GCGACGTCACCGAGGCTGTAGCCG-3'

The PCR reactions (Saïki *et al.,* 1985) were performed under the following conditions, in a total volume of 100 µl. The reaction mixture comprises 150 ng of genomic DNA from the strain to be studied, 1 µg of each of the two oligonucleotide primers (24-mer), 10 µl of 10XPCR buffer, the composition of which is as follows "500 mM KCl, 0.1 % gelatin, 20 mM MgCl₂, 100 mM Tris-HCl pH 7.5", and 2.5 units of Taq DNA polymerase (Amplitaq Perkin-Elmer). The PCR conditions, on the Perkin-Elmer Cetus DNA Thermal Cycler machine are as follows: 2 min at 91°C, 30 successive cycles of denaturation (1 min at 91°C), hybridization (2 min at 42°C) and elongation (3 min at 72°C), and finally 5 min at 72°C. The products thus obtained, which are or are not digested with a restriction enzyme, are analysed by agarose gel electrophoresis.

Analysis of the various plasmid species by DNA topoisomerases was performed according to the following procedure: the enzymes, purified in the laboratory, are incubated for 1 hour at 37°C. The reaction mixtures (total volume: 40 µl) have the following composition: 150 ng of plasmid, 300 ng of DNA topoisomerase I or 150 ng of E. coli DNA gyrase, or 160 ng of S. aureus DNA topoisomerase IV and 20 µl of buffer specific for each enzyme. The composition of these buffers is indicated below:
for DNA topoisomerase I:
   50 mM Tris-HCl pH 7.7, 40 mM KCl, 1 mM DTT, 100 µg/ml BSA, 3 mM MgCl₂, 1 mM
      EDTA;
for DNA topoisomerase IV:
   60 mM Tris-HCl pH 7.7, 6 mM MgCl₂, 10 mM DTT, 100 µg/ml BSA, 1.5 mM ATP, 350 mM potassium glutamate;
for DNA gyrase:
   50 mM Tris-HCl pH 7.7, 5 mM MgCl₂, 1.5 mM ATP, 5 mM DTT, 100 µg/ml BSA, 20 mM
      KCl.

### 2) Transformation of E. coli

This was performed routinely according to the TSB (Transformation and Storage Buffer) method described by Chung and Miller (1988). For a strain such as TG1 (Gibson *et al.*, 1984), the transformation efficiency obtained is about 10⁵-10⁶ transformants per µg of pUC4K (Vieira and Messing; 1982). When a higher transformation efficiency was necessary, the bacteria were transformed by electroporation according to the procedure recommended by the electroporator manufacturer (Biorad). This method makes it possible to achieve efficiencies of from 10⁸ to 10¹⁰ transformants per µg of pUC4K.

### 3) Cellular transfection mediated by a cationic lipofectant

The cells used are NIH 3T3 mouse fibroblasts seeded the day before into 24-well plates, at a density of 50,000 cells per well. The culture medium used is DMEM medium, containing 4.5 g/l of glucose and supplemented with 10 % fetal calf serum and 1 % of solutions of 200 mM glutamine and antibiotics (5.10³ µ/ml streptomycin, 5.10³ µg/ml penicillin) (Gibco). The plasmid DNA (1 µg in 25 µl of 9 ‰ NaCl) is mixed, on a volume-for-volume basis, with a suspension of lipofectant. Four "lipofectant charges/DNA charges" ratios are tested: 0, 3, 6 and 9. These ratios are calculated by considering that 1 µg of plasmid DNA carries 3.1 nmol of negative charges and that the lipofectant contains 3 positive charges per molecule. After a contact time of 10 minutes to allow formation of the DNA/lipid complex, 50 µl of DNA-lipofectant mixture are introduced onto the cells in serum-free culture medium (500 µl). The cells were prerinsed twice with this same medium. Inhibition of transfection by the serum is thus avoided. After incubation (2 hours at 37°C in the CO₂ incubator), 10 % fetal calf serum is added to the medium. The cells are then reincubated for 24 hours.

### 4) Measurement of the luciferase activity of eukaryotic cells

This is carried out 24 hours after the transfection. Luciferase catalyses the oxidation of luciferin in the presence of ATP, Mg²⁺ and O₂, with concomitant production of a photon. The total amount of light emitted, measured by a luminometer, is proportional to the luciferase activity of the sample. The reagents used are supplied by Promega (luciferase assay system) and used according to the recommended procedure. After lysis of the cells, the insoluble fraction from each extract is eliminated by centrifugation. The assay is carried out on 5 µl of supernatant, which may or may not be diluted in the cell lysis buffer.

### 5) Measurement of the protein concentration in the cell extracts

This is carried out according to the BCA method (Pierce) using bicinchoninic acid (Wiechelman *et al.,* 1988). The standard BSA range is prepared in the lysis buffer (cf. III-B-4). The samples to be assayed and those of the range are pretreated, on a volume-for-volume basis, with 0.1 M iodoacetamide/0.1 M Tris buffer, pH 8.2, for 1 hour at 37°C. This treatment makes it possible to prevent interference, during the assay, of the reducing agent (DTT) present in the lysis buffer. The assay result is read at 562 nm.

### EXAMPLE 1: Construction of XAC-1 pir and pir116 host strains by homologous recombination

The strain used was the *E. coli* strain XAC-1 (Normanly *et al.,* 1980). The *argE* gene of this strain advantageously includes a mutation of glutamine-53 (CAG) into the amber codon (TAG) (Meinnel *et al.*, 1992). The *argE* gene belongs to the *argECBH* divergent operon and codes for an arginine biosynthesis enzyme, N-acetylornithinase. XAC-1 cannot therefore synthesize arginine and, consequently, grow in minimal medium. This auxotrophy will be relieved if the strain harbors a plasmid which allows the expression of a suppressor tRNA. It will thus be possible, by culturing in minimal medium, to select bacteria that carry such a plasmid. In order to allow the replication therein of plasmids derived from R6K, it was necessary to introduce, by homologous recombination, the *pir* gene into the genome of XAC-1. The *pir* gene (wild-type or mutated) is introduced at the *uidA* locus by exchange between the wild-type *uidA* gene and a copy interrupted by the *pir* (or *pir116*) gene. The *uidA* gene codes for β-glucuronidase, the enzyme for hydrolysis of β-glucuronides. This gene may be inactivated without any problem since it is not essential for growth in standard synthetic media, in which β-glucuronides are not used. Furthermore, the β-glucuronidase activity can be monitored by means of a chromogenic substrate, X-Gluc, whose hydrolysis releases a blue pigment.

### 1) Construction of a suicide vector carrying the cassette "Km^{R}-uidA::pir (or pir116)

We used a strategy involving a single bacterial host and minimizing the modifications to the genome of the strain of interest. The phage M13mp10 (Messing et Vieira; 1982) was used as a suicide vector (Blum *et al.*, 1989). An amber mutation in the gene II, which is essential for replication, reduces the host spectrum of this M13 to the strains, such as TG1 (*supE*), which produce an amber suppressor tRNA; it will therefore not be able to replicate in *E. coli sup+* strains, such as XAC-1.

The 3.8 kb *Bam*HI cassettes, containing the kanamycin-resistance gene of Tn5 and *_uidA::pir* or *pir116*, were respectively purified from M13wm34 and 33 (Metcalf *et al.*, 1994). They were cloned into M13mp10 linearized with *Bam*HI. The recombinant clones were selected by plating on LB agar medium supplemented with kanamycin, after electroporating the ligation mixtures into TG1. The conformity of the clones obtained was shown by analysing the restriction profile and by sequencing the region corresponding to the *pir116* mutation.

### 2) Introduction of the pir or pir116 genes into the genome of E. coli XAC-1 by homologous recombination

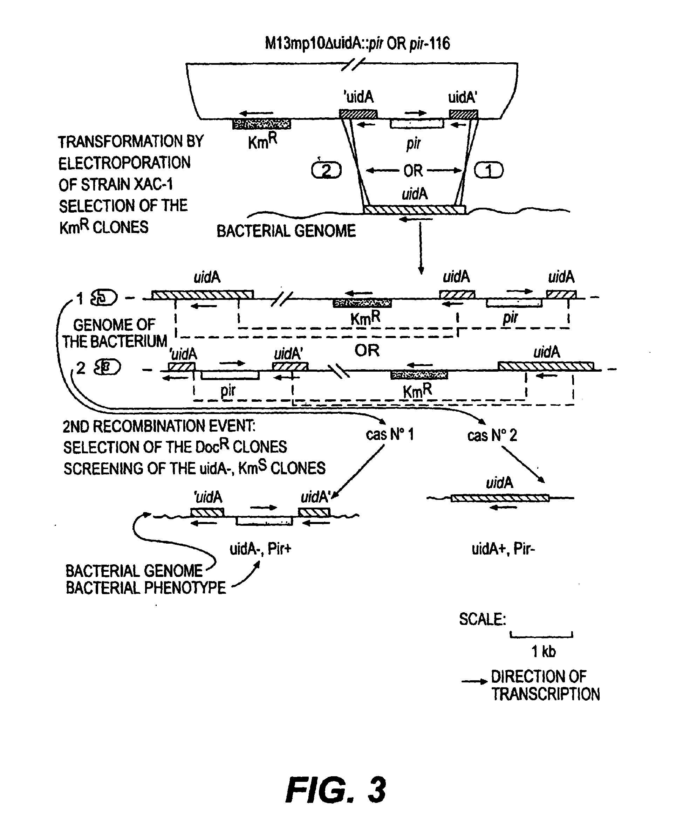

The strategy adopted and the various events involved are presented in Figure 3.

### a) First recombination event

The XAC-1 strain was transformed by electroporation with 10, 100 or 2000 ng of each RF (*mp10-_uidA::pir* or *pir116*). One-third of each expression mixture was plated out on LB plates containing kanamycin and incubated overnight at 37°C. The mp10-_*uidA::pir* or *pir116* phages cannot replicate in the strain XAC-1 (*sup*+). The kanamycin resistance ("Km^{R}") marker can therefore only be maintained by integration into the genome of the bacterium via a homologous recombination with the wild-type copy of the gene *uidA.* The results of the electroporations of XAC-1 are presented in Table 1. The transformation efficiency obtained was 4.10⁹ transformants per µg of pUC4K.

**TABLE 1**

| **CONSTRUCT** | **Number of colonies obtained with the amounts of DNA transformed** | | |
|---|---|---|---|
| | **10 ng** | **100 ng** | **2000 ng** |
| M13mp10-_*uidA::pir* | 1 | 41 | 146 |
| M13mp10-_*uidA::pir116* | 0 | 16 | 124 |

Under the test conditions, the number of integrants increased in a non-linear manner with the amount of DNA. Given the transformation efficiency and the size of the RFs (11.7 kbp), it was possible to have an approximate idea of the level of recombination. By considering the point at 100 ng, a recombination frequency of about 10⁻⁶ was obtained.

### b) Second recombination event

The second recombination event will then be selected by the resistance of the strains to deoxycholate ("Doc^{R}").

To do this, five integrants of each construct were cultured in 2XTY medium supplemented with 0.2 % sodium deoxycholate. Two distinct populations appeared. Certain clones gave quite visible cloudiness after about 8 hours at 37°C (two clones for the *pir* construction and three for the *pir116* construction). The other clones gave a dense culture only after one night at 37°C. They were virtually all sensitive to kanamycin ("Km^{s}"), as expected. For each of the electroporants studied, 50 Km^{s} descendants were streaked onto LB medium supplemented with X-Gluc. After 48 hours at 37°C, the UidA⁺ clones were pale blue whereas those which had undergone an allele replacement (case No. 1, Figure 3) remained white on this medium (UidA⁻). Table 2 summarizes the phenotypic analysis of the double recombinants obtained. From 18 to 30 % of the double recombinants underwent an allele replacement.

**TABLE 2**

| **Strain** | **Number of Km^{S} among the Doc^{R}** | **Percentage of UidA⁻ among the Km^{S}** |
|---|---|---|
| XAC-1 *pir-2* | 50/50 | 18 |
| XAC-1 *pir-3* | 50/50 | 24 |
| XAC-1 *pir-4* | 50/50 | 34 |
| XAC-1 *pir116-1* | 50/50 | 32 |
| XAC *pir116-4* | 35/50 | 30 |

### 3) Checking the Pir+ character nature of the strains obtained by recombination

To ensure the *Pir*+ character of the strains obtained by double recombination, we transformed three clones of each construct with pBW30 (Metcalf *et al.,* 1994). The fact that transformants were obtained for all the test strains made it possible to show the functionality of the *pir* and *pir116* genes, which were integrated into the genome of XAC-1. Under the same conditions, no transformant was obtained with the parental strain XAC-1. We continued to study two XAC-1*pir* clones (B and C) and two XAC-1*pir116* clones (E and D).

### 4) Checking, by PCR amplification, of the strains obtained by recombination

To confirm the allele replacement, we checked the genomic regions on either side of the *uidA* locus by PCR amplification. Each pair of oligonucleotides consisted of an oligonucleotide corresponding to an internal region of *pir* and a second oligonucleotide corresponding to a region, close to chromosomal *uidA*, but not within the fragment which served for the recombination. The sequence of the latter oligonucleotide was determined by means of the ECOUIDAA sequence from Genbank (access number: M14641). We were thus able to verify the exact location of the *pir* gene in the bacterial genome. The nature of the amplified fragments, whose size is in accordance with that which might be expected, was confirmed by digestion with *Mlu*I.

### EXAMPLE 2: Construction of plasmid vectors derived from R6K carrying the selection marker sup Phe

Vectors were constructed containing ori γ from R6K and the kanamycin-resistance gene (pXL2666). The observation of pXL2666 multimers in the strain BW19610 (*pir116*) 5 (Metcalf *et al.,* 1993) led us to study the effect of the *cer* fragment from ColE1 on this phenomenon. We then introduced the expression cassette of the phenylalanine suppressor tRNA *(sup* Phe) onto the vector ori γ -Km^{R}-cer (pXL2730). This vector, pXL2760, serves as a basis for the construction of vectors which can be used in gene therapy.

### 1) Construction and analysis of vectors containing ori γ from R6K and the kanamycin resistance gene

### a) Constructs

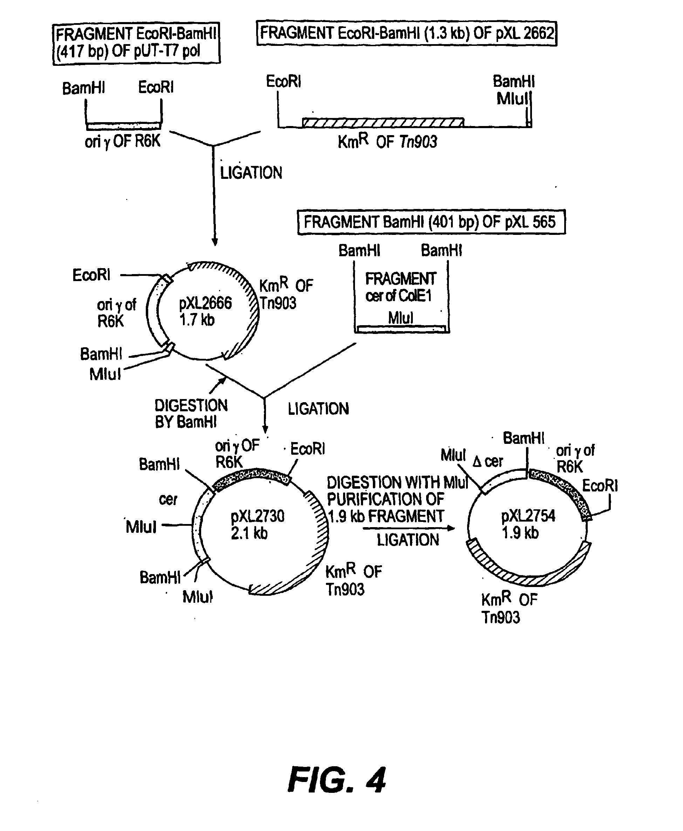

In the first plasmid constructed, pXL2666, the kanamycin resistance gene originated from pUC4K (Vieira and Messing; 1982) and the origin of replication, contained in a 417 bp *Eco*RI*-Bam*HI fragment, originated from the suicide vector pUT-T7pol (Herrero *et al.,* 1990) (Figure 4). The transfer of pXL2666 into the strains BW19094 and 19610 (Metcalf *et al.,* 1994) made it possible to show that the amount of plasmid is indeed increased in *a pir116* strain, when compared with the same plasmid in a *pir* strain. However, electrophoretic analysis of the undigested plasmids showed that this increase goes hand in hand with the appearance of a few multimeric forms. This phenomenon is quite probably associated with intermolecular recombination between the multiple copies of the plasmid. Thus, we constructed pXL2730 by cloning the *cer* fragment of the natural *E. coli* plasmid, ColE1, which had been shown to permit, in *cis,* the resolution of plasmid dimers (Summers and Sherrat, 1984), into pXL2666. The fragment used corresponds to a 382 bp *Hpa*II fragment from ColE1 (Leung *et al.,* 1985). It contains a specific intermolecular recombination site; in order to function, it involves only host proteins including the recombinases XerC and XerD and the accessory factors ArgR and PepA (Stirling *et al.,* 1988, 1989; Colloms *et al.,* 1990). To ensure that the effects observed are indeed due to the *cer* fragment, we also constructed the control plasmid pXL2754, in which the *cer* fragment has a 165 bp deletion. This deletion was shown to abolish the action of *cer* on the resolution of the multimers (Leung *et al.,* 1985). The various cloning steps leading to the construction of these plasmids are presented in Figure 4.

### b) Quantitative and qualitative analysis of the plasmid species

### (i) analysis by agarose gel electrophoresis

Electrophoretic analysis of the different plasmids constructed allowed the demonstration of various plasmid species, which are variable according to the strains used. The size of the undigested plasmids was evaluated relative to a supercoiled DNA marker. In the *pir* strain (BW19094), the plasmids pXL2666, 2754 and 2730 were almost entirely in monomeric form. The bands above each main band correspond to various slightly less supercoiled topoisomers, as confirmed by the profile observed after the action of DNA gyrase on pXL2730.

In the case of the *pir116* strain (BW19610), the profiles were different: with the plasmids pXL2666 and 2754 different species were observed ranging from the monomer to multimers (2, 3 or 4 units), the major form being the dimer. After digestion with EcoRI, only the linear plasmid DNA was found; these plasmid species correspond either to plasmid multimers or to various topoisomers. However, since the size of the forms determined according to the supercoiled DNA marker was a whole product of that of the monomer plasmid, it is highly probable that they are multimers. The formation of multimers was most probably attributable to the *pir116* mutation, although the two strains BW19094 and BW19610 are not strictly isogenic (BW19610 is *recA).* The profile obtained with pXL2730 was different: although multimeric forms were still visible, the major form is the monomeric form. The *cer* fragment can thus facilitate resolution of the plasmid multimers which we have constructed, independently of *recA,* in BW19610.

### (ii) analysis after treatment with DNA topoisomerases

To disprove the theory that the forms observed in the strains carrying the *pir116* allele are specific topoisomers, each plasmid preparation was subjected to the action of DNA topoisomerases. The activities of the various enzymes under the experimental conditions were as follows: relaxing of DNA for *E. coli* DNA topoisomerase I, negative supercoiling of relaxed DNA for *E*. *coli* DNA gyrase, and disentanglement of interlaced DNAs and relaxation of supercoiled DNA by *S*. *aureus* DNA topoisomerase IV. The action of DNA topoisomerase IV made it possible to show that the high-molecular-weight plasmid forms did not result from the entanglement of several plasmid molecules; in this case, they would then have been converted into the monomeric species. The functionality of the enzyme was, of course, checked on a preparation of kinetoplast DNA, composed of entangled DNA molecules (not shown). The relaxation activity was also visible since species are obtained which migrate less than in the untreated controls. The action of DNA gyrase made it possible to convert the slightly relaxed topoisomers into the more supercoiled species extracted from the bacterium (monomer or dimer mainly). Furthermore, it made it possible to verify that the DNAs prepared were mainly in supercoiled form. The samples thus treated allowed the above results to be confirmed as regards the major species for each construct. DNA topoisomerase I did indeed relax DNA, but only partially. This could be due to the fact that the plasmids studied contain only a few single-stranded regions, to which this enzyme preferably binds (Roca, 1995).

### 2) Introduction of the selection marker sup Phe into pXL2730

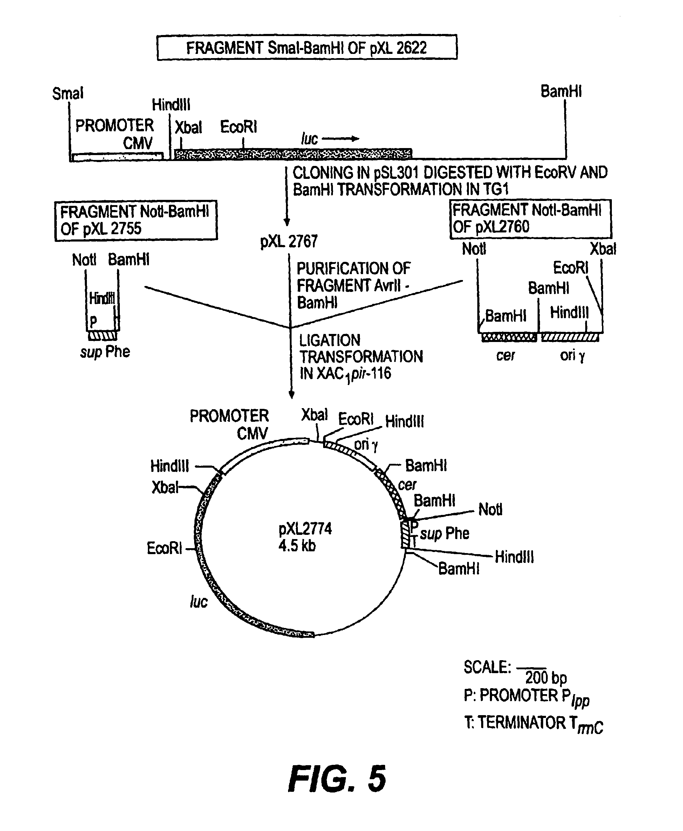

We used the expression cassette of the synthetic suppressor tRNA gene (Phe) (Kleina *et al.,* 1990). This introduced a phenylalanine into the growing polypeptide chain in response to a TAG codon. Furthermore, it allowed the production in XAC-1 of an ArgE protein that was sufficiently active to allow good growth in arginine-deficient medium. *sup* Phe was expressed constitutively on the plasmid pCT-2-F (Normanly *et al.,* 1986) from a synthetic promoter derived from the promoter sequence, *Plpp*, of the *E. coli lpp* gene. Downstream of this gene, transcription was stopped by the synthetic terminator, T*_{rmC}*, of the *E. coli* operon *rrnC* (Normanly *et al.,* 1986). The various cloning steps are indicated in Figure 5.

The various subclonings were performed in XAC-1. The functionality of the suppressor tRNA expression cassette was thus checked by means of the α-galactosidase activity of this strain, which only exists if there is suppression of the amber codon of the gene lacZᵤ₁₁₈ₐₘ. The final step consists of the introduction of the *sup* Phe expression cassette into pXL2730. The results obtained with the *cer* fragment (B-1-*b*) led us to select this plasmid rather than pXL2666. We retained the kanamycin resistance gene for ease of subsequent cloning, in particular in order to have available additional screening during the final cloning (loss of Km^{R}).

### EXAMPLE 3: Validation of the plasmid vector for applications in gene therapy by transfection of mouse fibroblasts

### 1) Construction of the reporter vector pXL2774

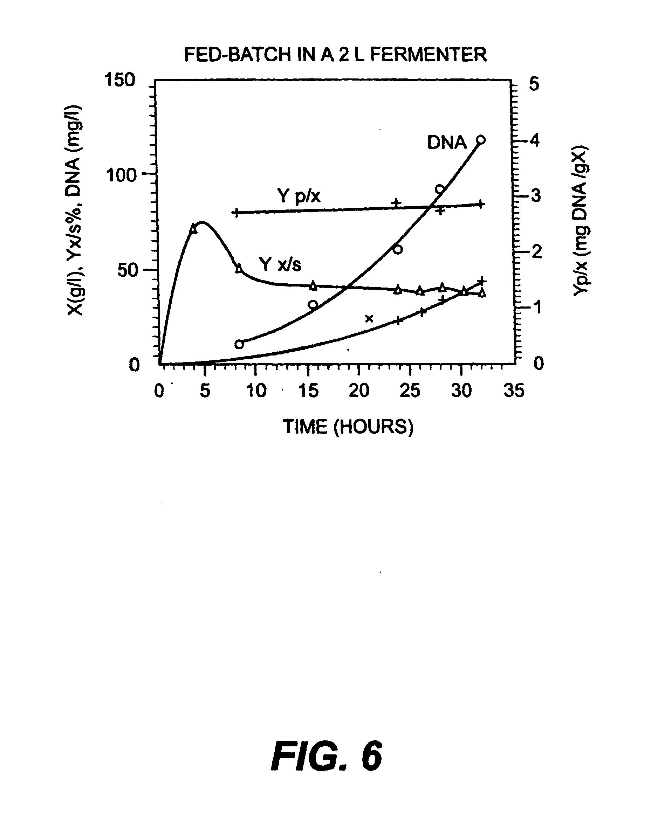

To test the validity for gene therapy of the system for producing plasmid DNA, we introduced a reporter gene, which can be used in eukaryotic cells, into pXL2760. We used the gene *luc,* which codes for *Photinus pyralis* luciferase, since the bioluminescence measurement test is very sensitive and is linear over a large range, and the background noise due to the endogenous activity of eukaryotic cells is very low. The *luc* gene was controlled by promoter-enhancer sequences of a human cytomegalovirus early gene (CMV promoter), which allowed a high level of expression. There was an untranslated region at the 3' end of *luc*, originating from the virus SV40, which contained the polyadenylation signal (poly(A)+). After intermediate cloning, which allowed the number of available restriction sites to be increased, the "CMV promoter-luc-poly(A)+" cassette was introduced into the minimal vector ori γ - *cer-sup* Phe (pXL2760) in place of the Km^{R} marker. The resulting plasmid has been named pXL2774. Figure 6 shows the various cloning steps. The ligation mixtures were transformed into *XAC*-1*pir116* by electroporation. Incubation allowing the bacteria to express selection markers was carried out in rich medium (SOC medium); it was thus necessary to wash the cells twice with M9 medium before plating out. This made it possible to remove the residual medium, which would have resulted in culture background noise on minimal medium.

The medium chosen to plate out the electroporated cells was M9 minimal medium, which makes it possible to select bacteria expressing a suppressor tRNA and thus the presence of our plasmids. The addition of X-Gal made it possible, by means of the blue colouration, to visualize the expression of the suppressor tRNA. The dishes were analysed after about 20 hours at 37°C. The absence of colonies on the DNA-free control assures us that the selection was correct, even with dense seedings. All the clones examined by restriction (8) do indeed carry a plasmid, corresponding to the expected profile. The plasmid thus constructed, pXL2774, was prepared from a clone cultured in one liter of liquid M9 medium (about 18 hours at 37°C), by a technique involving, inter alia, an ion-exchange step (Promega kit, MegaPreps). The amount of DNA collected was 2 mg.

### 2) Analysis of the reporter vector pXL2774 transfected into mammalian cells.

The capacity of pXL2774 to transfect eukaryotic cells and to allow the expression of luciferase was evaluated by transfection into NIH 3T3 mouse fibroblasts. The vector chosen as reference was the plasmid pXL2622 (this is the plasmid pGL2 from Promega whose SV40 promoter has been replaced by the CMV promoter), which carries the same luciferase expression cassette as pXL2774, but on a different replicon. This is a 6.2 kb ColEl derivative which carries the ampicillin-resistance gene. This plasmid serveed as a control. The luciferase activities (expressed as RLU, or relative luminescence units) are indicated in Table 3.

The best results were obtained with a "lipofectant charges/DNA charges" ratio of 6; under these conditions, pXL2622 and 2774 appear to be equivalent.

**TABLE 3**

| | **pXL2622** | | | **pXL2774** | | |
|---|---|---|---|---|---|---|
| **Charge ratios** | **RLU/pg of proteins and per well** | **Average** | **Coefficient of variation (%)** | **RLU/µg of proteins and per well** | **Average** | **Coefficient of variation (%)** |
| | 0.0 | not detectable | | 0.0 | not detectable | |
| 0 | 0.0 | | | 0.0 | | |
| | 0.0 | | | 0.0 | | |
| | 9.9 10⁶ | | | 3.3 10⁶ | | |
| 3 | 6.2106 | **7.610⁶** | 22 | 2.910⁶ | **2.910⁶** | 13 |
| | 6.6 10⁶ | | | 2.4 10⁶ | | |
| | 1.2 10⁷ | | | 9.4 10⁶ | | |
| 6 | 1.5 10⁷ | **1.5 10⁷** | 19 | 9.9 10⁶ | **1.0 10⁷** | 7 |
| | 1.9 10⁷ | | | 1.1 10⁷ | | |
| | 9.5 10⁶ | | | 1.110⁷ | | |
| 9 | 7.5 10⁶ | **1.0 10⁷** | 26 | 8.3 10⁶ | **6.4 10⁶** | 13 |
| | 1.4 10⁷ | | | 8.5 10⁶ | | |

### EXAMPLE 4: Verification of the suicide vector nature in E. coli of the pCOR plasmids

The non-replicative nature of the pCOR-type plasmids derived from R6K was verified by an electroporation experiment in JM109 *E*. *coli* (Yanisch-Perron et al., 1985) of plasmids pUC4K (ori ColEI-KmR, (Vieira and Messing, 1982)) and pXL2730 (ori gamma from R6K-KmR, see Example 2). The electroporator used was the Biorad Gene Pulser and the electrocompetent JM109 cells were prepared and used according to the manufacturer's procedure (Bacterial electro-transformation and pulse controller instruction manual. catalog number 165-2098).

The electrotransformed cells were plated out on LB medium supplemented with kanamycin (50 mg/l) and incubated overnight at 37°C. The results obtained are presented below.

### Results

| Plasmid | Amount transformed (ng) | Number of transformants | Efficacy (number of transformants/ ng of plasmid) |
|---|---|---|---|
| pUC4K | 0.01 | >>2000 | >2105 |
| pXL2730 | 5 | 0 | 0 |

These results show that there was a minimum of 5 logs of difference between the efficacy of transformation of a ColEI derivative (pUC4K) and that of an R6K derivative (pXL2730) in a strain which does not express the *pir* gene. In a pir+ strain such as XAC-1pir116, the electrotransformation efficacy of R6K-derived plasmids conventionally reaches or exceeds the 108 transformants/µg of plasmid.

### EXAMPLE 5: Production of plasmid DNA by high-density culturing of the E. coli strain XAC-1pir116 (pXL2774): fermentation process

### 5.1. Strains:

Production in XAC-1*pir116 E. coli* (Example 1) of a minimal plasmid, pXL2774; this plasmid comprises the following elements: ori R6K-cer-tRNAamsupPhe and an expression cassette of the luc reporter gene under the control of the CMV promoter (Example 3). A high-productivity process for the production of plasmids of this type was developed.

### 5.2. Culturing media and conditions:

### a) Growth medium:

Composition of the medium defined used for the inoculum cultures (g/l): Na₂HPO₄ 6, KH₂PO₄ 3, NaCl 0.5, NH₄Cl 1, NH₄H₂PO₄ 3, glucose 5, MgSO₄·7H₂O 0.24, CaCl₂-2H₂O 0.015, thiamine HCl 0.010

Composition of the complex medium used for the fed-batch cultures (g/l): KH₂PO₄ 8, K₂HPO₄ 6.3, Na₂HPO₄ 1.7, (NH₄)₂ SO₄ 0.74, NH₄Cl 0.12, yeast extract 3, glucose 2, MgSO₄·7H₂O 2.4 g/l, CaCl₂·2H₂O 0.015, thiamine 0.010, solution of salts (Fe, Mn, Co, Zn, Mo, Cu, B, Al).

Composition of the medium defined for the cultures in fed-batch medium identical to the complex medium, but the yeast extract is replaced by 2.5 g/l of NH₄Cl.

### b) Conditions of fed-batch culturing:

Studies in 2-liter fermenters (Setric France) containing 11 of medium were carried out in order to define the optimum conditions for growing and producing plasmid DNA. The fermenter was inoculated with 80 ml of an inoculum culture arrived at the start of the stationary phase of growth.

During the fermentation, the pH was controlled and adjusted automatically between 6.9 and 7.0 with 10% (w/v) aqueous ammonia; the temperature was maintained at 37°C; the aeration was set at 751/h ((1.1 vvm) at a pressure of 0.2 bar and the dissolved oxygen was adjusted to (40% of air saturation by retroaction on the stirring rate and, if necessary, by enrichment with pure oxygen.

All the parameters (pH, temperature, stirring, OD, O₂ and CO₂ in the effluent gases) were collected and calculated in line via an HP3852 interface connected to a Hewlett-Packard 9000.

The base composition of the supply medium was as follows: 50% carbon source, 0.7% magnesium sulphate, 0.02% thiamine; for the complex medium, yeast extract was added to a concentration preferably of between 5 and 10%.

To adapt the culture conditions to 800-liter fermenters, production sequences composed of two successive inoculum cultures were carried out, on a laboratory scale: inoculum I in an agitated conical flask and inoculum II in a 2-liter fermenter (batch culturing), followed by fed-batch production culturing, in a 7-liter fermenter.

### 5.3. Results

Various culture conditions were studied in complex medium, in defined medium, and at various growth rates. In all cases, after initial batch culturing of the bacterial strain and consumption of the carbon source, the supply medium was added to the fermenter by means of a peristaltic pump coupled to a pre-programmed addition profile. This profile was deduced from previous experiments in which the supply rate had been controlled either by the level of dissolved oxygen or by a constant growth rate.

Furthermore, in order to extrapolate without difficulty the 2-liter fermentation condition to an 8001 fermenter without overoxygenation of the medium, the maximum oxygen demand at the end of the culturing was set at 2.5-3 mM/min. For this, the growth rate of the microorganism was reduced, if necessary, by varying the supply rate of the complementary charge.

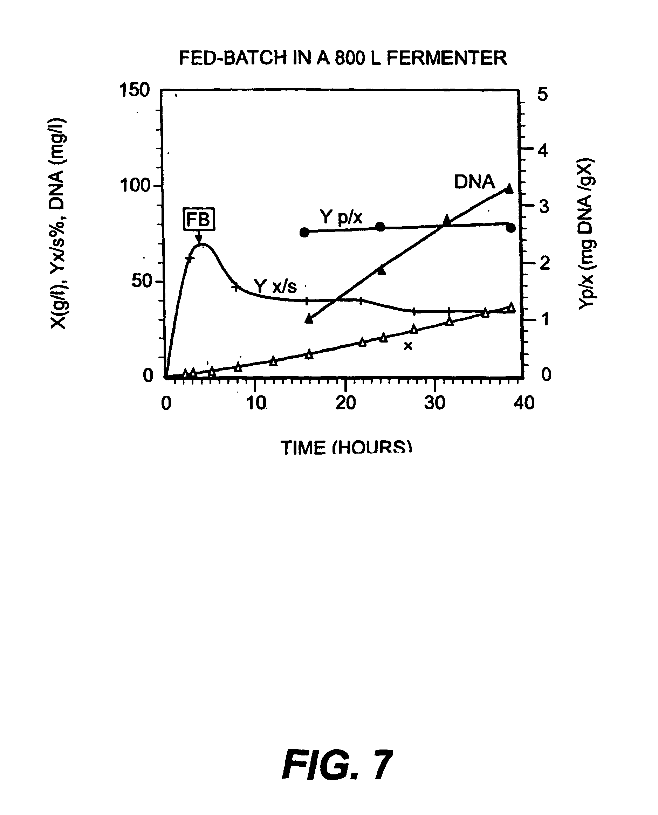

As seen in Table 4, very good results were obtained both in complex medium and in defined medium, both on the laboratory scale and on the 800-liter fermenter scale; furthermore, the plasmid DNA growth and production kinetics are entirely comparable (cf. Figures 6 and 7).

**Table 4:**

| | **Complex medium** | | **Defined medium** |
|---|---|---|---|
| | **2 or 71 fermenter** | **8001 fermenter** | **21 fermenter** |
| **Duration of fermentation (hours)** | 40 | 39 | 48 |
| **µh-I** | 0.130 | 0.132 | 0.124 |
| **OD (600 nm)** | 114 | 100 | 94 |
| **X g/l** | 44 | 37 | 30 |
| **Plasmid DNA (mg/l medium)** | 115 | 100 | 100 |
| **Plasmid DNA (mg/gX)** | 2.6 | 2.7 | 3.3 |

| | | | |
|---|---|---|---|
| X = Biomass (weight of dry cells) | | | |

From the overall results it emerges that:
- changing the scale of the fermenter from 2 liters to 800 liters can be carried out without any problem,
- the oxygen consumed is strongly correlated to the biomass produced (1.08 g O₂/g of biomass produced),
- the plasmid was stable for at least 50 generations without selection pressure,
- a high biomass, greater than 40 g of dry cells/liter, can be obtained in complex medium,
- the plasmid DNA production reaches 100 mg of supercoiled DNA/l of medium,
- there was very good correlation between the DNA production and the biomass: the production can be estimated to (1 mg of plasmid DNA/OD unit, or alternatively (2.7 mg of plasmid DNA/g of biomass, irrespective of the duration of fermentation,
- the use of a defined medium also makes it possible to achieve a high biomass (30 g of dry cells/l) and high plasmid DNA production (100 mg/l), without any loss of productivity.

### EXAMPLE 6: Transfer of pXL2774 into animal cells, in vitro and in vivo

### 6.1. In vitro transfer of pXL2774 into animal cells

The capacity of the minimal plasmid pXL2774 to transfect various cell lines was tested in vitro, on cells of both human origin and murine origin. The pXL2784 plasmid was used as control. It contains the same eukaryotic expression cassette (CMV promoter-luciferase-polyA) as pXL2774, but this is a 6.4 kb ColEl derivative which comprises the gene for imparting kanamycin resistance in E. coli.

The cells tested are the following:

| **Cells** | **Type** | **Atcc ref./ literature ref.** |
|---|---|---|
| 3LL | Mouse pulmomary carcinoma | |
| NIH 3T3 | Mouse embryo fibroblasts | CCL92 |
| 293 | Human embryo renal cells transformed with type-5 adenovirus | CRL1573 |
| HeLa | Human carcinoma from the neck of the womb | CCL2 |
| Caco-2 | Human colon adenocarcinoma | HTB37 |
| H460 | Human lung carcinoma with no small cells | HTB177 |
| ECV 304 | Human umbilical cord endothelial cells | Takahashi et al., 1990 |

The transfection conditions were as follows:
D-1: Inoculation of the cells at a density of 100,000 cells per 2 cm² well (24-well plate) in DMEM medium (Dulbecco's modified Eagle Medium) supplemented with 10% fetal calf serum (FCS).
D-3: Transfection of the cells, by 10 µl of a transfection solution containing: 0.5 µg of DNA, 150 mM NaCl, 5% glucose and 3 nmol of RPR120 535 lipofectant per µg of DNA, in 250 µl of culture medium, which was or was not supplemented with 10% FCS. After incubation for 2 hours, the medium was replaced by 500 µl of DMEM medium supplemented with 10% FCS.
D-4: Renewal of the culture medium
D-5: Washing of the cells with PBS, followed by lysis with 100 µl of Promega lysis buffer (Promega Cell Lysis Buffer E153 A). Assay of the luciferase activity was carried out in a Lumat LB 9501 luminometer (Berthold) on 10 µl of lysate, with a 10-second duration of integration. The reactant used was that from Promega (Promega Luciferase Assay Substrate). The results, collated in the following tables 5-8, are expressed in RLU (Relative Lights Units) for 10 µl of lysate (average of measurement on 4 wells). The coefficients of variation (CV) are also given.

The results of transfections in the absence of serum are presented below.

| | **CELL TYPES** | | | |
|---|---|---|---|---|
| | NIH 3T3 | 3LL | 293 | |
| **pXL2774** | 37 763 380 | 559270 | 1 884 200 | RLU |
| | 16 | 25 | 73 | CV |
| **pXL2784** | | 113 764 | 1 723 546 | RLU |
| | | 24 | 101 | CV |

### CELL TYPES

| | | | | |
|---|---|---|---|---|
| | | | | |

| | HeLa | CaCo2 | H460 | ECV304 |
|---|---|---|---|---|
| pXL2774 | 11000000 | 1 108 422 | 1459 501 | 36450 |
| | 15 | 14 | 5 | 23 |
| pXL2784 | 557930 | 93610 | 7 563 | 168795 |
| | 87 | 40 | 11 | 40 |

The results of transfections in the presence of serum (10%) are presented below:

### CELL TYPES

| | NIH 3T3 | 3LL | 293 |
|---|---|---|---|
| pXL2774 | 50612590 | 566377 | 992500 |
| | 12 | 18 | 59 |
| PXL2784 | 12 693 780 | 436 704 | 2 300 000 |
| | 38 | 12 | 47 |

### CELL TYPES

| | HeLa | H460 | ECV304 |
|---|---|---|---|
| pXL2774 | 9490000 | 857385 | 18 021 |
| | 25 | 16 | 30 |
| PXL2784 | 1 508 480 | 433 023 | 32 074 |
| | 23 | 27 | 47 |

These results reveal the capacity of pXL2774 to transfect effectively, *in vitro,* various cell types of both murine and human origin. The expression of the *luc* reporter gene made it possible to show that its transfection efficacy is at least as good as that of a "standard" plasmid, derived from ColE1, which carries the same expression cassette of luciferase.

### 6.2. In vivo transfer, in animals (mice), of pXL2774

### a) Model 1: Naked DNA in mouse cranial tibial muscle

Naked plasmid DNA, dissolved in "5% glucose, 150 mM NaCl" was injected into the cranial tibial muscle of OF1 mice. The muscles were removed 7 days after injection, chopped up, homogenized in 750 µl of lysis buffer (Promega Cell Lysis Buffer E153A) and then centrifuged at 20,000 × g for 10 minutes.

Assay of the luciferase activity was carried out on 10 µl of supernatant after addition of 50 µl of reagent (Promega Luciferase Assay Substrate). The reading was carried out on a Lumat LB9501 luminometer (Berthold) with a 10-second duration of integration.

The results are presented in the table below.

| **Plasmid** | **pXL2784** | **pXL2774** | **pXL2784** | **pXL2774** |
|---|---|---|---|---|
| **Number of muscles:** | 8 | 8 | 10 | 10 |
| **Volume injected (ìl):** | 30 | 30 | 33 | 33 |
| **µg of DNA/muscle** | 19 | 13.5 | 10 | 6.25 |
| **RLU (for 10 µl)** | | | | |
| **Average** | 80922 | 471 733 | 35329 | 30569 |
| **Standard deviation** | 104573 | 402602 | 37041 | 35774 |

These results show that a conditional replication plasmid such as pXL2774 was indeed capable of transfecting mouse muscle cells *in vivo* and of doing so with comparable, or even superior, efficacy to that of a "standard" plasmid derived from ColE1, which carries the same expression cassette of the luciferase gene.

### b) Model 2: 3T3 HER2 tumour model

The model is as follows:
- Swiss/nude adult female type mice
- Experimental tumours induced after injection of 107 3T3 HER2 cells subcutaneously into the flank.
- The transfection mixture was injected 7 days after injection of the cells.

Solutions injected: The DNA was first dissolved in the buffer. After addition of all the products, the mixture contained, besides the DNA, NaCl (150mM) and 5% D-glucose in water or 5 mM HEPES buffer.
- Two days after the injection, the tumour tissue was removed, weighed and then chopped up and homogenized in 750 ìl of lysis buffer (Promega Cell Lysis Buffer E153 A). After centrifugation (20,000 × g for 10 minutes), 10 µl of supernatant was removed and luciferase activity was evaluated. This activity was determined by measuring the total light emission obtained after mixing with 50 µl of reagent (Promega Luciferase Assay Substrate) in a Lumat LB 9501 luminometer (Berthold) with a 10-second duration of integration.

The resulting activity was expressed in RLU (Relative Light Units) estimated in the entire tumour lysis supernatant.

### Results

| Buffer | Plasmid | | | RLU/tumour results | | +/n |
|---|---|---|---|---|---|---|
| H20 or HEPES | reference | µg/tumour | [DNA] final in inj. sol. | average | standard deviation | |
| HEPES | pXL2784 | 10 | 0.5 µg/µl | 744 150 | 682434 | 6/6 |
| | pXL2774 | 10 | 0.5 µg/µl | 1016380 | 1 322 500 | 5/6 |
| H2O | pXL2784 | 24 | 0.6 µg/µl | 2 906 073 | 1 745 857 | 8/8 |
| | pXL2774 | 16.8 | 0.4 µg/µl | 4292043 | 4 995 187 | 6/6 |
| H2O | pXL2784 | 7.5 | 0.3 µg/µl | 702554 | 552207 | 6/7 |
| | pXL2774 | 5 | 0.2 µg/µl | 3 413 430 | 4000875 | 6/6 |

These results show that a conditional replication plasmid, such as pXL2774, was indeed capable of transfecting mouse tumour cells *in vivo* and of doing so with an efficacy at least comparable to that of a "standard" plasmid, derived from ColE1, which carries the same expression cassette of the luciferase gene.

These various experiments demonstrate that the conditional replication plasmids, and more particularly pXL2774, did indeed have animal cell transfection characteristics that are essential for use in gene therapy. More precisely, the following were shown:
1) the capacity of pXL2774 to transfect efficiently, *in vitro*, various cell types of human or murine origin;
2) the capacity of pXL2774 to transfect, *in vivo,* mouse muscle;
3) the capacity of pXL2774 to transfect, *in vivo,* tumour cells implanted into mice.

The electrotransformation, fermentation and transfection experiments thus made it possible to validate conditional replication plasmids as vectors which can be used in gene therapy by showing:
i) that they did not replicate detectably in an E. coli strain that does not express the *pir* gene (conditional origin of replication)
ii) that they could be produced on a scale compatible with industrial production, in a defined medium that does not contain antibiotics;
iii) that these plasmids could transfect, *in vitro* and especially *in vivo,* mammalian cells.

### EXAMPLE 7: In vitro production of recombinant proteins

### 7.1. Construction of the expression vector

To show the feasibility of such an approach, we constructed an expression vector according to the criteria described above (Examples 2 and 3). This vector, pXL3056, contains:
1) the bacterial part which comprises the conditional origin of replication (ori gamma), the cer fragment of ColE1, and the gene which ensures selection in bacteria *(sup)*
2) the expression cassette, based on the system described by Studier (Studier et al., 1990), comprising the promoter of gene 10 of bacteriophage T7, the *lacO* operator, the gene coding for aFGF 154 (acidic Fibroblast Growth factor, form containing 154 amino acids) (Jaye et al., 1986), and the TF terminator of bacteriophage T7. This expression cassette is identical to the one present on the pXL2434 plasmid, which is described in application WO 96/08572.

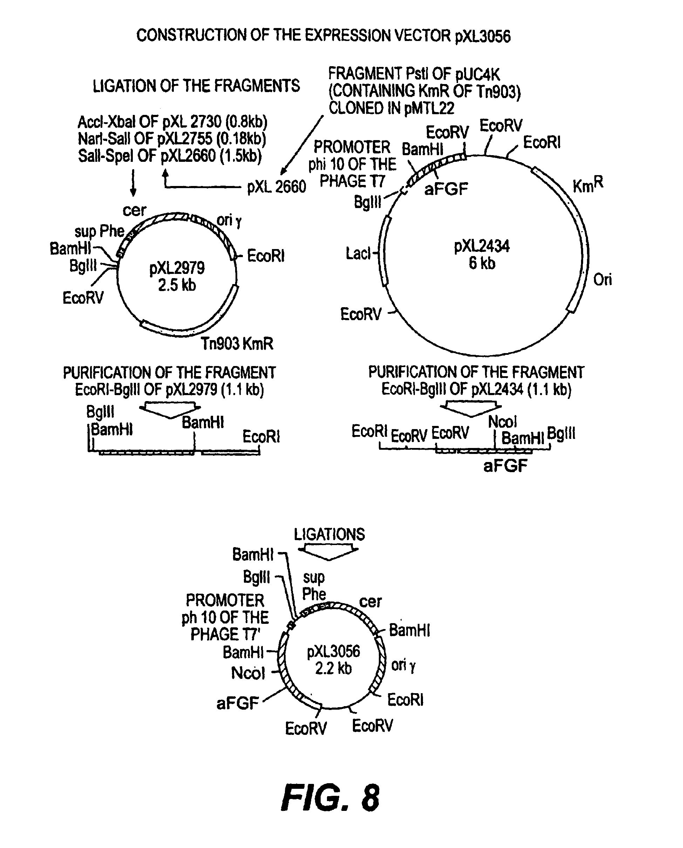

The construction of pXL3056 is presented in Figure 8. The *EcoR*I-*Bgl*II fragment of pXL2434 (1.1 kb) containing the aFGF expression cassette was cloned in the pXL2979 conditional replication vector (1.1 kb purified fragment) at the *Bgl*II and *Eco*RI sites to generate pXL3056.

pXL2979 results from the ligation of 3 fragments: i) *Acc*I*-Xba*I fragment of pXL2730 (0.8 kb, which provides ori gamma and *cer),* ii) *Nar*I*-Sal*I fragment of pXL2755 (0.18 kb, which provides the *sup Phe* gene), iii) *Sal*I*-Spe*I fragment of pXL2660 (1.5 kb, which provides the kanamycin resistance gene).

pXL2660 results from the cloning of the 1.2 kb *PstI* fragment of pUC4K (Vieira and Messing, 1982) in pMTL22 (Chambers et al., 1988) linearized with *Pst*I.

### 7.2. Production of the expression strain

Plasmid pXL3056 was introduced by transformation into the XAC-1*pir116* strain. The resulting strain was then transformed by the plasmid PT7pol23 (Mertens et al., 1995), at 30°C. In order to express the gene of interest under control of the T7 promoter, the bacterium must contain in its genome, on a plasmid, or a bacteriophage, a cassette allowing expression of the RNA polymerase of bacteriophage T7. In the example described, we used the plasmid pT7pol23, which is compatible with R6K derivatives such as pXL3056, and which allows the temperature-inducible expression of bacteriophage T7 RNA polymerase. However, it can also be envisaged to lysogenize the XAC-1*pir116* strain with lambda DE3 (Studier et al., 1990) to conserve only one plasmid and to induce the production of T7 RNA polymerase by IPTG rather than by temperature.

### 7.3. Expression of aFGF

The XAC-1*pir116* strain (pXL3056+PT7po123) was cultured at 30°C in M9 minimum medium supplemented with 0.2% of casamino acids (DIFCO) and kanamycin (25 µg/ml), up to an optical density at 600 nm of 0.6-1.0. Half of the culture was then placed at 42°C (induction of the T7 RNA polymerase), while the other half remained at 30°C (negative control). The same experiment was carried out with the XAC-1*pir116* (pXL3056+pUC4K) strain which constitutes a control for the expression of aFGF in the absence of T7 RNA polymerase.

The results obtained are presented in Figure 9. They show that the production of aFGF was comparable or superior to that observed with BL21 (DE3)(pXL2434) (WO 96/08572), which clearly shows the potential of conditional replication plasmids for the expression of recombinant proteins in vitro, especially in E. coli.

### EXAMPLE 8: Construction of a pCOR vector which expresses a wild-type or hybrid p53 protein or the FGF1 human protein

This example describes the construction of conditional replication vectors according to the invention containing a nucleic acid coding for a p53 protein. These vectors can be used to restore a p53-type activity in deficient (mutated, deleted) cells such as, in particular, tumour cells.

The eukaryotic expression cassette contains the following elements:
1) CMV "immediate early" promoter (positions -522 to +72) followed by the leader sequence of the thymidine kinase gene of type I herpes simplex virus (position -60 to +1 of the gene, with reference to the sequence in the article by McKnight, S.†L. (1980) Nucleic Acids Res. 8:5949-5964);
2a) a nucleic acid which codes for wild-type p53 protein or for a p53 variant, as described in application PCT/FR 96/01111 (V325K variant = V325 with a Kozak sequence with ATG);
2b) a nucleic acid which codes for the human FGFa or FGF-1 as described in Jaye M. (Sciences 1986; 233(4763) :451, US patent No: 4,686,113, and European Patent No: 259 475;
2c) a nucleic acid which codes for a fusion gene between human fibroblast interferon secretion signal (Taniguchi et al.) and the naturally occurring truncated form of human FGF-1 from amino acid 21 to 154 as described by Jaye et al., and US 5,849,538.
3) the polyA polyadenylation sequence of SV40.

These elements were placed in the form of a fragment *Asc*I-*Xba*I on the pCOR vector pXL2988 between the sites *Bss*HII and *Spe*I. pXL2988 is identical to pXL2979 (Example 7.1.) apart from the presence of an additional element, a sequence capable of forming a DNA triple helix composed of 17 copies of the trinucleotide GAA, placed alongside the gamma origin of replication.

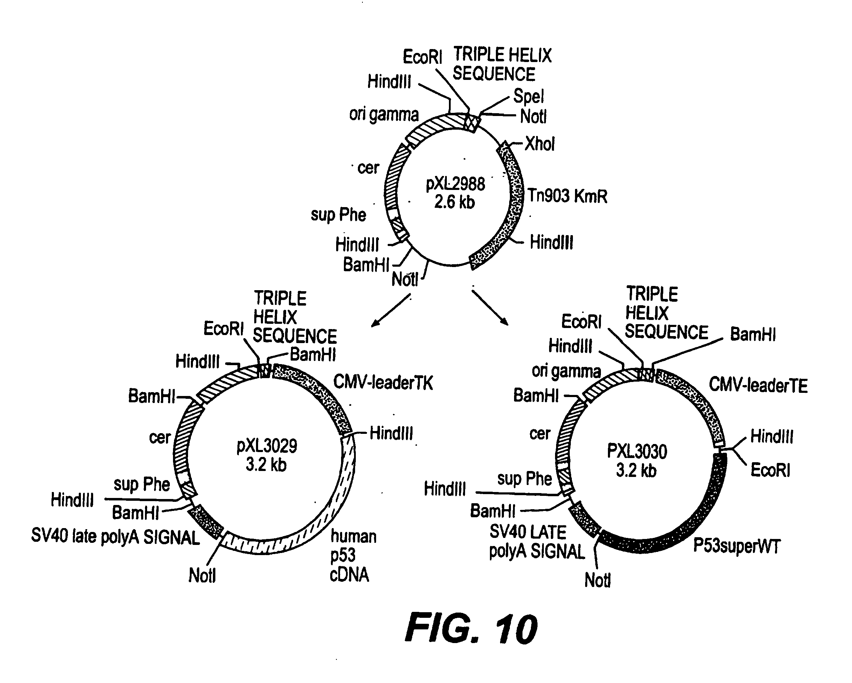

The resulting plasmids were named pXL3029, pXL3030, pXL3179 or NV1FGF (Figure 10).

The functionality of these constructions was verified *in vitro* on p53-SAOS2 cells in culture by measuring the transcriptional-activator activity of p53 or p53superWT, or by measuring the secretion of FGF1 for example by ELISA experiments which is well known in the art.

### EXAMPLE 9: Construction of TEX 1 (XAC1 pir116, endA⁻, traD⁻)

The *E. coli* XAC-1*pir116* contains an F' episome, a circular DNA molecule of approximately 100kb, that carries proB⁺ lacI₃₇₃lacZᵤ₁₁₈ₘ. Many male ***E*. c*****oli*** laboratory strains carry a *traD36* mutation on their episome, but no mutation affecting F' transfer ability has been described for XAC-1. The gene *traD* is at the 5' end of one of the *tra* (transfer) operons and encodes a membrane protein directly involved in DNA transfer and DNA metabolism (Frost et al., BBRC, 1994, 58:162-210). A 2kb central fragment from *traD,* comprising 92% of the gene, was replaced with the 2kb omega element (Genbank accession number M60473) from pHP45Ω (Prentki and Krisch, 1984, Gene, 29:303-313) by homologous recombination in XAC-*1pir116 endA⁻.* The omega element contains the *aadA* antibiotic resistance gene flanked by short inverted repeats. The gene *aadA* encodes aminoglycoside-3 adenyltransferase and confers resistance to streptomycin and spectinomycin ("Sp^{R}"). The omega fragment was used because it prematurely terminates RNA and protein synthesis leading to the inactivation of the whole *traD* operon. This new pCOR strain XAC-1*pir116 endA- traD*::SpR was designated TEX1. Transfer of any resident plasmids, either pCOR or pUC, was undetectable when the donor was TEX1.

The new pCOR host strain TEX1 was assessed in fermentation experiments. Complex media containing yeast extract were used for fed-batch fermentation with XAC-1*pir116.* pCOR stability (more than 50 generations) makes it possible to use a non-selective media. Under these conditions, XAC-1*pir116* produced more than 40 g/l dry cell weight and 100 mg/l of pCOR pXL2774 were obtained from 2-liter fermenters. pCOR copy number was estimated at 400-500 copies per cell and the rate of plasmid DNA synthesis was constant throughout fermentation. These results were extrapolated to an 800-liter fermenter suitable for manufacturing. The fermentation was also performed in the absence of yeast extract or any raw material from animal origin. Similar results (30g/l dry cell weight and 100 mg/l of plasmid DNA) were obtained using a defined medium in 2-liter cultures with no loss of productivity.

### EXAMPLE 10: Construction of XAC-1pir116pir42 host strains by homologous recombination

### 1) Construction of a suicide vector carrying the cassette "KmR-uidA:pir116;pir42"

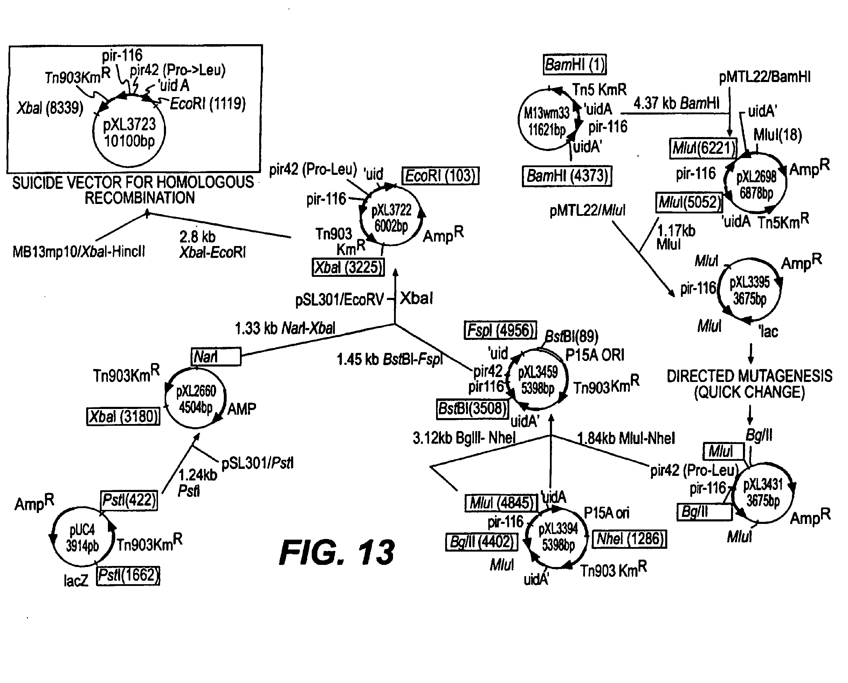

The *Km^{R}-uidA::pir116* cassette from M13wm33 as described in Example 1 (Metcalf W. et al. Gene, 1994, 138(1-2): p. 1-7), was modified by site-directed mutagenesis using PCR (QuickChange site-directed mutagenesis kit, Stratagene, La Jolla, CA) to introduce the *pir42* mutation into the *pir116* gene. The different cloning/mutagenesis steps are described in Figure 13

The oligonucleotides used for mutagenesis contained the *pir42* mutation along with a silent mutation that created a *Cla*I site to easily indicate the processing of *pir42* by restriction analysis when needed.

The sense and antisense oligonucleotides used are as follows:
Sense oligonucleotide number 11076 (SEQ ID NO: 7)
Antisense oligonucleotide number 11077 (SEQ ID NO: 8)

The technique used to replace *pir116* by *pir116pir42* in the genome of *E*. *coli* pCOR host TEX1 was based on that of Blum et al. (J.Bacteriol. 1989, 171, pp 538-46). The recombinant bacteriophage pXL3723 shown in Figure 13 is a suicide vector in all non-suppressor *E*. *coli* strains, because it has a non-sense mutation in gene II encoding M13 nickase that prevents viral genome replication.

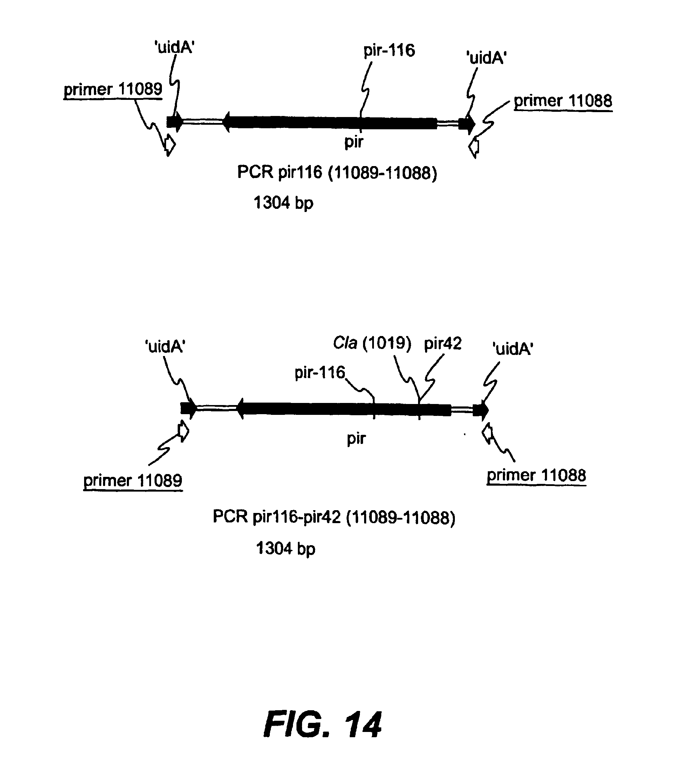

Double recombination was performed as described for the construction of XAC-1*pir116* (Example 1, point 2). Clones that had undergone double homologous recombination events were screened by PCR to test for the presence of the *pir42* mutation in the genome of TEX1. Genomic DNA isolated from double recombination candidates was used as a template for PCR. Secondly, sequencing was done on each unique amplified fragment, all of which were of the expected size. The PCR fragments are shown in Figure 14.

The PCR primers were the following:
Primer 11088 (SEQ ID NO : 9) :5'-GAGATCGCTGATGGTATCGG-3'
Primer 11089 (SEQ ID NO : 10): 5'-TCTACACCACGCCGAACACC-3'

This analysis showed that one out of the six double recombinants tested had undergone the allele exchange. This new strain, named TEX1*pir42*, was further evaluated for its ability to replicate pCOR plasmids compared to the parental strain TEX1.

### 2) Evaluation of TEX1pir42

pCOR plasmids were transformed in parallel into TEX1 and TEX1*pir42* and grown overnight in 2ml of selective M9 medium. Then, the plasmid DNA was extracted with the Wizard SV plus minipreps kit (Promega) to evaluate the relative plasmid copy number and topology of the pCOR plasmids in both strains.

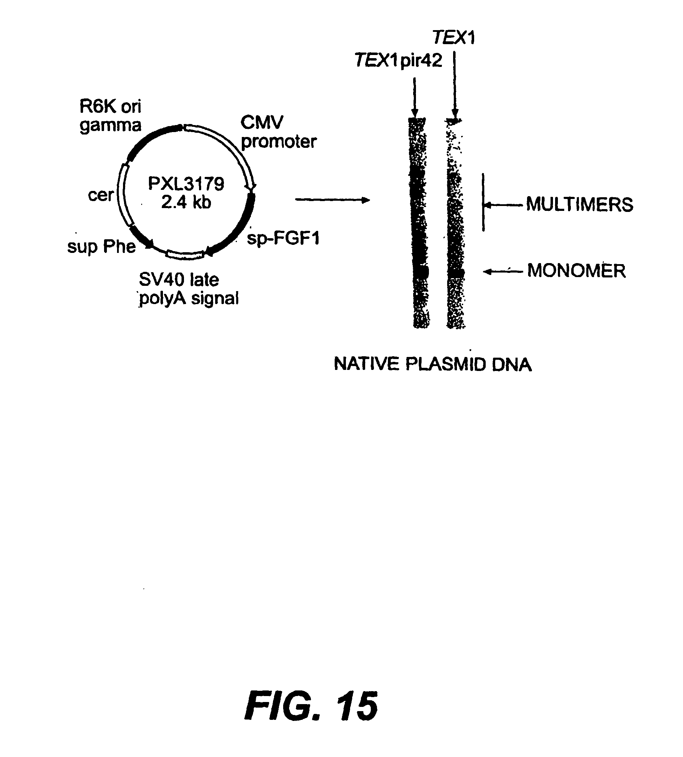

A 2-fold increase in copy number was obtained reproducibly in TEX1*pir42* transformed with the pCOR plasmid pXL3516 (2.56 kb). To further characterize TEX1*pir42,* the copy number and topology of pCOR plasmids such as pXL3179 and pXL2774 were evaluated by agarose gel electrophoresis analysis after small scale purification of plasmid DNA (4 to 6 clones/strain). Copy number was evaluated on plasmids linearized with *Eco*RI restriction enzyme. A topology test was run on non-digested plasmids, in the absence of ethidium bromide. The resulting agarose gel is displayed in Figure 15, and clearly shows a higher plasmid copy number when the plasmid pXL3179 was produced in TEX1*pir42,* than when produced in TEX1 strain. Figure 15 also displays the topology of the plasmid pXL3179, and shows that an increase in plasmid copy number, which were essentially in the form of monomers, with few plasmids in the form of multimers. The results obtained with these pCOR plasmids are also summarized in Table 5. Relative copy number was calculated in comparison with the same plasmid in TEX1. A 2-3 fold increase in plasmid copy number was observed with plasmids pXL3179 and 2774 produced in TEX1*pir42.*

**TABLE 5: Replication and copy number of pCOR plasmids produced in TEX1pir-42**

| **PLASMIDS** | **SIZE (kb)** | **RELATIVE COPY NUMBER*** |
|---|---|---|
| **pXL3179** | 2.4 | x3 |
| **pXL2774** | 4.5 | x2 |

| | | |
|---|---|---|
| * copy number was compared to the same plasmid in TEX1. | | |

### EXAMPLE 11: Comparative experiments: construction of TEX1cop21 (XAC-1endA-traD- pir116cop21)

### 1) Construction of TEX1cop21

The TEX1*cop21* strain was constructed similarly as that described in Example 10 for TEX1*pir42.* The following oligonucleotides used to introduced *cop21* into the *pir116* gene by directed mutagenesis were as follows:
Sense oligonucleotides: 11153 (SEQ ID NO: 11)
Antisense oligonucleotide : 11154 (SEQ ID NO: 12) 5'-G GCT ACT TAA GCG TAA GCT GGA CGT TAA CAA TTG CG-3'

The *cop21* mutation was introduced as a TCC serine codon instead of the TCA serine codon to eliminate a *Hind*III restriction site close to the mutation.

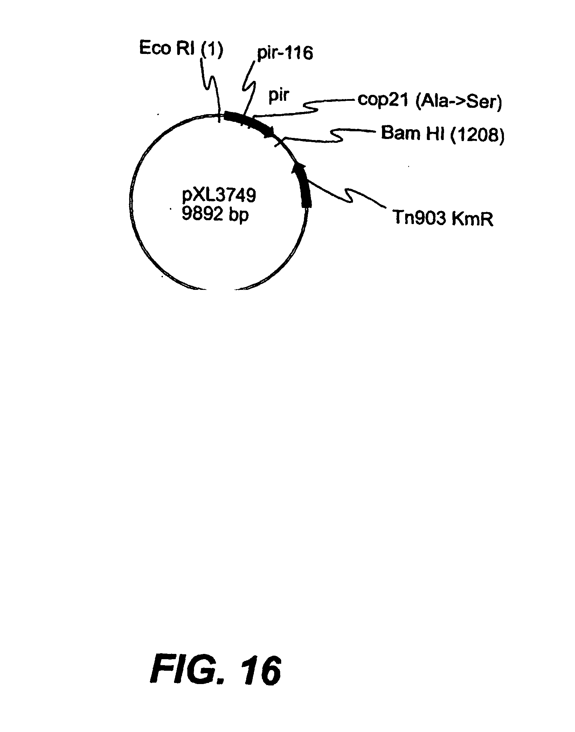

The template used for directed mutagenesis was pXL3395 (see Figure 13). The resultant plasmid named pXL3432 was used to construct the suicide M13 vector in a similar way as to what is shown for *pir42* in Figure 13. The suicide vector pXL3749 is shown in Figure 16.

The *E*. *coli* clones obtained after homologous recombination with pXL3749 were screened by PCR and subsequent restriction with *Hind*III and sequencing to monitor the *cop21* and *pir116* mutations. One clone out of the six double recombinants tested had undergone the gene replacement. The resulting strain was named TEX1*cop21.*

### 2) Evaluation of TEX1cop21

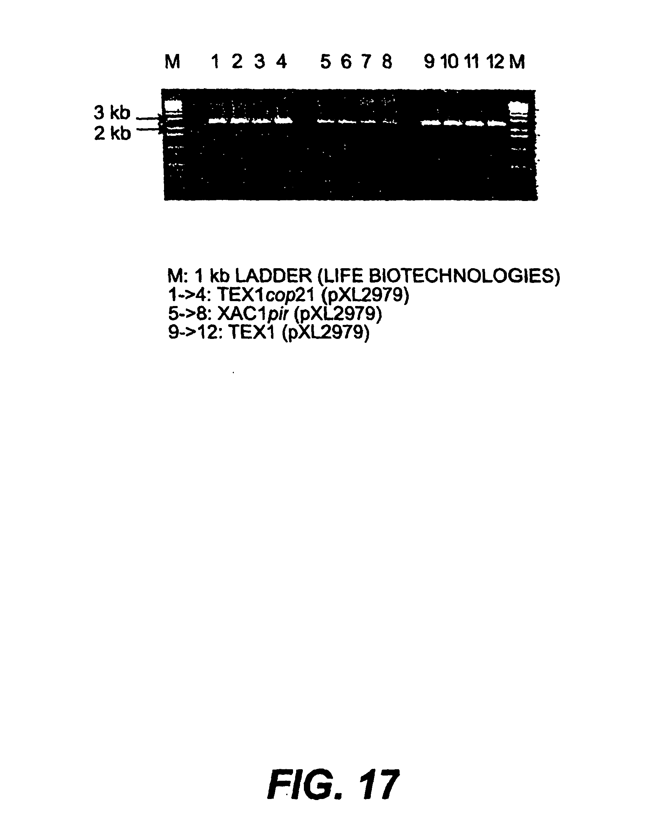

TEX1*cop21* was transformed by various pCOR plasmids, including pXL2979, a 2.5 kb Km^{R} pCOR vector (See Example 7.1), and assayed for increased copy number by gel electrophoresis. Such an experiment with pXL2979 is shown in Figure 17. Plasmid DNA from four independent clones for each strain prepared with Promega miniprep kit was linearized with *Eco*RI*,* electrophoresed on agarose gel and then stained with ethidium bromide. Each sample represented a similar amount of bacteria, as measured by optical density at 600 nm. The agarose gel electrophoresis obtained for the pCOR plasmid pXL2979 produced in *E. coli* TEX1*cop21,* XAC1*pir,* and TEX1 is displayed in Figure 17. It clearly shows there was no increase in plasmid copy number when the plasmids are produced in the TEX1*cop21* strain, as compared with TEX1.

### EXAMPLE 12: Construction of TEX2pir42 (XAC-1pir116pir42 recA⁻)

Firstly, a *recA-* derivative of TEX1 was constructed. The *pir42* mutation was then introduced into the resulting strain named TEX2 to generate TEX2*pir42.*

### 1) Construction of E. coli TEX2, a recA- derivative of TEX1

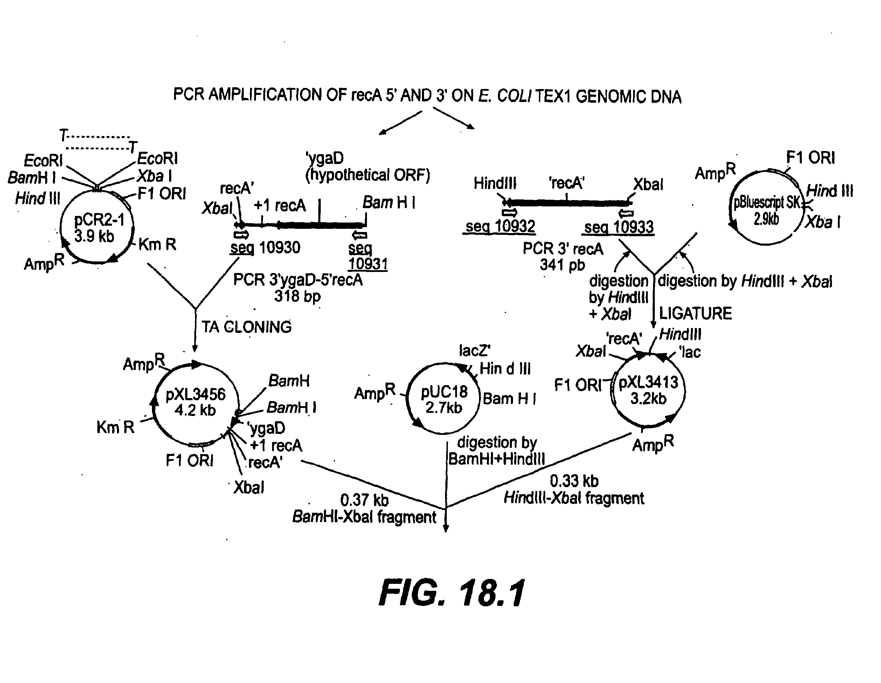

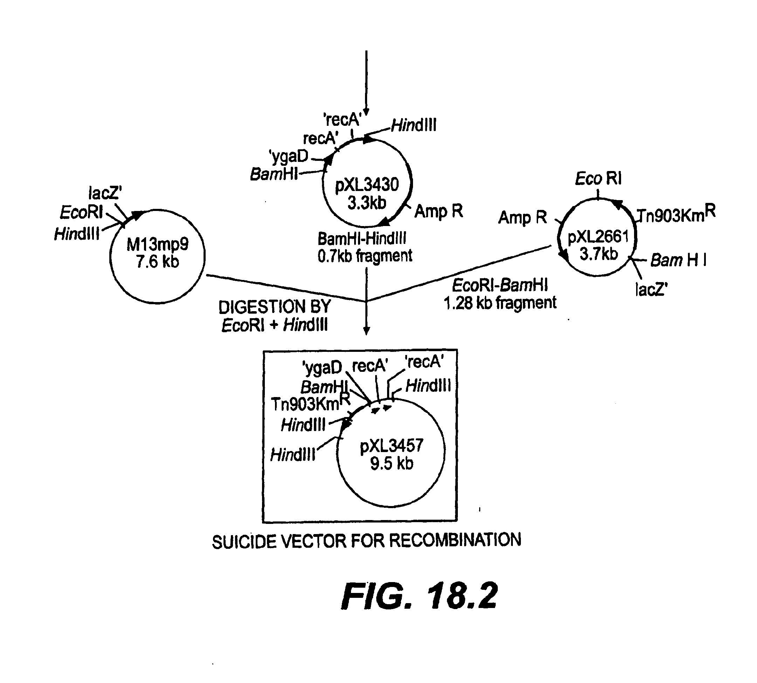

A deleted *recA* gene containing 3 translation stop codons (one in each frame) at its 5' end was obtained by PCR. This deleted *recA* gene was introduced by gene replacement (Blum et al., J. Bacteriol., 1989, 171, pp. 538-46) into the TEX1 genome. The construction of the suicide vector for homologous recombination is shown in Figure 18.

PCR primers used for the amplification of *recA* fragments are shown in the following Table 6:

**TABLE 6**

| primers | DNA sequences |
|---|---|
| seq 10930 SEQ ID NO: 13 | 5'CCCTCTAGATCGATAGCCATTTTTACTCCTG 3' |
| seq 10931 SEQ ID NO: 14 | 5'CGGGATCCTGATTATGCCGTGTCTATTAG 3' |
| seq 10932 SEQ ID NO: 15 | 5' CCCAAGCTTCTTCGTTAGTTTCTGCTACGCCTTCGC 3' |
| seq 10933 SEQ ID NO: 16 | 5'GGTCTAGAACGTGAAAGTGGTGAAGAACAAAATCG 3' |

### Restriction sites added to the recA sequence are underlined.

To maintain the RecA+ phenotype necessary for homologous recombination to occur, the *recA* function was provided to *E. coli* TEX1 with a plasmid containing a heterologous *recA* gene that can complement *E. coli recA* mutants, such as for example the *recA* gene of the bacterium *Agrobacterium radiobacter,* and an antibiotic gene resistance, such as the ampicillin resistance gene. After gene replacement, the plasmid was eliminated from the recombinant strain by culture-dilution in non-selective medium (LB). The absence of the plasmid was screened for the loss of antibiotic resistance.

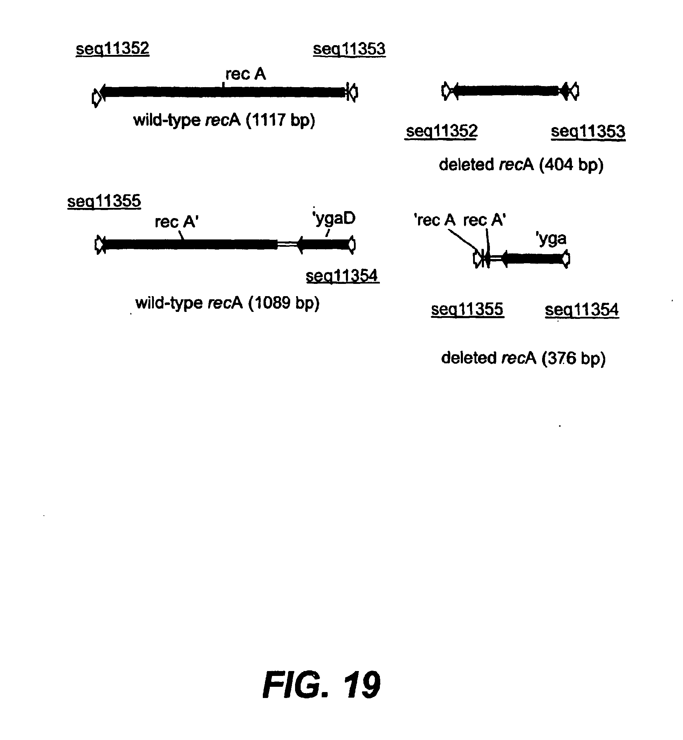

The resulting strain was named TEX2. Gene replacement was monitored by PCR in Figure 19. PCR Primers are described in the following Table 7.

**TABLE 7**

| | **Primers 11355-11354** | **Primers 11355-11354** |
|---|---|---|
| **Wild type recA** | 1117 bp | 1089 bp |
| **Deleted recA** | 404 bp | 376 bp |

The first primer was based on the sequence of the *recA* gene. The second one was based on a sequence close to but outside the homology region present in the suicide vector pXL3457 (immediately 5' or 3' of *recA)* to ensure that amplification can only occur on a genomic fragment. The sequence of both oligonucleotides was chosen according to the sequence of *E. coli,* which comprises the *recA* locus (Genbank ECAE000354).

The PCR fragments obtained from a recA-deleted strain were shorter as compared to those obtained with a wild-type strain, as presented in the following Table 8.

**TABLE 8: PCR primers for amplication of recA**

| primers | 5'->3' sequence |
|---|---|
| seq 11352 - SEQ ID NO: 17 | GCGACCCTTGTGTATCAAAC |
| seq 11353 - SEQ ID NO: 18 | GGTATTACCCGGCATGACAG |
| seq 11355 - SEQ ID NO: 19 | GTGGTGGAAATGGCGATAGG |
| seq 11354 - SEQ ID NO: 20 | GCGATTTTGTTCTTCACCAC |

The PCR profile obtained was as expected and demonstrated the presence of a truncated *recA* gene in the genome of TEX2. The *recA-* phenotype (sensitivity to UV light), as well as phenotypic characteristics of TEX2, were checked. Phenotypic characteristics of TEX2 were the same as those of TEX1 strain, *i.e*., ara- , Rif^{R}, Nal^{R}, Sp^{R}, UidA-, Arg-, Km^{S} Amp^{S}), as expected.

### B) Construction of E. coli TEX2pir42

*A TEX2pir42* strain was constructed by double homologous recombination, according to the strategy described in Example 10, with the exception that recombination in TEX2 was carried out in presence of a plasmid carrying a heterologous *recA* gene capable of complementing the *E*. *coli recA* mutants, in order to maintain a *recA*+ phenotype required for homologous recombination.

Gene replacement was monitored by restriction analysis of the PCR product digested with *Cla*I (see Figure 14). Gene replacement had occurred in two out of the four-studied double recombinant clones.

### C) Evaluation of E. coli TEX2pir42

### 1) Evaluation at lab scale plasmid production:

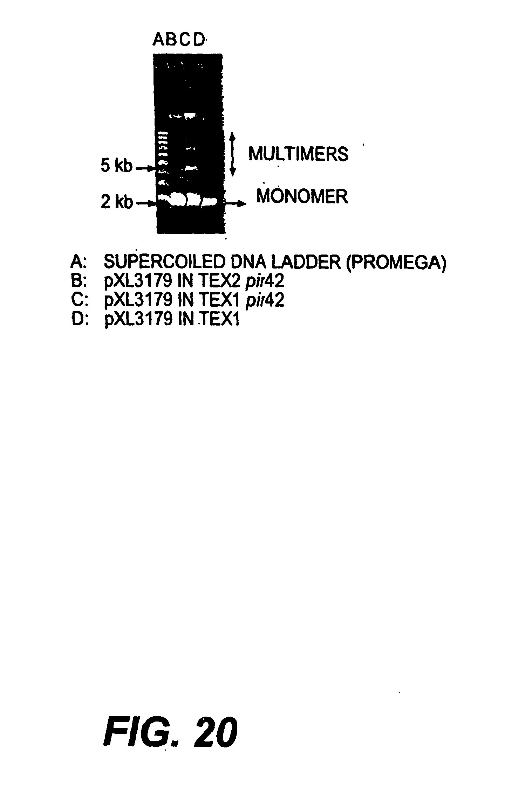

*TEX2pir42* was transformed by the pCOR plasmid pXL3179 (2.4 kb). Production of pXL3179 in *TEX2pir42* was intensively studied at the lab scale, in terms of reproducibility of the improvement of plasmid copy number, conditions of culture, as well as stability (number of generations). All the studies consistently showed a 2 to 5-fold increase of plasmid copy number as compared to production of pXL3179 in TEX1 under the same conditions. Plasmid copy number was assessed further to the production of pXL3179 in TEX2*pir42,* and TEX1*pir42* and TEX1 as comparative experiments. In this experiment, plasmids were extracted from identical bacterial biomass, based on the OD at 600 nm, and analyzed by agarose gel electrophoresis. The gel was stained with ethidium bromide after electrophoresis. The agarose gel electrophoresis, which is displayed in Figure 20, clearly shows that plasmids are produced in TEX2*pir42* at high copy number, and advantageously shows that plasmid multimers are reduced when produced in TEX2*pir42* instead of TEX1*pir42.*

### 2) Evaluation in fermenters:

These results were confirmed at a larger scale in 7-liter fermenters, as described below.

### a) Composition of fermentation media

The composition of the medium used for inoculum cultures was: Na₂HPO₄ 6 g/l, KH₂PO₄ 3 g/l, NaCl 0.5 g/l, NH₄Cl 1 g/l, NH₄H₂PO₄ 3 g/l, glucose 5 g/l, MgSO₄,7H₂0 0.24 g/l, CaCl₂,2H₂O 0.015 g/l, thiamine HCl 0.010 g/l.

The composition of the medium used for fed-batch culture was as follows: KH₂PO₄ 8 g/l, K₂HPO₄ 6.3 g/l, Na₂HPO₄ 1.7 g/l, NH₄Cl 2.5 g/l, glucose 10 g/l, MgSO₄,7H₂O 2.6g/l, thiamine 0.011g/l, Biospumex36 antifoam 0.1ml/l, salt mix (see table 9) 2.5 ml/l.

**TABLE 9 : Composition of salt mix**

| | Salt mix Solution (g/100ml) | Final concentration in fed-batch medium |
|---|---|---|
| FeSO₄, 7H₂O | 1.6 | 40 |
| CaCl₂, 2H₂O | 1.6 | 40 |
| MnSO₄, H₂O | 0.4 | 10 |
| CoCl₂, 6H₂O | 0.16 | 4 |
| ZnSO₄, 7H₂O | 0.08 | 2 |
| MoO₄Na₂, 2H₂O | 0.072 | 1.8 |
| CuCl₂, 2H₂O | 0.04 | 1 |
| H₃BO₃ | 0.02 | 0.5 |
| AlCl₃, 6H₂O | 0.04 | 1 |

The composition of the supply medium was as follows: 50% glucose, 0.7%, magnesium 0.02% thiamine-HCl, 1% Biospumex36 antifoam.

### b) Fermentation parameters

A 7-liter fermenter containing 3 liters of the fed-batch medium was inoculated with 1.2% of the inoculum culture. Inoculum was prepared as follows: 250ml of the inoculum medium in a 2-liter flask was inoculated with 0.25ml of a frozen cell suspension of the *E*. *coli* strain *TEX2pir42* (pXL3179).

Flasks were incubated for 24 hours at 37°C at 220 rpm. After 24 hours, different parameters were measured: residual glucose: 0g/l, OD₆₀₀ₙₘ was 2.7 and pH 6.24. During fermentation, the pH was controlled and adjusted automatically between 6.9 and 7 with NH₃. The temperature was maintained at 37°C and the dissolved oxygen adjusted to a 45% pO2 by retroaction on the stirring rate.

After initial batch culturing of the bacterial strain for about 17 hours and consumption of the carbon source (glucose), the supply medium was added. Glucose and acids, such lactate and acetate, were maintained at a concentration close to 0.

### c) Results

Final results are presented in Table 10, as compared to production in a 100-liter fermenter with *E*. *coli* TEX1 (pXL3179) in optimized conditions.

As for XAC-1*pir116,* there was no difference between 7-liter and 800-liter fermenters in terms of plasmid copy number of pXL3179 produced in TEX1.

Plasmids pXL3179 produced in a 7-liter fermenter using a *E. coli* TEX2*pir42* was compared to the production of pXL3179 in a 100-liter fermenter with *E*. *coli* TEX1, in optimized conditions. It was demonstrated that as for XAC-1*pir116* (See Example 5.3), there is a stable plasmid production rate in a 7-liter, 100-liter, or 800- liter fermenter in TEX1.

**Table 10: Characteristics of the fermentation of TEX1 and TEX2pir42 strains containing pXL3179.**

| | Reference | Duration of fermentation (h) | Final OD (600nm) | Cell Dry weight | Concentration of DNA (mg/l) | Estimated copy number (copy/ bacterium) |
|---|---|---|---|---|---|---|
| **TEX1 (pXL31 79)** | OpGen 090 | 43.00 | 104 | 33.1 | 96 | 616-627 |
| **TEX2*pi r42* (pXL31 79)** | Op132 8S5 | 48.47 | 72 | 27.1_ | 205 | 1896-1904 |

There were 3-fold more copies of plasmid pXL3179 per bacterium in TEX2*pir42* as compared to TEX1.

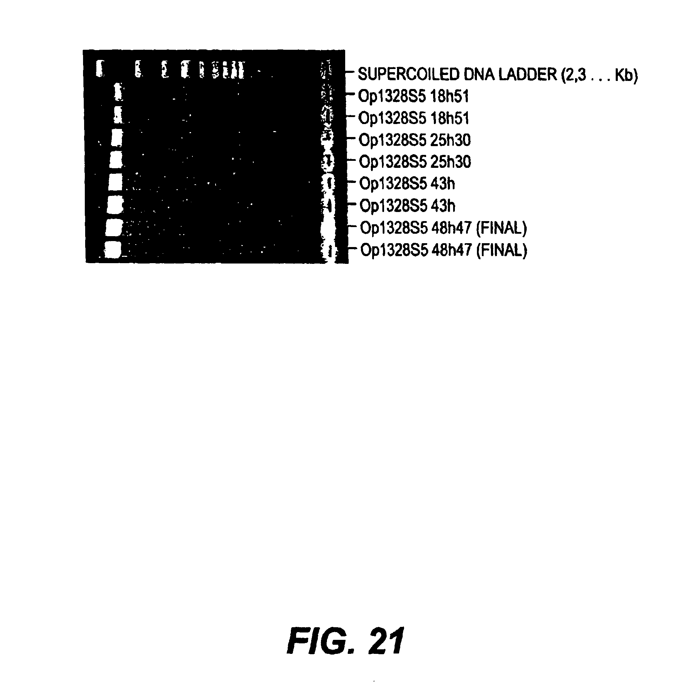

Plasmids corresponding to different fermentation time points were extracted from identical bacterial biomass, based on the OD at 600 nm, and analyzed by agarose gel electrophoresis. Figure 21 clearly shows an increase of the plasmid copy number with the duration of the fermentation. Also, Figure 21 shows the topology of the pXL3179 plasmid produced in Op1328S5 *TEX2pir42,* which was nearly exclusively in a monomeric form.

In conclusion, the *E. coli* host strain *TEX2pir42* according to the present invention provided an unexpectedly high plasmid copy number improvement of pCOR plasmids, such as pXL3179, of 2 to 5-fold in *TEX2pir42* as compared to TEX1, at a lab scale and in fermenters. Furthermore, while the plasmid copy number was greatly improved, plasmids so produced exhibited a monomeric topology, not only at lab-scale but also at a larger scale **(7-liter fermenter) compatible with industrial production.**

### EXAMPLE 13: New Copy-Up Mutants of pir116 Identified by a Novel Fluorescence-Based Screening Method

To increase pCOR plasmid copy number in bacterial host cells, we have mutagenized the *pir116* gene, which encodes a copy-up mutated version of the *pir* gene. To date all of the mutations increasing the copy number of R6K-derived plasmids, such as embodiments of pCOR, have been found within the *pir* gene.

After random mutagenesis by PCR, mutated *pir116* genes were introduced into a pCOR vector containing the *cobA* reporter gene, which is described below. After fluorescence based screening, the copy number and topology of the selected mutant plasmid were evaluated. We obtained three different mutants of *pir116* gene that increase plasmid copy number. These novel mutations have not been described previously.

A classical screening method for copy-up mutants is based on antibiotic resistance. In this method, the level of resistance of a host bacterium to an antibiotic is a function of the copy number of an antibiotic resistance gene located on a plasmid within the cell. As the copy number of the plasmid, and therefore the antibiotic resistance gene, increases, the level of antibiotic resistance also increases. This method, however, was not applicable for R6K-derived plasmids in host cells containing the *pir116* mutation due to a too high baseline copy number of the plasmid (about 400 copies/cell) in these cells. Accordingly a new screening method based on fluorescence to identify copy-up mutations of the *pir116* gene was developed.

For this new method, the *cobA* gene was introduced into a pCOR vector to provide a simple means of monitoring improvement in plasmid copy number. The *cobA* gene was obtained from *Pseudomonas denitrificans* ( Crouzet et al., J. Bacteriol, 172:5968-79 (1990)). It encodes uroIII methyltransferase, an enzyme of the vitamin B12 pathway that adds two methyl groups to the urogen III molecule. When overexpressed in *E*. *coli, cobA* leads to the accumulation of red products that are fluorescent under near UV light. When exposed to UV, bacterial colonies overexpressing this gene appear pink to red. We tested this gene to determine if it could serve as a reporter gene for plasmid copy number in the pCOR system.

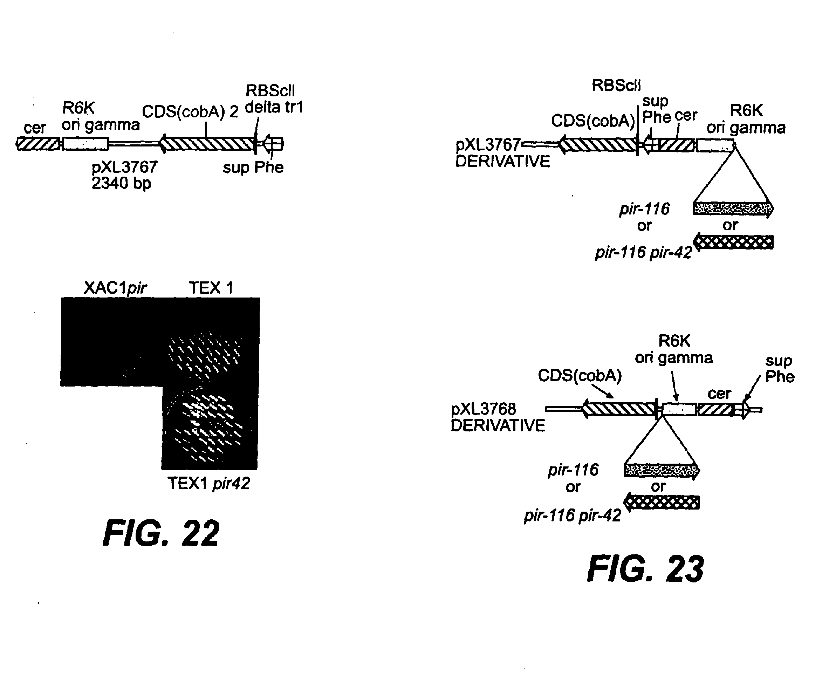

To evaluate the relationship between plasmid copy number and level of fluorescence of transformed bacteria exposed to UV light a control plasmid (pXL3767) was constructed comprising *cobA* deleted of its own promoter (Fig. 22). This plasmid was transformed into three different host strains (XAC1*pir,* XAC1*pir116* and TEX1*pir42).* These strains were selected based on previous experiments showing that the average copy number of a pCOR plasmid in XAC1*pir* is 1, it is approximately five to ten fold higher in TEX1, and 15 to 30 fold higher in TEX1*pir42.*

Recombinant colonies were streaked on M9 minimal medium and exposed to UV light on a transilluminator as shown in Figure 22. We observed that the fluorescence intensity of the colonies was positively correlated with the plasmid copy number, with XAC1*pir116* exhibiting more fluorescence than XAC*pir,* and TEX1*pir42* exhibiting more fluorescence than XAC1*pir116.*

The results shown in Fig. 22 demonstrate that this fluorescence-based assay method easily discriminates between the tested plasmid copy numbers, especially between the plasmid copy number found in strains TEX1 and TEX1*pir42.* That is, the intensity of red fluorescence observed in this assay increases with the plasmid pCOR-*cobA* copy-number.

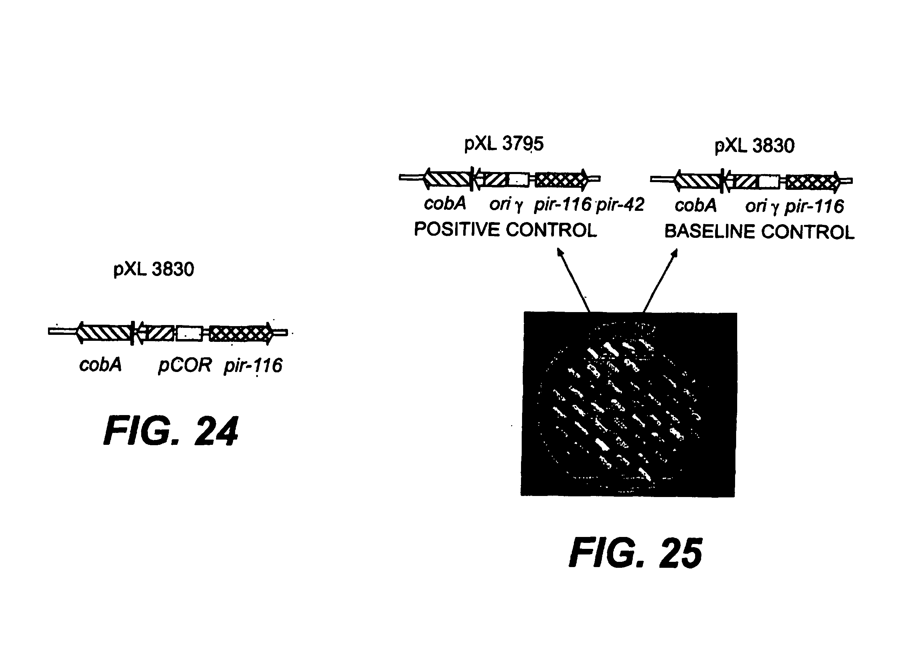

Having demonstrated a positive correlation between fluorescence and *cobA* copy number, we constructed a plasmid into which mutagenized *pir116* genes were introduced for screening. Four plasmids with different combinations of constitutive modules, as shown in Fig. 23, were constructed and tested. One of these plasmids demonstrated a significantly different level of fluorescence when transformed into *pir116* and *pir116pir42* isogenic strains. This plasmid, pXL3830, is shown in pertinent part in Fig. 24.

Control plasmids were used during the screening and evaluation experiments. First, a baseline level control plasmid, pXL3830, containing "wild type" *pir116* was used to set a baseline fluorescence level. Second, pXL3795, that contains the double mutation *pir116-pir42* which increases the copy number of the plasmid by 4 to 6 as compared to pXL3830, was used as a positive control.

Random mutagenesis was performed on *pir116* gene using the Diversify PCR random mutagenesis kit (BD Biosciences Clontech, Palo Alto, CA, USA). Condition 1, which introduced an average of 2 mutations per 1000 base pairs, was used. A preliminary experiment run using "condition 1" has shown by sequencing of 12 mutants that the mutation rate was actually about 2 mutations in the *pir116* gene. The *pir116* gene was amplified as an *Eco*RI-*Sst*I fragment with oligonucleotides C8832 (5-CTTAACGGCTGACATGGGAATTC-3') (SEQ ID NO: 23) and C8833 (5'-CGATGGGCGAGCTCCACCG-3') (SEQ ID NO: 24). After digestion with *Eco*RI and *Sst*I, the mutagenized fragment containing *pir116* was cloned into pXL3830 in place of the "wild-type" *pir116* gene.

Plasmids carrying mutagenized *pir116 ("pir116*")* were transformed into *E. coli* strain XAC-1, the parent of pCOR host XAC1*pir116.* Transformants were screened for increased fluorescence under UV light and compared to XAC1(pXL3830) and XAC1(pXL3795) controls. A duplicate plate was not exposed to UV to minimize secondary mutations. A representative screening plate under UV light is shown in Fig. 25.

The following flow chart summarizes the results of the screening experiment.

The evaluation of the three selected mutants is summarized in Fig. 26. Each mutant showed an increase in copy number as compared to the *pir116* plasmid. In the case of mutants 114C and 100B, the plasmid was essentially in monomeric form. This could be an advantage as compared to a *pir116pir42* plasmid, which has an increased copy number and a high multimer content, like mutant 201C.

The *pir116** gene of each mutant was sequenced. Each clone contains a single non-isocodant mutation in *pir116* ORF. All three of the mutations affect the C terminus of the pi protein, which is involved in DNA binding. None of these mutations have been described before.

Once detected by screening in a plasmid system, these mutations were evaluated in a production system, that is, where the *pir116** gene is introduced into the genome of an *E. coli* pCOR host strain. The strategy for this evaluation is summarized in Fig. 27.

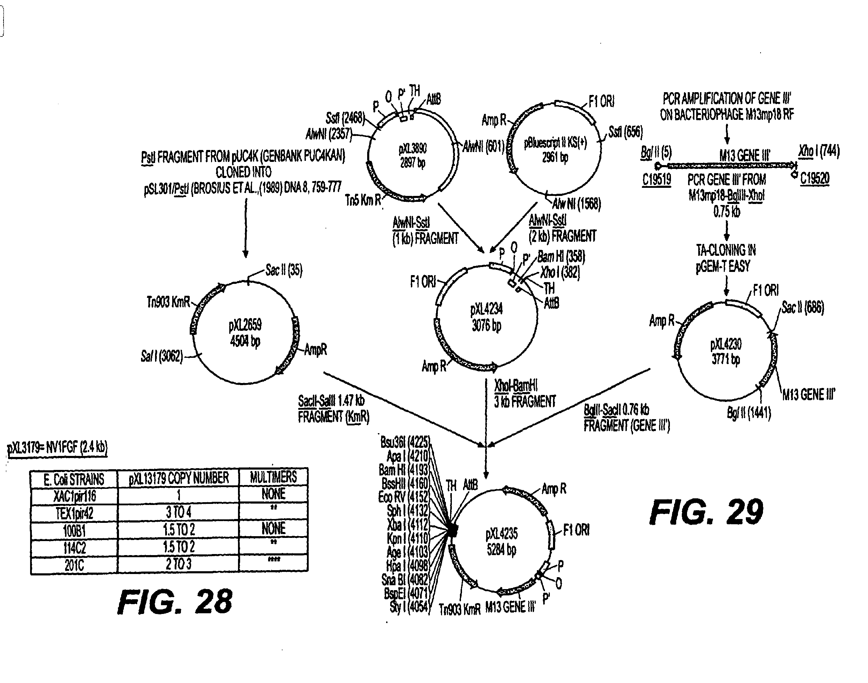

For this evaluation, plasmid pXL3179 plasmid was transformed into each of the three *E. coli* strains bearing the mutations identified in Fig. 26, and assessed for plasmid copy number and topology. The results of these experiments are presented in Fig. 28. It was observed that plasmid copy number was significantly increased relative to XAC1*pir116* only for 201C mutant.

### EXAMPLE 14: Minicircle with M13 gene III as a tool for integration by homologous recombination in E. coli

### 1. Suicide vectors

Gene replacement by double homologous recombination in *E. coli* requires the use of a suicide vector. These vectors are constructed and produced in a host capable of replicating them and used subsequently for recombination into the chromosome of a host unable to replicate them.

Bacteriophage M13 is a very useful genetic tool that can be used in *rep* mutants (Metcalf, W., W. Jiang, et al., Gene 138:1-7 (1994)) or in non-suppressor strains of *E. coli* when M13 mp8 through 11 are used (Blum, P. et al., J Bacteriol., 171:538-46 (1989)). Certain limitations in terms of construction, insert size, and instability are frequently encountered. Plasmids carrying the R6K gamma DNA replication origin are well known suicide vectors (Miller, V. and J. Mekalanos, J Bacteriol., 170:2575-83 (1988)), but they are not useful for modifying *E. coli* strains that express the pi protein, which permits such plasmids to replicate.

A universal suicide vector was engineered with a novel counter selectable marker and used to construct *E. coli* strains wherein mutants of the *pir116* gene *(pir116*)* are inserted into a bacterial genome by homologous recombination. The strategy presented here is demonstrated for mutant 114C, but has also been used to produce strains bearing other *pir*116* mutants.

### 2. Counter-selectable marker

Different markers can be used to select for bacteria having undergone a second recombination event. This event leads to the loss of this marker and in some cases to gene replacement after recombination between the chromosome and a suicide vector. For instance, the *SacB* gene from *Bacillus* is lethal when bacteria expressing the gene are plated on a medium containing sucrose (Ried, J. L. and A. Collmer, Gene 57:239-46 (1987)). As another example, the tetracycline resistance gene confers sensitivity to fusaric acid (Bochner, B. R., et al., J Bacteriol. 143(2):926-3 (1980)). The infection by the bacteriophage M13 confers the sensitivity to the detergent deoxycholate (Blum, P., et al., J Bacteriol.171:538-46 (1989)).

Due to a lack of efficiency in some *E. coli* strains, a positive selection method for double recombinants was developed. Gene III from bacteriophage M13 was evaluated as a counter-selectable marker. This gene encodes a minor virion component responsible for the infectivity of the particles. When overexpressed from the multicopy plasmid pBR322, gene III confers deoxycholate sensitivity on the cells due to insertion of the gene III protein into the membrane of the bacteria (Boeke, J. D. et al., Mol Gen Genet 186:185-92 (1982)). No report indicated if this gene could be used as an efficient counter-selectable marker when present as a single copy in the genome of the *E. coli.* Therefore, we tested this hypothesis with a minicircle suicide vector.

### 3. Amplification by PCR of the deleted version of gene III from M13

To reduce the size of the minicircle vector to be constructed, a deleted version of gene III (gene III') that is still able to confer sensitivity to deoxycholate (Boeke, J. D., P. Model, et al., Mol Gen Genet 186:185-92 (1982)) was chosen. It was amplified by PCR along with its own promoter from M13mp18 (Yanisch-Perron, C., J. Vieira, et al., Gene 33:103-19 (1985)) as a BglII- XhoI fragment (see Figure 29).

The oligonucleotides were as follows:

The amplified fragment was cloned by T-A cloning into pGEMT-easy (Promega Corporation, Madison,WI, USA) to generate pXL4230 (Fig. 29). The nucleotide sequence of the insert was found to agree with that described in GenBank under accession no. VB0018. pXL4230 confers sensitivity to deoxycholate when transformed in *E. coli* strain DH10B (Invitrogen), indicating that it functions as expected.

### 4. Minicircle-based suicide vector

As it does not contain any origin of replication, a minicircle plasmid may be used as a universal suicide vector. For this purpose, a selectable marker such as the kanamycin resistance gene must be added to select for a first homologous recombination event. To counter-select for bacteria that have not undergone a second event of recombination, the gene III' was added to the minicircle vector. The construction of the plasmid used to produce minicircle for recombination is shown in Fig. 29.

The minicircle is generated from a plasmid, such as pXL4235, after induction of the bacteriophage lambda integrase, which recombines between *attP* and *attB* on the plasmid (Darquet, A. M et al., Gene Ther 4(12):1341-9 (1997)). This recombinase is expressed under the control of P_{BAD} in a arabinose-dependent manner in *E. coli* strain G6264, which is described in U.S. application no. 09/981,803. The resulting minicircle contains *attL*, a TH (triple-helix) forming sequence for purification, the selectable marker *Tn903* kanamycin resistance gene, the counter-selectable marker gene III' and the fragment of interest for homologous recombination, cloned in the multi-cloning site of pXL4235 (Fig. 29).

As an example, the constructs used to generate *E. coli* strains expressing a copy-up mutation of *pir116* are described in Fig. 30. These strains can be used to produce pCOR plasmids (Soubrier, F. et al., Gene Ther 6:1482-1488 (1999)). Since there is no homology between pir and the bacterial genome, the *pir116** sequences were inserted into the *E. coli* chromosomal *uidA* gene, which encodes β-D-glucuronidase. This gene provides sufficient sequence similarity with the *E. coli* genome for homologous recombination to occur.

The protocol for the purification of minicircle and recombination occurred as follows. Plasmid pal4256 (Fig. 30) was transformed in *E. coli* strain G6264 to generate G6656. Fifty ml of LB medium supplemented with ampicillin (100 mg/l) were inoculated with 0.5 ml of an overnight culture of G6656 and incubated at 37°C, with shaking at 200 rpm until the optical density at 600 nm reached 0.7. Minicircle production was induced by the addition of 250µl of a sterile solution of 10% arabinose to the medium. After 30 minutes at 37°C, 200 rpm, total plasmid DNA was extracted using the Wizard Plus Midipreps DNA Purification system (Promega Corporation, Madison WI, USA).

Six µg of the plasmid DNA preparation were loaded onto a 0.8% agarose preparative gel. A supercoiled DNA ladder (Promega Corporation, Madison WI, USA) was used as a molecular weight standard. After electrophoresis overnight at 50V, the supercoiled minicircle construct (5.1 kb) was extracted and purified from the gel using an SV gel purification kit (Promega Corporation, Madison WI, USA).

### 5. Double homologous recombination with minicircle 4256 (uidA::pir116* minicircle suicide vector)

The recombination steps for constructing the strains and the corresponding phenotypes are described in Fig. 31.

For the first recombination event (integration), 0.2, 1 and 5 µl of purified minicircle 4256 were electroporated in *E. coli* strain XAC1 (Normanly, J et al., Proc Natl Acad Sci USA 83:6548-52 (1986)), which is the parental strain for pCOR hosts. Kanamycin resistant colonies were obtained on LB Agar supplemented with kanamycin (50 mg/l) after overnight incubation at 37°C.

To evaluate the number of colonies potentially containing contaminant non-recombined pXL4256, 50 Km^{R} colonies were streaked in parallel on LB Agar supplemented with kanamycin or ampicillin. Only 4 colonies out of 50 were resistant to kanamycin and ampicillin and were shown by plasmid restriction analysis to contain non-recombined pXL4256. This indicated that 46 colonies out of 50 obtained by electroporation were actually minicircle 4256 integrants.

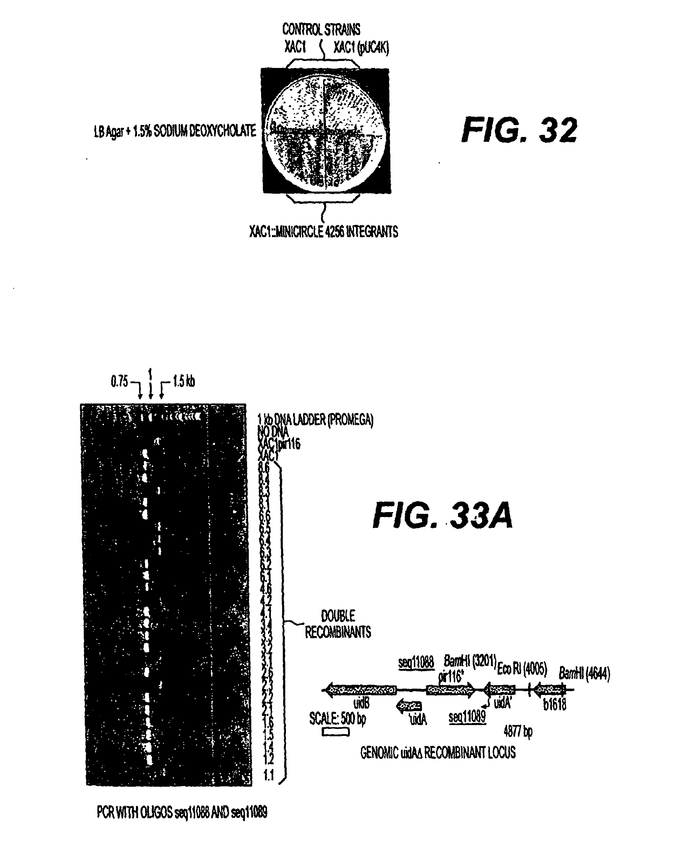

For the second recombination event (excision), all of the 46 Km^{R} integrants were isolated on freshly prepared LB Agar plates containing 1.5 % sodium deoxycholate ("Doc"; Sigma) and incubated at 37°C overnight. Only a few deoxycholate-resistant (Doc^{R}) colonies (1 to 15) were obtained for each integrant, as shown in Fig. 32. This result was consistent with the selection of a relatively rare event, such as the second recombination event. 100 Doc^{R} colonies obtained from 15 integrants were patched in parallel on LB Agar with 1.5% Doc and LB Agar plus kanamycin to screen for Doc^{R} and Km^{S} double recombinants. 86 % of the screened colonies were Km^{S}, indicating that they had lost the suicide vector.

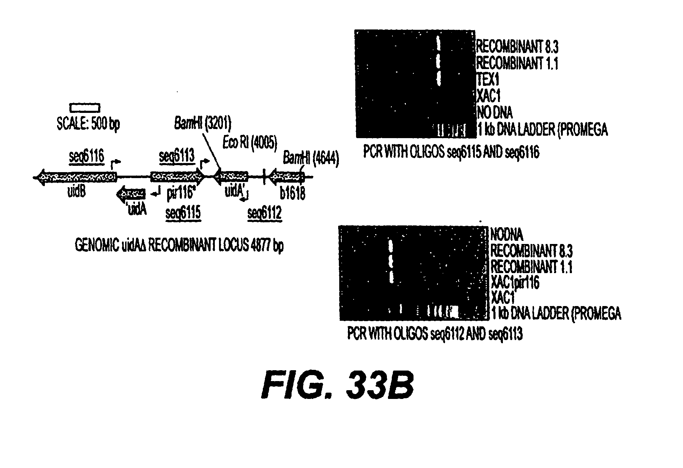

To screen for allele replacement, the chromosomal *uidA* locus was amplified by PCR. If allele replacement has occurred, the expected PCR fragment size is 1.3 kb. The fragment size corresponding to wild-type *uidA* locus, that is, without an integrated *pir116** mutation, is 0.85 kb. The results presented in Fig. 33-panel A indicate that allele replacement has occurred in 30% of the double recombinants. This was confirmed by phenotypic analysis because these clones are also UidA- (beta glucuronidase -) and give white colonies on LB agar supplemented with Xgluc.

The integrity of the bacterial genome in the region close to the site of homologous recombination was checked by PCR on two independent recombinants. The first primer (seq6113 or seq6115) was based on the sequence of the *pir* gene and the second (seq6112 or seq6116) had a sequence based on a sequence close to, but outside of, the homology region (immediately 5' or 3' of *uidA*). XAC1 DNA was used as a negative control, whereas XAC1*pir116* or TEX1 (Soubrier, F. et al., Gene Ther 6:1482-1488 (1999)) were used as positive controls.

The oligonucleotides used as PCR primers were the following:

| | |
|---|---|
| Seq11088 : | 5'-GAGATCGCTGATGGTATCGG-3' (SEQ ID NO: 27) |
| Seq11089: | 5'-TCTACACCACGCCGAACACC-3' (SEQ ID NO: 28) |
| Seq6112: | 5'-GACCAGTATTATTATCTTAATGAG-3' (SEQ ID NO: 29) |
| Seq6113: | 5'-GTATTTAATGAAACCGTACCTCCC-3'(SEQ ID NO: 30) |
| Seq6115: | 5'-CTCTTTTAATTGTCGATAAGCAAG-3' (SEQ ID NO: 31) |
| Seq6116: | 5'-GCGACGTCACCGAGGCTGTAGCCG-3' (SEQ ID NO: 32) |

The expected size for the PCR product is 0.83 kb using primers seq6112 and seq6113, and 0.88 kb when using primers seq6114 and seq6115. Results are presented in Figure 33-panel B. The two double recombinants obtained with the minicircle suicide vector showed the expected PCR profile. This demonstrates that double homologous recombination can be easily achieved in *E. coli* using minicircle plasmids as suicide vector and the M13 gene III' as a counterselectable marker. This gene replacement technique can be directly universally carried out in any micro-organisms genetic background.

### BIBLIOGRAPHY

Alting-Mees, M.A., J.A. Sorge, and J.M. Short. 1992. Methods Enzymol. 216:483-495

Blum, P., D. Holzschu, H.S. Kwan, D. Riggs, and S. Artz. 1989. J. Bacteriol. 171:538-546.

Brosius, J. 1989. Methods Enzymol. 216:469-483.

Chambers, S.P., S.E. Prior, D.A. Barstow, and N.P. Minton. 1988. Gene 68:139-149.

Chung, C.T., and R.H. Miller. 1988. Nucleic Acids Res. 16:3580.

Colloms, S.D., P. Sykora, G. Szatmari, and D.J. Sherrat. 1990 J. Bacteriol. 172:6973-6980.

Datta, N., and P. Kontomichalou. 1965. Nature 208:239-241.

Dickely, F., D. Nilsson, E.B. Hansen, and E. Johansen. 1995. Mol. Microbiol. 15:839-847.

Filutowicz, M., S. Dellis, I. Levchenko, M. Urh, F. Wu, and D. York. 1994. Prog. in Nucleic Acid Res. and Mol. Biol. 48:239-273.

Gibson, T.J. 1984. Ph.D Thesis. University of Cambridge.

Greener, A., M. Filutowicz, M. McEachern, and D. Helsinki. 1990. Mol. Gen. Genet. 224:24-32.

Herrero, M., V. de Lorenzo, and K.N. Timmis. 1990. J. Bacteriol. 172:6557-6567.

Hodgson, C.P. 1995. Bio/Technology 13:222-225.

Inuzuka, M., and Y. Wada. 1985. EMBO J. 4:2301-2307.

Jaye, M. et al., (1986) Science 233:541-5

Kleina, L.G., J.M. Masson, J. Normanly, J. Abelson, and J.H. Miller. 1990. J. Mol. Biol. 213:705-717.

Kowalczykowski, S.C., and A.K. Eggleston. 1994. Annu. Rev. Biochem. 63:9991-10043.

Leung, D.W., E. Chen, G. Cachianes, and D.V. Goeddel. 1985. DNA 4:351-355.

Maniatis, T., E.F. Fritsch, and J. Sambrook. 1989. Cold Spring Harbor Laboratory, Cold Spring Harbor, N.Y.

Meinnel, T., E. Schmitt, Y. Mechulam, and S. Blanquet. 1992. J. Bacteriol. 174:2323-2331.

Mertens, N., E. Remant and W. Fiers. (1995) Bio/Technology 13:175-179

Messing, J., and J. Vieira. 1982. Gene 19: 269-276.

Metcalf, W.W., W. Jiang, and B.L. Wanner. 1994. Gene 138:1-7.

Miller, V.L., and J.J. Mekalanos. 1988. J. Bacteriol. 170:2575-2583.

Normanly, J., J.M. Masson, L.G. Kleina, J. Abelson, and J.H. Miller. 1986. Proc. Natl. Acad. Sci. USA 83:6548-6552.

Normanly, J., L.G. Kleina, J.M. Masson, J. Abelson, and J.H. Miller. 1990. J. Mol. Biol. 213:719-726.

Roca, J. 1995. TIBS 20:156-160.

Saïki, R.K., S. Scharf, F. Faloona, K.B. Mullis, G.T. Horn, H.A. Erlich, and N. Arnheim. 1985. Science 230:1350-1354.

Sanger, F., S. Nicklen, and A.R. Coulson. 1977. Proc. Natl. Acad. Sci. USA 74:5463-5467.

Sawadogo, M., and M.W. Van Dyke. 1991. Nucleic Acids Res. 19:674.

Scott, J.R. 1984. Microbiol. Rev. 48:1-23.

Simoes, D.A., M. Dal Jensen, E. Dreveton, M.O. Loret, S. Blanchin-Roland, J.L. Uribelarrea, and J.M. Masson. 1991. Ann. N.Y. Acad. Sci. 646:254-258.

Simon, R., U. Priefer, and A. Pühler. 1983. Bio/Technology 1:784-791.

Sinha, N.D., J. Biernat, J. McManus, and H. Köster. 1984. Nucleic Acids Res. 12:4539-4557.

Stirling, C.J. G. Stewart, and D.J. Sherrat. 1988. Mol. Gen. Genet. 214:80-84.

Stirling, C.J., S.D. Colloms, J.F. Collins, G. Szatmari, and D.J. Sherrat. 1989. EMBO J. 8:1623-1627.

Studier, F.W., A.H. Rosenberg., J.J. Dunn and J.W. Dubendorff (1990). Methods Enzymol 185:60-89.

Summers, D.K., and D.J. Sherrat. 1984. Cell 36:1097-1103.

Takahashi, K., Y. Sawasaki, J. Hata, K. Mukai and T. Goto. (1990) In Vitro Cell Dev. Biol. 26:265-74.

Vieira, J., and J. Messing. 1982. Gene 19:259-268.

Wiechelman, K., R. Braun, and J. Fitzpatrick. 1988. Anal. Biochem. 175:231-237.

Yanisch-Perron, C. Vieira and J. Messing (1985) Gene 33:103-119 13.

## Claims

1. A prokaryotic recombinant host cell comprising a heterologous pir gene encoding a pi protein and an extrachromosomal DNA molecule comprising a heterologous therapeutic gene and a conditional origin of replication whose functionality in the prokaryotic recombinant host cell requires the pi protein, wherein the heterologous pir gene comprises at least one mutation in addition to the pir116 mutation, said additional mutation being selected from the group consisting of residue 130 of the pi protein being a valine, residue 292 of the pi protein being a methionine or residue 117 of the pi protein being a glycine.

2. The prokaryotic recombinant host cell of any of the previous claims, wherein the heterologous pir gene is in a plasmid.

3. The prokaryotic recombinant host cell of claim 1, wherein the heterologous pir gene is in the genome of the host cell.

4. The prokaryotic recombinant host cell of any of the previous claims, wherein the conditional origin of replication is from a bacterial plasmid or a bacteriophage.

5. The prokaryotic recombinant host cell of claim 4, wherein the conditional origin of replication is from R2K, R6K, RI, pSC101, Rtsl, F, RSF1010, P1, P4, lambda, Phi82 or Phi80.

6. The prokaryotic recombinant host cell of claim 5, wherein the conditional origin of replication is from bacterial plasmid R6K.

7. The prokaryotic recombinant host cell of claim 6, wherein the origin of replication comprises SEQ ID NO: 1.

8. The prokaryotic recombinant host cell of any of claims 4-7, wherein the plasmid further comprises a selection gene that does not impart resistance to an antibiotic.

9. The prokaryotic recombinant host cell of claim 8, wherein the selection gene encodes a suppressor transfer RNA for specific codons.

10. The prokarytic recombinant host cell of claim 8 wherein the selection gene is a suppressor of an amber transfer RNA, and the host cell further comprises a gene comprising an amber mutation.

11. The prokaryotic recombinant host cell of any of the previous claims, wherein said host cell is deficient for the gene endA.

12. The prokaryotic recombinant host cell of any of the previous claims, wherein said host cell is deficient for the gene traD.

13. The prokaryotic recombinant host cell of any of the previous claims, wherein said host cell further comprises a mutated recA gene.

14. A method for producing a plasmid comprising a heterologous therapeutic gene and a conditional origin of replication whose functionality in a prokaryotic host cell requires a replication initiating protein foreign to the host cell, comprising: a) culturing the prokaryotic recombinant host cell of any of the previous claims under conditions wherein the plasmid is produced; and b) isolating the plasmid.

## Patentansprüche

1. Prokaryontische rekombinante Wirtszelle, umfassend ein für ein pi-Protein codierendes heterologes pir-Gen sowie ein extrachromosomales DNA-Molekül, umfassend ein heterologes therapeutisches Gen und einen konditionalen Replikationsursprung, dessen Funktionalität in der prokaryontischen rekombinanten Wirtszelle das pi-Protein benötigt, wobei das heterologe pir-Gen wenigstens eine zusätzliche Mutation zu der pir116-Mutation enthält, wobei die zusätzliche Mutation ausgewählt ist aus der Gruppe bestehend aus Rest 130 des pi-Proteins, bei dem es sich um ein Valin handelt, Rest 292 des pi-Proteins, bei dem es sich um ein Methionin handelt oder Rest 117 des pi-Proteins, bei dem es sich um ein Glycin handelt.

2. Prokaryontische rekombinante Wirtszelle nach dem vorhergehenden Anspruch, wobei das heterologe pir-Gen in einem Plasmid vorliegt.

3. Prokaryontische rekombinante Wirtszelle nach Anspruch 1, wobei das heterologe pir-Gen im Genom der Wirtszelle vorliegt.

4. Prokaryontische rekombinante Wirtszelle nach einem der vorhergehenden Ansprüche, wobei der konditionale Replikationsursprung aus einem bakteriellen Plasmid oder einem Bakteriophagen stammt.

5. Prokaryontische rekombinante Wirtszelle nach Anspruch 4, wobei der konditionale Replikationsursprung aus R2K, R6K, R1, pSC101, Rts1, F, RSF1010, P1, P4, lambda, Phi82 oder Phi80 stammt.

6. Prokaryontische rekombinante Wirtszelle nach Anspruch 5, wobei der konditionale Replikationsursprung aus dem bakteriellen Plasmid R6K stammt.

7. Prokaryontische rekombinante Wirtszelle nach Anspruch 6, wobei der Replikationsursprung SEQ ID NO: 1 umfasst.

8. Prokaryontische rekombinante Wirtszelle nach einem der Ansprüche 4-7, wobei das Plasmid ferner ein Selektionsgen umfasst, das keine Antibiotikaresistenz verleiht.

9. Prokaryontische rekombinante Wirtszelle nach Anspruch 8, wobei das Selektionsgen eine Suppressor-Transfer-RNA für spezifische Codons codiert.

10. Prokaryontische rekombinante Wirtszelle nach Anspruch 8, wobei es sich bei dem Selektionsgen um einen Suppressor einer Amber-Transfer-RNA handelt und die Wirtszelle ferner ein eine Amber-Mutation enthaltendes Gen umfasst.

11. Prokaryontische rekombinante Wirtszelle nach einem der vorhergehenden Ansprüche, wobei die Wirtszelle einen Mangel an dem Gen endA aufweist.

12. Prokaryontische rekombinante Wirtszelle nach einem der vorhergehenden Ansprüche, wobei die Wirtszelle einen Mangel an dem Gen traD aufweist.

13. Prokaryontische rekombinante Wirtszelle nach einem der vorhergehenden Ansprüche, wobei die Wirtszelle ferner ein mutiertes recA-Gen aufweist.

14. Verfahren zur Herstellung eines Plasmids, umfassend ein heterologes therapeutisches Gen und einen konditionalen Replikationsursprung, dessen Funktionalität in einer prokaryontischen Wirtszelle ein für die Wirtszelle fremdes Replikationsstarterprotein benötigt, wobei man in dem Verfahren a) die prokaryontische rekombinante Wirtszelle nach einem der vorhergehenden Ansprüche unter Bedingungen, bei denen das Plasmid produziert wird, kultiviert und b) das Plasmid isoliert.

## Revendications

1. Cellule hôte recombinante procaryote comprenant un gène pir hétérologue codant pour une protéine pi et une molécule d'ADN extra-chromosomique comprenant un gène thérapeutique hétérologue et une origine de réplication conditionnelle dont la fonctionnalité dans la cellule hôte recombinante procaryote nécessite la protéine pi, le gène pir hétérologue comprenant au moins une mutation outre la mutation pir116, ladite mutation supplémentaire étant choisie dans le groupe constitué du résidu 130 de la protéine pi qui est une valine, du résidu 292 de la protéine pi qui est une méthionine ou du résidu 117 de la protéine pi qui est une glycine.

2. Cellule hôte recombinante procaryote selon la revendication précédente, **caractérisée en ce que** le gène pir hétérologue est dans un plasmide.

3. Cellule hôte recombinante procaryote selon la revendication 1, **caractérisée en ce que** le gène pir hétérologue est dans le génome de la cellule hôte.

4. Cellule hôte recombinante procaryote selon l'une quelconque des revendications précédentes, **caractérisée en ce que** l'origine de réplication conditionnelle provient d'un plasmide bactérien ou d'un bactériophage.

5. Cellule hôte recombinante procaryote selon la revendication 4, **caractérisée en ce que** l'origine de réplication conditionnelle provient de R2K, R6K, RI, pSC101, Rtsl, F, RSF1010, P1, P4, lambda, Phi82 ou Phi80.

6. Cellule hôte recombinante procaryote selon la revendication 5, **caractérisée en ce que** l'origine de réplication conditionnelle provient du plasmide bactérien R6K.

7. Cellule hôte recombinante procaryote selon la revendication 6, **caractérisée en ce que** l'origine de réplication comprend SEQ ID NO: 1.

8. Cellule hôte recombinante procaryote selon l'une quelconque des revendications 4 à 7, **caractérisée en ce que** le plasmide comprend en outre un gène de sélection qui ne confère pas de résistance à un antibiotique.

9. Cellule hôte recombinante procaryote selon la revendication 8, **caractérisée en ce que** le gène de sélection code pour un ARN de transfert suppresseur pour des codons spécifiques.

10. Cellule hôte recombinante procaryote selon la revendication 8, **caractérisée en ce que** le gène de sélection est un suppresseur d'un ARN de transfert ambre, et la cellule hôte comprend en outre un gène comprenant une mutation ambre.

11. Cellule hôte recombinante procaryote selon l'une quelconque des revendications précédentes, **caractérisée en ce que** ladite cellule hôte est déficiente pour le gène endA.

12. Cellule hôte recombinante procaryote selon l'une quelconque des revendications précédentes, **caractérisée en ce que** ladite cellule hôte est déficiente pour le gène traD.

13. Cellule hôte recombinante procaryote selon l'une quelconque des revendications précédentes, **caractérisée en ce que** ladite cellule hôte comprend en outre un gène recA muté.

14. Méthode de production d'un plasmide comprenant un gène thérapeutique hétérologue et une origine de réplication conditionnelle dont la fonctionnalité dans une cellule hôte procaryote nécessite une protéine d'initiation de réplication étrangère à la cellule hôte, comprenant : a) la culture de la cellule hôte recombinante procaryote selon l'une quelconque des revendications précédentes dans des conditions dans lesquelles le plasmide est produit ; et b) l'isolement du plasmide.


### SEQUENCE LISTING

<110> CENTELION
   Soubrier, Fabienne
<120> circular DNA Molecule with Conditional Origin of Replication, Method for
   Preparing same, and use Thereof in Gene Therapy
<130> 8888.0132-01
<150> US 10/268,948
   <151> 2002-10-11
<160> 39
<170> PatentIn version 3.1
<210> 1
   <211> 389
   <212> DNA
   <213> Escherichia coli
<400> 1
   tgtcagccgt taagtgttcc tgtgtcactg aaaattgctt tgagaggctc taagggcttc 60
   tcagtgcgtt acatccctgg cttgttgtcc acaaccgtta aaccttaaaa gctttaaaag 120
   ccttatatat tctttttttt cttataaaac ttaaaacctt agaggctatt taagttgctg 180
   atttatatta attttattgt tcaaacatga gagcttagta cgtgaaacat gagagcttag 240
   tacgttagcc atgagagctt agtacgttag ccatgagggt ttagttcgtt aaacatgaga 300
   gcttagtacg ttaaacatga gagcttagta cgtgaaacat gagagcttag tacgtactat 360
   caacaggttg aactgctgat cttcagatc 389
<210> 2
   <211> 960
   <212> DNA
   <213> Escherichia coli
<400> 2
   tatacagaat gatgaggttt ttttatgaga ctcaaggtca tgatggacgt gaacaaaaaa 60
   acgaaaattc gccaccgaaa cgagctaaat cacaccctgg ctcaacttcc tttgcccgca 120
   aagcgagtga tgtatatggc gcttgctccc attgatagca aagaacctct tgaacgaggg 180
   cgagttttca aaattagggc tgaagacctt gcagcgctcg ccaaaatcac cccatcgctt 240
   gcttatcgac aattaaaaga gggtggtaaa ttacttggtg ccagcaaaat ttcgctaaga 300
   ggggatgata tcattgcttt agctaaagag cttaacctgc cctttactgc taaaaactcc 360
   cctgaagagt tagatcttaa cattattgag tggatagctt attcaaatga tgaaggatac 420
   ttgtctttaa aattcaccag aaccatagaa ccatatatct ctagccttat tgggaaaaaa 480
   aataaattca caacgcaatt gttaacggca agcttacgct taagtagcca gtattcatct 540
   tctctttatc aacttatcag gaagcattac tctaatttta agaagaaaaa ttattttatt 600
   atttccgttg atgagttaaa ggaagagtta acagcttata cttttgataa agatggaaat 660
   attgagtaca aataccctga ctttcctatt tttaaaaggg atgtgttaaa taaagccatt 720
   gctgaaatta aaaagaaaac agaaatatcg tttgttggct tcactgttca tgaaaaagaa 780
   ggaagaaaaa ttagtaagct gaagttcgaa tttgtcgttg atgaagatga attttctggc 840
   gataaagatg atgaagcttt ttttatgaat ttatctgaag ctgatgcagc ttttctcaag 900
   gtatttaatg aaaccgtacc tcccaaaaaa gctaaggggt gatatatggc taaaatttac 960
<210> 3
   <211> 24
   <212> DNA
   <213> Escherichia coli
<400> 3
   gaccagtatt attatcttaa tgag 24
<210> 4
   <211> 24
   <212> DNA
   <213> Escherichia coli
<400> 4
   gtatttaatg aaaccgtacc tccc 24
<210> 5
   <211> 24
   <212> DNA
   <213> Escherichia coli
<400> 5
   ctcttttaat tgtcgataag caag 24
<210> 6
   <211> 24
   <212> DNA
   <213> Escherichia coli
<400> 6
   gcgacgtcac cgaggctgta gccg 24
<210> 7
   <211> 36
   <212> DNA
   <213> Escherichia coli
<400> 7
   gtatatggcg cttgctctca tcgatagcaa agaacc 36
<210> 8
   <211> 36
   <212> DNA
   <213> Escherichia coli
<400> 8
   ggttctttgc tatcgatgag agcaagcgcc atatac 36
<210> 9
   <211> 20
   <212> DNA
   <213> Escherichia coli
<400> 9
   gagatcgctg atggtatcgg 20
<210> 10
   <211> 20
   <212> DNA
   <213> Escherichia coli
<400> 10
   tctacaccac gccgaacacc 20
<210> 11
   <211> 36
   <212> DNA
   <213> Escherichia coli
<400> 11
   cgcaattgtt aacgtccagc ttacgcttaa gtagcc 36
<210> 12
   <211> 36
   <212> DNA
   <213> Escherichia coli
<400> 12
   ggctacttaa gcgtaagctg gacgttaaca attgcg 36
<210> 13
   <211> 31
   <212> DNA
   <213> Escherichia coli
<400> 13
   ccctctagat cgatagccat ttttactcct g 31
<210> 14
   <211> 29
   <212> DNA
   <213> Escherichia coli
<400> 14
   cgggatcctg attatgccgt gtctattag 29
<210> 15
   <211> 36
   <212> DNA
   <213> Escherichia coli
<400> 15
   cccaagcttc ttcgttagtt tctgctacgc cttcgc 36
<210> 16
   <211> 35
   <212> DNA
   <213> Escherichia coli
<400> 16
   ggtctagaac gtgaaagtgg tgaagaacaa aatcg 35
<210> 17
   <211> 20
   <212> DNA
   <213> Escherichia coli
<400> 17
   gcgacccttg tgtatcaaac 20
<210> 18
   <211> 20
   <212> DNA
   <213> Escherichia coli
<400> 18
   ggtattaccc ggcatgacag 20
<210> 19
   <211> 20
   <212> DNA
   <213> Escherichia coli
<400> 19
   gtggtggaaa tggcgatagg 20
<210> 20
   <211> 20
   <212> DNA
   <213> Escherichia coli
<400> 20
   gcgattttgt tcttcaccac 20
<210> 21
   <211> 918
   <212> DNA
   <213> Escherichia coli
<400> 21
   atgagactca aggtcatgat ggacgtgaac aaaaaaacga aaattcgcca ccgaaacgag 60
   ctaaatcaca ccctggctca acttcctttg cccgcaaagc gagtgatgta tatggcgctt 120
   gctctcatcg atagcaaaga acctcttgaa cgagggcgag ttttcaaaat tagggctgaa 180
   gaccttgcag cgctcgccaa aatcacccca tcgcttgctt atcgacaatt aaaagagggt 240
   ggtaaattac ttggtgccag caaaatttcg ctaagagggg atgatatcat tgctttagct 300
   aaagagctta acctgctctt tactgctaaa aactcccctg aagagttaga tcttaacatt 360
   attgagtgga tagcttattc aaatgatgaa ggatacttgt ctttaaaatt caccagaacc 420
   atagaaccat atatctctag ccttattggg aaaaaaaata aattcacaac gcaattgtta 480
   acggcaagct tacgcttaag tagccagtat tcatcttctc tttatcaact tatcaggaag 540
   cattactcta attttaagaa gaaaaattat tttattattt ccgttgatga gttaaaggaa 600
   gagttaatag cttatacttt tgataaagat ggaaatattg agtacaaata ccctgacttt 660
   cctattttta aaagggatgt gttaaataaa gccattgctg aaattaaaaa gaaaacagaa 720
   atatcgtttg ttggcttcac tgttcatgaa aaagaaggaa gaaaaattag taagctgaag 780
   ttcgaatttg tcgttgatga agatgaattt tctggcgata aagatgatga agcttttttt 840
   atgaatttat ctgaagctga tgcagctttt ctcaaggtat ttgatgaaac cgtacctccc 900
   aaaaaagcta aggggtga 918
<210> 22
   <211> 305
   <212> PRT
   <213> Escherichia coli
<400> 22
   Met Arg Leu Lys Val Met Met Asp Val Asn Lys Lys Thr Lys Ile Arg
   1               5                   10                  15
   His Arg Asn Glu Leu Asn His Thr Leu Ala Gln Leu Pro Leu Pro Ala
               20                  25                  30
   Lys Arg Val Met Tyr Met Ala Leu Ala Leu Ile Asp Ser Lys Glu Pro
           35                  40                  45
   Leu Glu Arg Gly Arg Val Phe Lys Ile Arg Ala Glu Asp Leu Ala Ala
       50                  55                  60
   Leu Ala Lys Ile Thr Pro Ser Leu Ala Tyr Arg Gln Leu Lys Glu Gly
   65                  70                  75                  80
   Gly Lys Leu Leu Gly Ala Ser Lys Ile Ser Leu Arg Gly Asp Asp Ile
                   85                  90                  95
   Ile Ala Leu Ala Lys Glu Leu Asn Leu Leu Phe Thr Ala Lys Asn Ser
               100                 105                 110
   Pro Glu Glu Leu Asp Leu Asn Ile Ile Glu Trp Ile Ala Tyr Ser Asn
           115                 120                 125
   Asp Glu Gly Tyr Leu Ser Leu Lys Phe Thr Arg Thr Ile Glu Pro Tyr
       130                 135                 140
   Ile Ser Ser Leu Ile Gly Lys Lys Asn Lys Phe Thr Thr Gln Leu Leu
   145                 150                 155                 160
   Thr Ala Ser Leu Arg Leu Ser Ser Gln Tyr Ser Ser Ser Leu Tyr Gln
                   165                 170                 175
   Leu Ile Arg Lys His Tyr Ser Asn Phe Lys Lys Lys Asn Tyr Phe Ile
               180                 185                 190
   Ile Ser Val Asp Glu Leu Lys Glu Glu Leu Ile Ala Tyr Thr Phe Asp
           195                 200                 205
   Lys Asp Gly Asn Ile Glu Tyr Lys Tyr Pro Asp Phe Pro Ile Phe Lys
       210                 215                 220
   Arg Asp Val Leu Asn Lys Ala Ile Ala Glu Ile Lys Lys Lys Thr Glu
   225                 230                 235                 240
   Ile Ser Phe Val Gly Phe Thr Val His Glu Lys Glu Gly Arg Lys Ile
                   245                 250                 255
   Ser Lys Leu Lys Phe Glu Phe Val Val Asp Glu Asp Glu Phe Ser Gly
               260                 265                 270
   Asp Lys Asp Asp Glu Ala Phe Phe Met Asn Leu Ser Glu Ala Asp Ala
           275                 280                 285
   Ala Phe Leu Lys Val Phe Asp Glu Thr Val Pro Pro Lys Lys Ala Lys
       290                 295                 300
   Gly
   305
<210> 23
   <211> 23
   <212> DNA
   <213> Escherichia coli
<400> 23
   cttaacggct gacatgggaa ttc 23
<210> 24
   <211> 19
   <212> DNA
   <213> Escherichia coli
<400> 24
   cgatgggcga gctccaccg 19
<210> 25
   <211> 29
   <212> DNA
   <213> Bacteriophage M13mp18
<400> 25
   ggcagatctt aaaccgatac aattaaagg 29
<210> 26
   <211> 36
   <212> DNA
   <213> Bacteriophage M13mp18
<400> 26
   ccgctcgagt tacgattggc cttgatattc acaaac 36
<210> 27
   <211> 20
   <212> DNA
   <213> Escherichia coli
<400> 27
   gagatcgctg atggtatcgg 20
<210> 28
   <211> 20
   <212> DNA
   <213> Escherichia coli
<400> 28
   tctacaccac gccgaacacc 20
<210> 29
   <211> 24
   <212> DNA
   <213> Escherichia coli
<400> 29
   gaccagtatt attatcttaa tgag 24
<210> 30
   <211> 24
   <212> DNA
   <213> Escherichia coli
<400> 30
   gtatttaatg aaaccgtacc tccc 24
<210> 31
   <211> 24
   <212> DNA
   <213> Escherichia coli
<400> 31
   ctcttttaat tgtcgataag caag 24
<210> 32
   <211> 24
   <212> DNA
   <213> Escherichia coli
<400> 32
   gcgacgtcac cgaggctgta gccg 24
<210> 33
   <211> 12
   <212> DNA
   <213> Escherichia coli
<400> 33
   agaaaaaaag ga 12
<210> 34
   <211> 12
   <212> DNA
   <213> Escherichia coli
<400> 34
   tctttttttc ct 12
<210> 35
   <211> 14
   <212> DNA
   <213> Escherichia coli
<400> 35
   aagaaaaaaa agaa 14
<210> 36
   <211> 14
   <212> DNA
   <213> Escherichia coli
<400> 36
   ttcttttttt tctt 14
<210> 37
   <211> 12
   <212> DNA
   <213> Escherichia coli
<400> 37
   tctttttttc ct 12
<210> 38
   <211> 14
   <212> DNA
   <213> Escherichia coli
<400> 38
   ttcttttttt tctt 14
<210> 39
   <211> 17
   <212> DNA
   <213> Escherichia coli
<400> 39
   aaaaaaggga ataaggg 17